(19) Europäisches Patentamt
European Patent Office
Office européen des brevets

(11) Veröffentlichungsnummer : **0 065 229**
**B1**

(12) # EUROPÄISCHE PATENTSCHRIFT

(45) Veröffentlichungstag der Patentschrift : **07.08.85**

(21) Anmeldenummer : **82103931.0**

(22) Anmeldetag : **06.05.82**

(51) Int. Cl.⁴ : **C 07 D223/16**, C 07 D243/04, C 07 D243/02, A 61 K 31/55// C07C59/64, C07C91/04, C07C93/14, C07C103/64, C07D205/04, C07D405/12, C07D491/04

(54) **Neue Benzazepinderivate, ihre Herstellung und ihre Verwendung als Arzneimittel.**

(30) Priorität : **19.05.81 DE 3119874**

(43) Veröffentlichungstag der Anmeldung : **24.11.82 Patentblatt 82/47**

(45) Bekanntmachung des Hinweises auf die Patenterteilung : **07.08.85 Patentblatt 85/32**

(84) Benannte Vertragsstaaten : **AT BE CH DE FR IT LI LU NL SE**

(56) Entgegenhaltungen :
**EP-A- 0 002 624**
**EP-A- 0 009 800**
**EP-A- 0 011 747**
**DE-A- 2 639 718**
**US-A- 3 474 090**
**US-A- 3 780 023**
**US-A- 4 210 749**
**Die Akte enthält technische Angaben, die nach dem Eingang der Anmeldung eingereicht wurden und die nicht in dieser Patentschrift enthalten sind.**

(73) Patentinhaber : **Dr. Karl Thomae GmbH**
**Postfach 1755**
**D-7950 Biberach (Riss) (DE)**

(72) Erfinder : **Reiffen, Manfred, Dr., Dipl.-Chem.**
**Amriswilstrasse 7**
**D-7950 Biberach 1 (DE)**
Erfinder : **Heider, Joachim, Dr., Dipl.-Chem.**
**Am Hang 3**
**D-7951 Warthausen (DE)**
Erfinder : **Hauel, Norbert, Dr., Dipl.-Chem.**
**Händelstrasse 12**
**D-7950 Biberach 1 (DE)**
Erfinder : **Austel, Volkhard, Dr., Dipl.-Chem.**
**Kapellenweg 7**
**D-7950 Biberach 1 (DE)**
Erfinder : **Eberlein, Wolfgang, Dr., Dipl.-Chem.**
**Obere Au 6**
**D-7950 Biberach 1 (DE)**
Erfinder : **Kobinger, Walter, Prof. Dr.**
**Belghofergasse 27**
**A-1121 Wien (AT)**
Erfinder : **Lillie, Christian, Dr.**
**Hansl Niese Weg 12**
**A-1130 Wien (AT)**
Erfinder : **Noll, Klaus, Dr., Dipl.-Chem.**
**Im Schönblick 3**
**D-7951 Warthausen (DE)**
Erfinder : **Pieper, Helmut, Dr., Dipl.-Chem.**
**Kapellenweg 5**
**D-7950 Biberach 1 (DE)**
Erfinder : **Krüger, Gerd, Dr., Dipl.-Chem.**
**Ginsterhalde 30**
**D-7950 Biberach 1 (DE)**
Erfinder : **Keck, Johannes, Dr., Dipl.-Chem.**
**Talfeldstrasse 27**
**D-7950 Biberach 1 (DE)**

**EP 0 065 229 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

In der britischen Patentschrift 1 546 844, welche der DE-A-2 639 718 entspricht, wird als strukturähnlichste vorbekannte Verbindung 1-[7,8-Dimethoxy-1,2,3,4-tetrahydro-5H-2-benzazepin-1-on-2-yl]-3-[N-methyl-N-(2-(3,4-dimethoxy-phenyl)-ethyl)-amino]-propan sowie als therapeutisch wertvollste Verbindung 1-[6,7-Dimethoxy-3,4-dihydro-2H-isochinolin-1-on-2-yl]-3-[N-methyl-N-(2-(3,4-dimethoxy-phenyl)-ethyl)-amino]-propan und deren physiologisch verträgliche Säureadditionssalze beschrieben, welche wertvolle pharmakologische Eigenschaften aufweisen, insbesondere blutdrucksenkende und herzfrequenzsenkende Wirkungen.

Es wurde nun gefunden, daß die neuen Benzazepinderivate der allgemeinen Formel

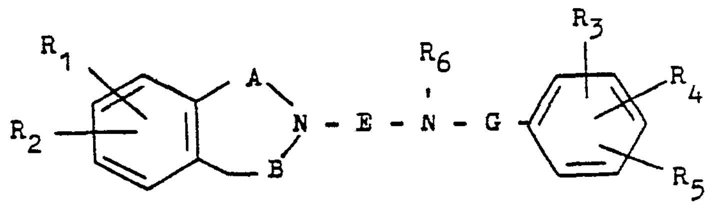

(I)

überlegene pharmakologische Eigenschaften aufweisen, nämlich bei einer längeren Wirkungsdauer und geringeren Nebenwirkungen insbesondere eine stärkere herzfrequenzsenkende Wirkung.

Gegenstand der vorliegenden Erfindung sind somit die neuen Benzazepinderivate der obigen allgemeinen Formel I, deren Säureadditionssalze, insbesondere deren physiologisch verträgliche Säureadditionssalze mit anorganischen oder organischen Säuren, Verfahren zu ihrer Herstellung und diese Verbindungen enthaltende Arzneimittel.

In der obigen allgemeinen Formel I bedeutet

A eine $-CH_2-CH_2-$, $-CH=CH-$, $-NH-CO-$, $-CH_2-CO-$ oder
5 5

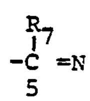 -Gruppe,

in der $R_7$ eine Alkylgruppe mit 1 bis 3 Kohlenstoffatomen darstellt, und B die Methylen- oder Carbonylgruppe oder

A die $-CO-CO-$Gruppe und B die Methylengruppe,

E eine gegebenenfalls durch eine Alkylgruppe mit 1 bis 1 bis 3 Kohlenstoffatomen substituierte Ethylen-, n-Propylen- oder n-Butylengruppe, eine durch eine Hydroxygruppe in 2-Stellung substituierte n-Propylengruppe oder eine durch eine Hydroxygruppe in 2- oder 3-Stellung substituierte n-Butylengruppe.

G eine gegebenenfalls durch eine Alkylgruppe mit 1 bis 3 Kohlenstoffatomen substituierte Methylen- oder Ethylengruppe,

$R_1$ und $R_2$, die gleich oder verschieden sein können, Hydroxygruppen, Alkyl-, Alkoxy- oder Phenylalkoxygruppen, in denen der Alkylteil jeweils 1 bis 3 Kohlenstoffatome enthalten kann, oder einer der Reste $R_1$ oder $R_2$ auch ein Wasserstoffatom oder $R_1$ zusammen mit $R_2$ eine Alkylendioxygruppe mit 1 oder 2 Kohlenstoffatomen,

$R_3$ und $R_4$, die gleich oder verschieden sein können, Wasserstoff- oder Halogenatome, Hydroxygruppen, Alkyl- oder Alkoxygruppen mit jeweils 1 bis 4 Kohlenstoffatomen, Trifluormethyl- oder Cyangruppen oder einer der Reste $R_3$ oder $R_4$ auch eine Nitrogruppe oder $R_3$ zusammen mit $R_4$, eine Alkylendioxygruppe mit 1 oder 2 Kohlenstoffatomen,

$R_5$ ein Wasserstoffatom, eine Alkyl-, Hydroxy-, Alkoxy-, Amino-, Alkylamino-, Dialkylamino-, Alkanoylamino-, Alkoxycarbonylamino- oder Bis(alkoxycarbonyl)aminogruppe, in denen der Alkylteil jeweils 1 bis 3 Kohlenstoffatome enthalten kann, oder eine durch eine Trifluormethylgruppe substituierte Methylamino- oder Ethylaminogruppe, und

$R_6$ ein Wasserstoffatom, eine Alkyl-, Phenylalkyl-, Alkanoyl- oder Alkoxycarbonylgruppe, in denen der Alkylteil jeweils 1 bis 3 Kohlenstoffatome enthalten kann, oder eine Alkenylgruppe mit 3 bis 5 Kohlenstoffatomen.

Für die bei der Definition der Reste $R_1$ bis $R_6$, E und G eingangs erwähnten Bedeutungen kommt beispielsweise

für $R_1$ die der Methyl-, Ethyl-, Propyl-, Isopropyl-, Hydroxy-, Methoxy-, Ethoxy-, Propoxy-, Isopropoxy-, Benzyloxy-, 1-Phenylethoxy-, 2-Phenylethoxy- oder 3-Phenylpropoxygruppe,

für $R_2$ die des Wasserstoffatoms, der Methyl-, Ethyl-, Propyl-, Isopropyl-, Hydroxy-, Methoxy-, Ethoxy-, Propoxy-, Isopropoxy-, Benzyloxy-, 1-Phenylethoxy-, 2-Phenylethoxy-, 2-Phenylpropoxy- oder 3-Phenylpropoxygruppe,

für $R_3$ bzw. $R_4$, die gleich oder verschieden sein können, die des Wasserstoff-, Fluor-, Chlor-, Brom- oder Jodatoms, der Methyl-, Ethyl-, Propyl-, Isopropyl-, n-Butyl-, Hydroxy-, Methoxy-, Ethoxy-, Propoxy-, Isopropoxy-, n-Butoxy-, Trifluormethyl- oder Cyangruppe oder für einen der Reste $R_3$ oder $R_4$ auch die der Nitrogruppe,

für $R_5$ die des Wasserstoffatoms, der Methyl-, Ethyl-, Propyl-, Isopropyl-, Hydroxy-, Methoxy-, Ethoxy-, Propoxy-, Isopropoxy-, Amino-, Methylamino-, Ethylamino-, Propylamino-, Isopropylamino-, Dimethylamino-, Diethylamino-, Dipropylamino-, Methyl-ethylamino-, Ethylpropylamino-, Methylpropylamino-, Formylamino-, Acetylamino-, Propionylamino-, Methoxycarbonylamino-, Ethoxycarbonylamino-, Propoxycarbonylamino-, Isopropoxycarbonylamino-, Bis(ethoxycarbonyl)amino- oder β,β,β-Trifluorethylaminogruppe,

für $R_6$ die des Wasserstoffatoms, der Methyl-, Ethyl-, Propyl-, Isopropyl-, Benzyl-, 1-Phenylethyl-, 2-Phenylethyl-, 3-Phenylpropyl-, Allyl-, Buten-2-yl-, Buten-3-yl-, Pentenyl-, Formyl-, Acetyl-, Propionyl-, Methoxycarbonyl-, Ethoxycarbonyl-, Propoxycarbonyl- oder Isopropoxycarbonylgruppe,

für $R_1$ zusammen mit $R_2$ und/oder $R_3$ zusammen mit $R_4$ jeweils die der Methylendioxy- oder Ethylendioxygruppe,

für E die der Ethylen-, n-Propylen-, n-Butylen-, 1-Methyl-ethylen-, 2-Ethyl-ethylen-, 1-Propyl-ethylen-, 1-Methyl-n-propylen-, 2-Methyl-n-propylen-, 1-Ethyl-n-propylen-, 3-Ethyl-n-propylen-, 2-Propyl-n-propylen-, 2-Methyl-n-butylen-, 2-Hydroxy-n-propylen-, 2-Hydroxy-n-butylen- oder 3-Hydroxy-n-butylen-gruppe und

für G die der Methylen-, Ethyliden-, Propyliden-, n-Butyliden-, 2-Methyl-propyliden-, Ethylen-, 1-Methyl-ethylen-, 2-Ethyl-ethylen-, 1-Propyl-ethylen- oder 2-Methyl-ethylengruppe in Betracht.

Bevorzugte Verbindungen der obigen allgemeinen Formel I sind diejenigen, in denen

A eine $—CH_2—CH_2$-, —CH=CH-, $—\underset{5}{NH}—CO$-, $—\underset{5}{CH_2}—CO$- oder

$$\begin{array}{l} CH_3 \\ | \\ -\underset{5}{C} = N\text{-Gruppe} \end{array}$$

und B die Methylen- oder Carbonylgruppe oder

A die —CO—CO-Gruppe und B die Methylengruppe,

E die Ethylen-, n-Propylen-, n-Butylen-, 2-Methyl-n-propylen- oder 2-Hydroxy-n-propylengruppe,

G die Methylen-, Ethylen- oder 1-Methyl-ethylengruppe,

$R_1$ eine Methyl-, Hydroxy-, Benzyloxy- oder Alkoxygruppe mit 1 bis 3 Kohlenstoffatomen,

$R_2$ ein Wasserstoffatom, eine Methyl-, Hydroxy- oder Methoxygruppe oder $R_1$ und $R_2$ zusammen die Methylendioxygruppe,

$R_3$ ein Wasserstoff-, Fluor-, Chlor- oder Bromatom, eine Trifluormethyl-, Nitro-, Alkyl- oder Alkoxygruppe mit jeweils 1 bis 4 Kohlenstoffatomen,

$R_4$ ein Wasserstoff-, Chlor- oder Bromatom, eine Methyl-, Methoxy- oder Cyangruppe oder $R_3$ und $R_4$ zusammen die Methylendioxygruppe,

$R_5$ ein Wasserstoffatom, eine Hydroxy-, Methoxy-, Amino-, Acetylamino-, Ethoxycarbonylamino-, Bis(ethoxycarbonyl)amino-, Dimethylamino- oder β,β,β-Trifluorethylaminogruppe und

$R_6$ ein Wasserstoffatom, eine Alkylgruppe mit 1 bis 3 Kohlenstoffatomen, eine Allyl-, Benzyl-, Acetyl- oder Ethoxycarbonylgruppe bedeuten, und deren Säureadditionssalze, insbesondere deren physiologisch verträgliche Säureadditionssalze mit anorganischen oder organischen Säuren.

Besonders bevorzugte Verbindungen der obigen allgemeinen Formel I sind diejenigen, in denen

A die $—CH_2—CH_2$-, —CH=CH- oder

$$\begin{array}{l} CH_3 \\ | \\ -\underset{5}{C} = N\text{-Gruppe} \end{array}$$

und B die Carbonylgruppe oder

A die —CH=CH-, $—\underset{5}{NH}—CO$- oder —CO—CO-Gruppe und B die Methylengruppe,

E die n-Propylengruppe,

G die Ethylengruppe,

$R_1$ die Methoxy- oder die Hydroxygruppe,

$R_2$ die Methoxygruppe,

$R_3$ die Methoxy- oder Trifluormethylgruppe, ein Chlor- oder Bromatom,

$R_4$ die Methoxygruppe, ein Wasserstoff-, Chlor- oder Bromatom oder $R_3$ und $R_4$ zusammen die Methylendioxygruppe,

$R_5$ ein Wasserstoffatom, eine Amino- oder Methoxygruppe und

$R_6$ ein Wasserstoffatom, die Methyl-, Ethyl-, n-Propyl- oder Allylgruppe bedeuten, und deren physiologisch verträgliche Säureadditionssalze mit anorganischen oder organischen Säuren.

Erfindungsgemäß erhält man die neuen Verbindungen nach folgenden Verfahren :

a) Umsetzung einer Verbindung der allgemeinen Formel

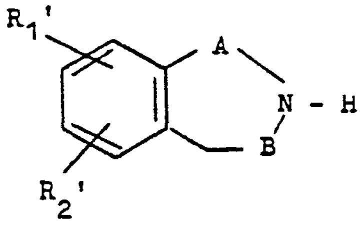

(II)

in der

A und B wie eingangs definiert sind,

$R_1'$ und $R_2'$ je eine durch einen Schutzrest geschützte Hydroxygruppe darstellen, z. B. eine Trimethylsilyloxy-, Acetoxy-, Benzyloxy- oder Tetrahydropyranyloxygruppe, oder die für $R_1$ und $R_2$ eingangs erwähnten Bedeutungen besitzen, mit einer Verbindung der allgemeinen Formel

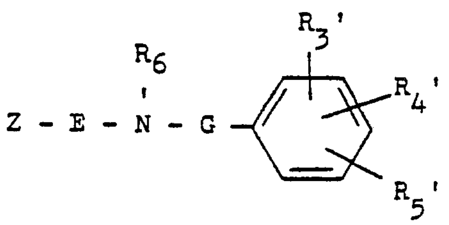

(III)

in der

$R_6$, E und G wie eingangs definiert sind,

$R_3'$ bis $R_5'$ je eine durch einen Schutzrest geschützte Hydroxygruppe darstellen, z. B. eine Trimethylsilyloxy-, Acetoxy-, Benzyloxy- oder Tetrahydropyranyloxygruppe, oder die für $R_3$ bis $R_5$ eingangs erwähnten Bedeutungen besitzen und

Z eine nukleophile Austrittsgruppe wie ein Halogenatom oder eine Sulfonyloxygruppe, z. B. ein Chlor-, Brom- oder Jodatom, die Methansulfonyloxy-, p-Toluolsulfonyloxy- oder Ethoxysulfonyloxygruppe, bedeutet oder mit deren gegebenenfalls im Reaktionsgemisch vorliegenden inneren quartären Salzes der allgemeinen Formel

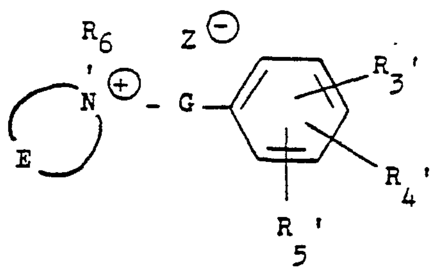

(IIIa)

in der E, G, Z, $R_3'$ bis $R_5'$ und $R_6$ wie eingangs definiert sind, und gegebenenfalls anschließende Abspaltung eines verwendeten Schutzrestes.

Die Umsetzung wird gegebenenfalls in einem Lösungsmittel oder Lösungsmittelgemisch, z. B. in Aceton, Dimethylformamid, Aceton/Dimethylformamid, Dimethylsulfoxid oder Chlorbenzol, und zweckmäßigerweise je nach der Reaktionsfähigkeit des Restes Z bei Temperaturen zwischen 0 und 150 °C, vorzugsweise jedoch bei der Siedetemperatur des verwendeten Lösungsmittels, durchgeführt. Vorteilhaft ist die Gegenwart eines säurebindenden Mittels, wie z. B. eines Alkoholats wie Natriummethylat, eines Alkalihydroxyds wie Natriumhydroxid, eines Alkalicarbonats wie Kaliumcarbonat, eines Alkaliamids wie Natriumamid, eines Alkalihydrids wie Natriumhydrid oder einer tertiären organischen Base wie Triethylamin oder Pyridin, oder eines Reaktionsbeschleunigers wie beispielsweise Kaliumjodid.

Die gegebenenfalls anschließende Abspaltung eines verwendeten Schutzrestes erfolgt vorzugsweise hydrolytisch in einem wässrigen Lösungsmittel, z. B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 °C, vorzugsweise bei der Siedetemperatur des Reaktionsgemisches. Die Abspaltung eines Benzylrestes kann jedoch auch hydrogenolytisch erfolgen, z. B. mit Wasserstoff in Gegenwart eines Katalysators wie Palladium/Kohle, in einem Lösungsmittel wie Methanol, Ethanol, Essigsäureethylester oder Eisessig gegebenenfalls unter Zusatz einer Säure wie Salzsäure bei Temperaturen zwischen 0 und

50 °C, vorzugsweise jedoch bei Raumtemperatur, und einem Wasserstoffdruck von 1 bis 7 bar, vorzugsweise jedoch von 3 bis 5 bar.

b) Zur Herstellung von Verbindungen der allgemeinen Formel I, in der $R_6$ ein Wasserstoffatom, eine Alkenylgruppe mit 3 bis 5 Kohlenstoffatomen, eine Alkyl- oder Phenylalkylgruppe mit jeweils 1 bis 3 Kohlenstoffatomen im Alkylteil darstellt:

Umsetzung einer Verbindung der allgemeinen Formel

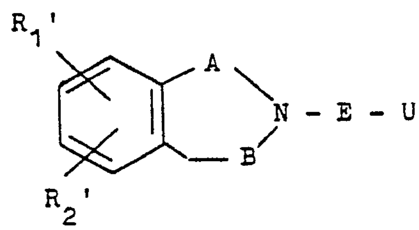

(IV)

mit einer Verbindung der allgemeinen Formel

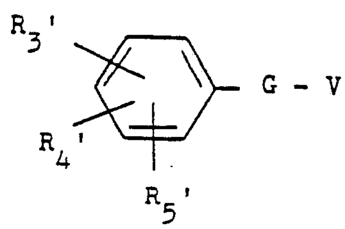

(V)

in denen

A, B, E + G wie eingangs definiert sind,

$R_1'$ bis $R_5'$ je eine durch einen Schutzrest geschützte Hydroxygruppe, z. B. je eine Trimethylsilyloxy-, Acetoxy-, Benzyloxy- oder Tetrahydropyranyloxygruppe, darstellen, oder die für $R_1$ bis $R_5$ eingangs erwähnten Bedeutungen besitzen, einer der Reste U oder V die $R_6'$—NH-Gruppe darstellt, wobei $R_6'$ eine Wasserstoffatom, eine Alkenylgruppe mit 3 bis 5 Kohlenstoffatomen, eine Alkyl- oder Phenylalkylgruppe mit jeweils 1 bis 3 Kohlenstoffatomen bedeutet, der andere der Reste U oder V eine nukleophile Austrittsgruppe wie ein Halogenatom oder eine Sulfonyloxygruppe, z. B. ein Chlor-, Brom- oder Jodatom, die Methansulfonyloxy-, p-Toluolsulfonyloxy- oder Ethoxysulfonyloxygruppe oder der Rest U zusammen mit einem β-ständigen Wasserstoffatom des Restes E ein Sauerstoffatom und V eine $R_6'$—NH-Gruppe darstellen, wobei $R_6'$ wie vorstehend erwähnt definiert ist, und gegebenenfalls anschließende Abspaltung eines verwendeten Schutzrestes.

Die Umsetzung wird zweckmäßigerweise in einem Lösungsmittel oder Lösungsmittelgemisch wie Aceton, Diethylether, Methylformamid, Dimethylformamid, Dimethylsulfoxid, Benzol, Chlorbenzol, Tetrahydrofuran, Benzol/Tetrahydrofuran, Dioxan oder in einem Überschuß der eingesetzten Verbindungen der allgemeinen Formeln IV und/oder V und gegebenenfalls in Gegenwart eines säurebindenden Mittels, z. B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid, eines Alkalihydrids wie Natriumhydrid, einer tertiären organischen Base wie Triethylamin oder Pyridin wobei die letzteren gleichzeitig auch als Lösungsmittel dienen können, oder eines Reaktionsbeschleunigers wie Kaliumjodid je nach der Reaktionsfähigkeit des nucleophil austauschbaren Restes zweckmäßigerweise bei Temperaturen zwischen 0 und 150 °C, vorzugsweise bei Temperaturen zwischen 50 und 120 °C, z. B. bei der Siedetemperatur des verwendeten Lösungsmittels, durchgeführt. Die Umsetzung kann jedoch auch ohne Lösungsmittel durchgeführt werden. Besonders vorteilhaft wird die Umsetzung jedoch in Gegenwart einer tertiären organischen Base oder eines Überschusses des eingesetzten Amins der allgemeinen Formel V durchgeführt.

Die gegebenenfalls anschließende Abspaltung eines verwendeten Schutzrestes erfolgt vorzugsweise hydrolytisch in einem wässrigen Lösungsmittel, z. B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 °C, vorzugsweise bei der Siedetemperatur des Reaktionsgemisches. Die Abspaltung eines Benzylrestes kann jedoch auch hydrogenolytisch erfolgen, z. B. mit Wasserstoff in Gegenwart eines Katalysators wie Palladium/Kohle, in einem Lösungsmittel wie Methanol, Ethanol, Essigsäureethylester oder Eisessig gegebenenfalls unter Zusatz einer Säure wie Salzsäure bei Temperaturen zwischen 0 und 50 °C, vorzugsweise jedoch bei Raumtemperatur, und einem Wasserstoffdruck von 1 bis 7 bar, vorzugsweise jedoch von 3 bis 5 bar.

c) Zur Herstellung von Verbindungen der allgemeinen Formel I, in der $R_6$ ein Wasserstoffatom, eine

Alkenylgruppe mit 3 bis 5 Kohlenstoffatomen, eine Alkyl- oder Phenylalkylgruppe mit jeweils 1 bis 3 Kohlenstoffatomen im Alkylteil darstellt :

Umsetzung einer Verbindung der allgemeinen Formel

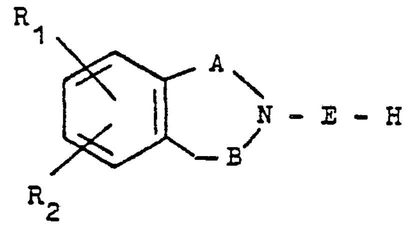

(VI)

in der A, B, E, $R_1$ und $R_2$ wie eingangs definiert sind, wobei jedoch im Rest E zwei Wasserstoffatome in einer $—CH_2$- oder $CH_3$-Gruppe des Restes E durch ein Sauerstoffatom ersetzt sind, mit einem Amin der allgemeinen Formel

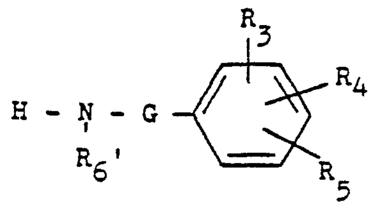

(VII)

in der G und $R_3$ bis $R_5$ wie eingangs definiert sind und $R_6'$ ein Wasserstoffatom, eine Alkenylgruppe mit 3 bis 5 Kohlenstoffatomen, eine Alkyl- oder Phenylalkylgruppe mit jeweils 1 bis 3 Kohlenstoffatomen im Alkylteil darstellt, in Gegenwart eines Reduktionsmittels.

Die Umsetzung wird zweckmaßigerweise in einem geeigneten Lösungsmittel oder Lösungsmittelgemisch wie Methanol, Ethanol, Ethanol/Essigsäureethylester oder Dioxan bei Temperaturen zwischen 0 und 100 °C, vorzugsweise jedoch bei Temperaturen zwischen 20 und 80 °C, durchgeführt.

Besonders vorteilhaft wird die reduktive Aminierung in Gegenwart eines komplexen Metallhydrids wie Lithium- oder Natriumcyanborhydrid vorzugsweise bei einem pH-Wert von 6-7 und bei Raumtemperatur oder zur Herstellung von Verbindungen der allgemeinen Formel I, in der $R_6$ ein Wasserstoffatom darstellt, in Gegenwart eines Hydrierungskatalysators, z. B. mit Wasserstoff in Gegenwart von Palladium/Kohle bei einem Wasserstoffdruck von 5 bar, durchgeführt. Hierbei können gegebenenfalls vorhandene Benzylgruppen gleichzeitig hydrogenolytisch abgespalten und/oder Doppelbindungen aufhydriert werden.

d) Zur Herstellung von Verbindungen der allgemeinen Formel I, in der $R_6$ ein Wasserstoffatom, eine Alkenylgruppe mit 3 bis 5 Kohlenstoffatomen, eine Alkyl- oder Phenylalkylgruppe mit jeweils 1 bis 3 Kohlenstoffatomen im Alkylteil darstellt :

Umsetzung einer Verbindung der allgemeinen Formel

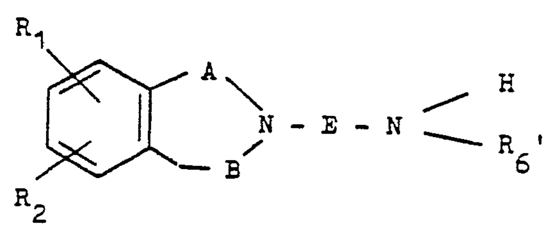

(VIII)

in der A, B, E, $R_1$ und $R_2$ wie eingangs definiert sind und $R_6'$ ein Wasserstoffatom, eine Alkenylgruppe mit 3 bis 5 Kohlenstoffatomen, eine Alkyl- oder Phenylalkylgruppe mit jeweils 1 bis 3 Kohlenstoffatomen im Alkylteil bedeutet, mit einer Verbindung der allgemeinen Formel

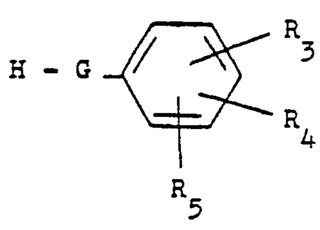

(IX)

in der $R_3$ bis $R_5$ und G wie eingangs definiert sind, wobei jedoch im Rest G zwei Wasserstoffatome in einer —$CH_2$- oder $CH_3$-Gruppe des Restes G durch ein Sauerstoffatom ersetzt sind, in Gegenwart eines Reduktionsmittels.

Die Umsetzung wird zweckmäßigerweise in einem geeigneten Lösungsmittel oder Lösungsmittelgemisch wie Methanol, Ethanol, Ethanol/Essigsäureethylester oder Dioxan bei Temperaturen zwischen 0 und 100 °C, vorzugsweise jedoch bei Temperaturen zwischen 20 und 80 °C, durchgeführt.

Besonders vorteilhaft wird die reduktive Aminierung in Gegenwart eines komplexen Metallhydrids wie Lithium- oder Natriumcyanborhydrid vorzugsweise bei einem pH-Wert von 6-7 und bei Raumtemperatur oder zur Herstellung von Verbindungen der allgemeinen Formel I, in der $R_6$ ein Wasserstoffatom darstellt, in Gegenwart von Palladium/Kohle bei einem Wasserstoffdruck von 5 bar, durchgeführt. Hierbei können gegebenenfalls vorhandene Benzylgruppen gleichzeitig hydrogenolytisch abgespalten und/oder Doppelbindungen aufhydriert werden.

e) Zur Herstellung von Verbindungen der allgemeinen Formel I, in der A die —NH—CO-Gruppe, E eine gegebenenfalls durch eine Alkylgruppe mit 1 bis 3 Kohlenstoffatomen substituierte Ethylen-, n-Propylen- oder n-Butylengruppe und $R_6$ kein Wasserstoffatom darstellen :

Umsetzung einer Verbindung der allgemeinen Formel

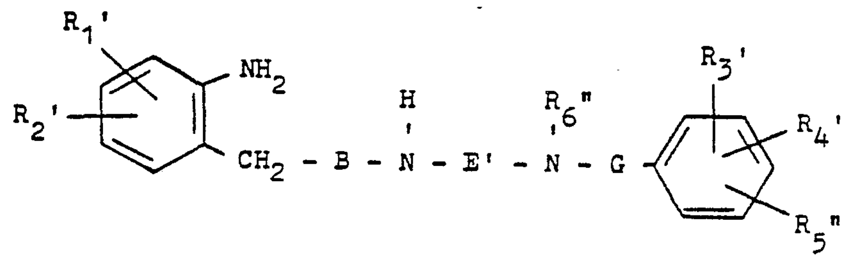

(X)

in der

B und G wie eingangs definiert sind,

E' eine gegebenenfalls durch eine Alkylgruppe mit 1 bis 4 Kohlenstoffatomen substituierte Ethylen-, n-Propylen- oder n-Butylengruppe,

$R_1'$ bis $R_4'$ je eine durch einen Schutzrest geschützte Hydroxygruppe darstellen, z. B. eine Trimethylsilyloxy-, Acetoxy-, Benzyloxy- oder Tetrahydropyranyloxygruppe, oder die für $R_1$ bis $R_4$ eingangs erwähnten Bedeutungen besitzen,

$R_5''$ eine durch einen Schutzrest geschützte Hydroxy-, Amino- oder Alkylaminogruppe darstellt oder mit Ausnahme der Amino- oder Alkylaminogruppe die für $R_5$ eingangs erwähnten Bedeutungen besitzt und

$R_6''$ mit Ausnahme von Wasserstoff die für $R_6$ eingangs erwähnten Bedeutungen besitzt oder eine Schutzgruppe für eine Aminogruppe darstellt, mit einem Kohlensäurederivat der allgemeinen Formel

$$W—CO—W \qquad (XI)$$

in der W, die gleich oder verschieden sein können, jeweils eine nukleophile Austrittsgruppe wie ein Chlor- oder Bromatom, eine gegebenenfalls durch Halogenatome substituierte Alkoxygruppe mit 1 bis 3 Kohlenstoffatomen oder eine Imidazolyl-(2)-gruppe bedeuten, und gegebenenfalls anschließende Abspaltung eines verwendeten Schutzrestes.

Die Umsetzung wird zweckmäßigerweise in einem Lösungsmittel oder Lösungsmittelgemisch wie Methylenchlorid, Tetrachlorkohlenstoff, Benzol, Tetrahydrofuran, Benzol/Tetrahydrofuran, Dioxan oder Acetonitril zweckmäßigerweise bei Temperaturen zwischen 0 und 150 °C, vorzugsweise jedoch bei der Siedetemperatur des verwendeten Lösungsmittels, z. B. bei Temperaturen zwischen 40 und 100 °C, und gegebenenfalls in Gegenwart eines säurebindenden Mittels wie Kaliumcarbonat, Natriumhydroxid, Kaliumhydroxid, Pyridin oder Triethylamin, wobei die letzteren gleichzeitig auch als Lösungsmittel dienen können, durchgeführt. Die Umsetzung kann jedoch auch ohne Lösungsmittel durchgeführt werden. Bedeutet in einer eingesetzten Verbindung der allgemeinen Formel XI mindestens einer der Reste W eine Alkoxygruppe mit 1 bis 3 Kohlenstoffatomen, so wird die Umsetzung vorzugsweise in einem Überschuß des eingesetzten Esters als Lösungsmittel durchgeführt.

Die gegebenenfalls anschließende Abspaltung eines verwendeten Schutzrestes erfolgt vorzugsweise hydrolytisch in einem wässrigen Lösungsmittel, z. B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 °C, vorzugsweise bei der Siedetemperatur des Reaktionsgemisches. Die Abspaltung eines Benzylrestes kann jedoch auch hydrogenolytisch erfolgen, z. B. mit Wasserstoff in Gegenwart eines Katalysators wie Palladium/Kohle, in einem Lösungsmittel wie Methanol, Ethanol, Essigsäureethylester oder Eisessig gegebenenfalls unter Zusatz einer Säure wie Salzsäure bei Temperaturen zwischen 0 und

50 °C, vorzugsweise jedoch bei Raumtemperatur, und einem Wasserstoffdruck von 1 bis 7 bar, vorzugsweise jedoch von 3 bis 5 bar.

f) Zur Herstellung von Verbindungen der allgemeinen Formel I, in der A die $—CH_2—CH_2$-Gruppe, $R_1$ und $R_2$ keine Benzyloxygruppe, $R_3$ oder $R_4$ keine Nitrogruppe und $R_6$ keine Benzylgruppe oder keine Alkenylgruppe mit 3 bis 5 Kohlenstoffatomen darstellen :

Hydrierung einer Verbindung der allgemeinen Formel

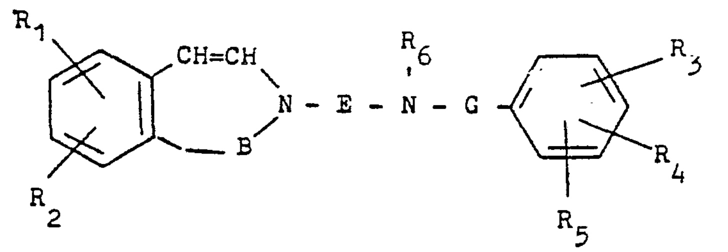

(XII)

in der $R_1$ bis $R_5$, B, E und G wie eingangs definiert sind, wobei $R_3$ oder $R_4$ keine Nitrogruppe darstellen können, falls $R_5$ eine Aminogruppe bedeutet.

Die Hydrierung wird in einem Lösungsmittel oder Lösungsmittelgemisch wie Methanol, Ethanol, Essigsäureethylester oder Eisessig mit katalytisch angeregtem Wasserstoff, z. B. mit Wasserstoff in Gegenwart von Platin oder Palladium/Kohle, bei einem Wasserstoffdruck von 1 bis 7 bar, vorzugsweise jedoch von 3 bis 5 bar, und bei Temperaturen zwischen 0 und 75 °C, vorzugsweise jedoch bei Temperaturen zwischen 20 und 50 °C, durchgeführt. Bedeutet in einer Verbindung der allgemeinen Formel XII $R_6$ die Benzylgruppe, so wird diese während der Hydrierung gleichzeitig durch ein Wasserstoffatom ersetzt oder bedeuten $R_1$ und/oder $R_2$ eine Benzyloxygruppe, so werden diese während der Hydrierung jeweils in eine Hydroxygruppe übergeführt.

g) Umsetzung einer Verbindung der allgemeinen Formel

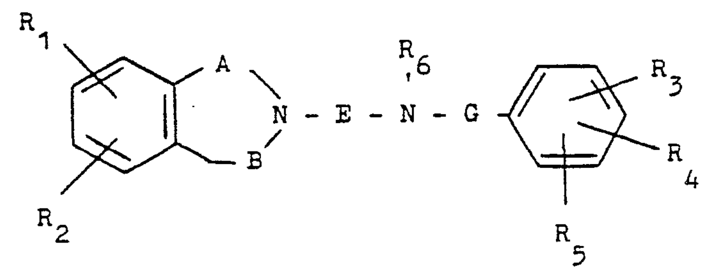

(XIII)

in der

$R_1$ bis $R_6$, A, B, E und G wie eingangs definiert sind, wobei jedoch mindestens einer der Reste $R_1$ bis $R_4$ eine Hydroxygruppe, $R_5$ eine Hydroxygruppe, eine Amino- oder Alkylaminogruppe mit 1 bis 3 Kohlenstoffatomen oder $R_6$ ein Wasserstoffatom darstellen muß, mit einer Verbindung der allgemeinen Formel

$$R_8—X \qquad (XIV)$$

in der

$R_8$ eine Alkylgruppe mit 1 bis 3 Kohlenstoffatomen oder auch eine Alkenylgruppe mit 3 bis 5 Kohlenstoffatomen, wenn $R_6$ ein Wasserstoffatom darstellt, oder auch eine durch eine Phenylgruppe substituierte Alkylgruppe mit 1 bis 3 Kohlenstoffatomen, wenn $R_1$ oder $R_2$ die Hydroxygruppe und/oder $R_6$ ein Wasserstoffatom darstellen, und

X eine nukleophile Austrittsgruppe wie ein Halogenatom oder eine Sulfonyloxygruppe, z. B. ein Chlor-, Brom- oder Jodatom, eine Methansulfonyloxy-, p-Toluolsulfonyloxy- oder Methoxy-sulfonyloxygruppe, oder, falls mindestens einer der Reste $R_1$ bis $R_5$ eine Hydroxygruppe darstellt, X zusammen mit einem α-ständigen Wasserstoffatom des Restes $R_8$ eine Diazogruppe oder auch, falls $R_6$ ein Wasserstoffatom oder $R_5$ eine Amino- oder Alkylaminogruppe darstellen, ein Sauerstoffatom bedeuten.

Die Umsetzung wird zweckmäßigerweise in einem Lösungsmittel oder Lösungsmittelgemisch wie Diethylether, Methanol, Aceton, Tetrahydrofuran, Dioxan, Acetonitril, Pyridin oder Dimethylformamid gegebenenfalls in Gegenwart einer Base wie Kaliumcarbonat, Kaliumhydroxid, Kalium-tert.butylat oder Natriumhydrid bei Temperaturen zwischen 0 und 150 °C, vorzugsweise jedoch bei Temperaturen zwischen 20 und 120 °C, durchgeführt.

Bedeutet X eine nukleophile Austrittsgruppe, so wird die Umsetzung vorzugsweise mit einem Alkylierungsmittel wie Methyljodid, Dimethylsulfat, Ethyljodid, Diethylsulfat, Propylbromid, Allylbromid,

Benzylchlorid, Phenylethylbromid oder p-Toluolsulfonsäureisopropylester in Gegenwart eines säurebindenden Mittel bei Temperaturen zwischen 20 und 80 °C durchgeführt. Die Umsetzung kann jedoch auch ohne Lösungsmittel durchgeführt werden.

Bedeutet X zusammen mit einem α-ständigen Wasserstoffatom des Restes $R_8$ eine Diazogruppe, so wird zur Alkylierung einer Hydroxygruppe die Umsetzung vorzugsweise mit Diazomethan oder Diazoethan bei Temperaturen zwischen 0 und 30 °C durchgeführt, oder ein Sauerstoffatom, so wird zur Alkylierung des Stickstoffatoms die Umsetzung in Gegenwart eines Reduktionsmittels bei Temperaturen zwischen 0 und 120 °C, z. B. mit Ameisensäure bei Temperaturen zwischen 80 und 110 °C oder mit Natriumcyanborhydrid bei Raumtemperatur und einem pH-Wert von 6-7 durchgeführt.

h) Zur Herstellung von Verbindungen der allgemeinen Formel I, in der A keine -CH=CH-Gruppe, $R_3$ ein Chlor- oder Bromatom und $R_5$ eine Amino- oder Alkylaminogruppe darstellen :

Halogenierung einer Verbindung der allgemeinen Formel

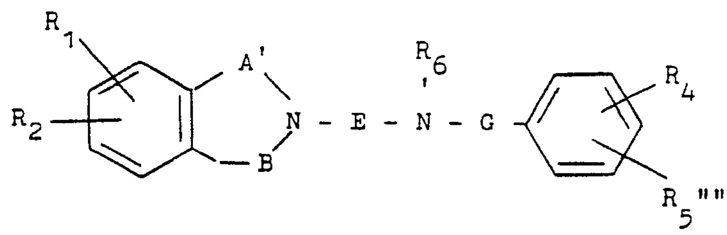

(XV)

in der

$R_1$, $R_2$, $R_4$, $R_6$, B, E und G wie eingangs definiert sind, A' mit Ausnahme der -CH=CH-Gruppe die für A eingangs erwähnten Bedeutungen besitzt und

$R_5$"" eine Amino- oder Alkylaminogruppe mit jeweils 1 bis 3 Kohlenstoffatomen in jedem Alkylteil darstellt.

Die Umsetzung wird einem Halogenierungsmittel, z. B. mit Chlor, Brom, Tribromphenolbrom oder Phenyljoddichlorid, vorzugsweise in einem Lösungsmittel oder Lösungsmittelgemisch, z. B. in 50 bis 100 %iger Essigsäure oder in Tetrahydrofuran in Gegenwart einer tertiären organischen Base, gegebenenfalls in Gegenwart einer Schwermetallverbindung wie Quecksilber (II)-oxid und zweckmäßigerweise bei Temperaturen zwischen 0 und 50 °C durchgeführt. Pro Mol einer Verbindung der allgemeinen Formel XV, welche als Base oder auch als Salz, z. B. als Mono-, Di- oder Trihydrochlorid, eingesetzt werden kann, werden zweckmäßigerweise 1 oder 2 Mol an Halogenierungsmittel oder ein geringer Überschuß verwendet. Falls bei der Umsetzung ein halogenwasserstoffsaures Salz entsteht, kann dieses als solches direkt isoliert oder es kann gewünschtenfalls über die Base weitere gereinigt werden.

i) Zur Herstellung von Verbindungen der allgemeinen Formel I, in der A die —COCO-Gruppe, E eine gegebenenfalls durch eine Alkylgruppe mit 1 bis 3 Kohlenstoffatomen substituierte Ethylen-, n-Propylen- oder n-Butylengruppe und B die —$CH_2$-Gruppe darstellen :

Oxidation einer Verbindung der allgemeinen Formel

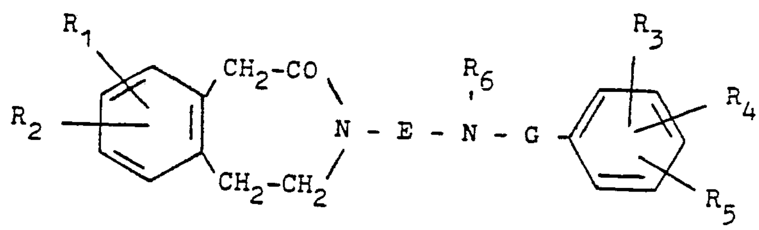

(XVI)

in der $R_1$ bis $R_6$, E und G wie eingangs definiert sind.

Die Oxidation wird vorzugsweise mit einem Oxidationsmittel wie Kaliumpermanganat, Selendioxid oder Natriumdichromat in einem geeigneten Lösungsmittel oder Lösungsmittelgemisch wie Wasser, Wasser/Dioxan, Eisessig, Wasser/Essigsäure oder Acetanhydrid bei Temperaturen zwischen 0 und 100 °C, vorzugsweise bei Temperaturen zwischen 20 und 80 °C, durchgeführt.

Erhält man erfindungsgemäß eine Verbindung der allgemeinen Formel I, in der B die Carbonylgruppe darstellt, so kann diese mittels Reduktion in eine entsprechende Verbindung der allgemeinen Formel I, in der B die Methylengruppe darstellt, übergeführt werden und/oder

erhält man eine Verbindung der allgemeinen Formel I, in der

A keine —CH=CH- oder

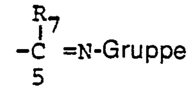

und $R_6$ eine Benzyl- oder 1-Phenylethylgruppe darstellen, so kann diese mittels katalytischer Hydrierung in eine entsprechende Verbindung der allgemeinen Formel I, in der $R_6$ ein Wasserstoffatom darstellt, übergeführt werden und/oder

erhält man eine Verbindung der allgemeinen Formel I, in der $R_3$ oder $R_4$ eine Nitrogruppe darstellt, so kann diese mittels Reduktion in eine entsprechende Verbindung der allgemeinen Formel I, in der $R_5$ eine Aminogruppe dartstellt, übergeführt werden und/oder

erhält man eine Verbindung der allgemeinen Formel I, in der $R_6$ ein Wasserstoffatom und/oder $R_5$ eine Aminogruppe darstellt, so kann diese mittels Acylierung in eine entsprechende Verbindung der allgemeinen Formel I, in der $R_6$ eine Alkanoyl- oder Alkoxycarbonylgruppe und/oder $R_5$ eine Alkonoylamino-, Alkoxycarbonylamino- oder Bis (alkoxycarbonyl)aminogruppe darstellen, wobei der Alkylteil jeweils 1 bis 3 Kohlenstoffatome enthalten kann, übergeführt werden und/oder

erhält man eine Verbindung der allgemeinen Formel I, in der $R_5$ eine Aminogruppe, $R_6$ kein Wasserstoffatom, $R_3$ und $R_4$ jeweils keine Cyangruppe darstellen, so kann diese über ein entsprechendes Diazoniumsalz in eine entsprechende Verbindung der allgemeinen Formel I, in der $R_5$ ein Wasserstoffatom, eine Hydroxy- oder Alkoxygruppe oder $R_5$ ein Wasserstoffatom und $R_3$ ein Halogenatom oder die Cyangruppe bedeuten, übergeführt werden.

Die nachträgliche Reduktion wird vorzugsweise mit einem Metallhydrid wie Lithiumaluminiumhydrid oder Diboran in einem Lösungsmittel wie Diethylether, Tetrahydrofuran, oder Dioxan bei Temperaturen zwischen 0 und 100 °C, vorzugsweise jedoch bei Temperaturen zwischen 30 und 85 °C, durchgeführt.

Die nachträgliche Reduktion bzw. katalytische Hydrierung wird in einem Lösungsmittel wie Methanol, Ethanol, Essigsäureethylester oder Eisessig mit Wasserstoff in Gegenwart eines Katalysators wie Platin oder Palladium/Kohle bei einem Wasserstoffdruck von 1 bis 7 bar, vorzugsweise jedoch von 3 bis 5 bar, und bei Temperaturen zwischen 0 und 75 °C, vorzugsweise jedoch bei Temperaturen zwischen 20 und 50 °C, durchgeführt. Hierbei werden vorhandene Nitro- und Cyangruppen gleichzeitig mitreduziert.

Die nachträgliche Acylierung wird beispielsweise mit Acetylchlorid, Acetanhydrid, Propionsäureanhydrid oder einem entsprechenden Chlorameisensäureester, z. B. Chlorameisensäureethylester, welcher gleichzeitig als Lösungsmittel dienen kann, gegebenenfalls in einem Lösungsmittel dienen kann, gegebenenfalls in einem Lösungsmittel wie Wasser/Tetrahydrofuran, Diethylether, Tetrahydrofuran oder Methylenchlorid, gegebenenfalls in Gegenwart einer Base wie Triethylamin oder Pyridin, wobei eine tertiäre organische Base gleichzeitig auch als Lösungsmittel dienen kann, bei Temperaturen zwischen 0 und 100 °C, vorzugsweise jedoch bei Raumtemperatur, durchgeführt. Die Umsetzung kann auch ohne Lösungsmittel durchgeführt werden.

Die nachträgliche Umsetzung eines Diazoniumsalzes, z. B. des Fluoroborats, des Hydrosulfates in Schwefelsäure, des Hydrochlorids oder des Hydrojodids, erforderlichenfalls in Gegenwart von Kupfer oder eines entsprechenden Kupfer-(I)-Salzes wie Kupfer-(I)-chlorid/Salzssäure, Kupfer-(I)-bromid/Bromwasserstoffsäure oder Trinatrium-kupfer-(I)-tetracyanid bei pH 7 wird bei leicht erhöhten Temperaturen, z. B. bei Temperaturen zwischen 15 und 100 °C, durchgeführt; die nachträgliche Umsetzung mit unterphosphoriger Säure wird vorzugsweise bei − 5 bis 0 °C durchgeführt. Das hierzu erforderliche Diazoniumsalz wird zweckmäßigerweise in einem geeigneten Lösungsmittel, z. B. in Wasser/Salzsäure, Methanol/Salzsäure, Ethanol/Salzsäure oder Dioxan/Salzsäure, durch Diazotierung einer entsprechenden Aminoverbindung mit einem Nitrit, z. B. Natriumnitrit oder einem Ester der salpetrigen Säure, bei niederen Temperaturen, z. B. bei Temperaturen zwischen − 10 und 5 °C, hergestellt.

Die erhaltenen Verbindungen der allgemeinen Formel I lassen sich ferner in ihre Säureadditionssalze, insbesondere in ihre physiologisch verträgliche Säureadditionssalze mit anorganischen oder organischen Säuren überführen. Als Säuren kommen hierbei beispielsweise Salzsäure, Bromwasserstoffsäure, Schwefelsäure, Phosphorsäure, Essigsäure, Milchsäure, Zitronensäure, Weinsäure, Bernsteinsäure, Maleinsäure oder Fumarsäure in Betracht.

Die als Ausgangsstoffe verwendeten Verbindungen der allgemeinen Formeln II bis XVI sind teilweise literaturbekannt bzw. man erhält sie nach an sich bekannten Verfahren.

So erhält man beispielsweise eine Ausgangsverbindung der allgemeinen Formeln IV und VIII durch Umsetzung eines entsprechenden Benzazepins mit einer entsprechenden Halogenverbindung und gegebenenfalls durch anschließende Umsetzung mit einem entsprechenden Amin. Das hierfür erforderliche Benzazepin der allgemeinen Formen II erhält man durch Cyclisierung einer entsprechenden Verbindung, z. B. durch Cyclisierung einer Verbindung der allgemeinen Formel

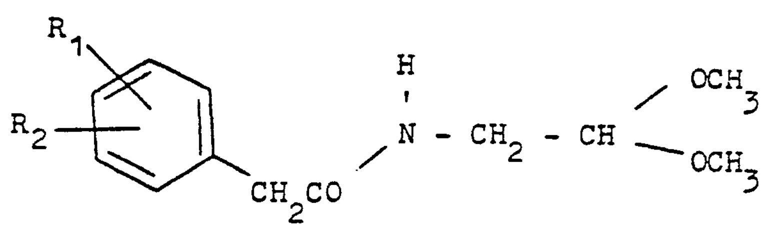

(XVII)

oder durch Ringschluß einer entsprechenden o-Aminoverbindung und gegebenenfalls anschließender katalytischer Hydrierung und/oder Reduktion der Carbonylgruppe beispielsweise mit Natriumborhydrid/Eisessig (siehe EP-A1 0 007 070) und/oder Oxidation, z. B. mit Selendioxid.

Eine als Ausgangsverbindung verwendete Verbindung der allgemeinen Formel VI erhält man beispielsweise durch Umsetzung eines entsprechenden Benzazepins mit einem entsprechenden Halogenacetal und anschließender Hydrolyse.

Eine als Ausgangsstoff verwendete Verbindung der allgemeinen Formel X erhält man beispielsweise durch Reduktion einer entsprechenden Nitroverbindung.

Eine als Ausgangsstoff verwendete Verbindung der allgemeinen Formeln XII, XIII, XV oder XVI erhält man vorzugsweise durch Umsetzung einer entsprechenden Halogenverbindung mit einem entsprechenden Amin und gegebenenfalls anschließende Abspaltung von Schutzresten, die zum Schutze von Hydroxygruppen verwendet werden.

Wie bereits eingangs erwähnt, weisen die neuen Verbindungen der allgemeinen Formel I und deren physiologisch verträgliche Säureadditionssalze mit anorganischen oder organischen Säuren wertvolle pharmakologische Eigenschaften auf, insbesondere bei geringen Nebenwirkungen, z. B. einer geringen antimuskarinischen, eine lang anhaltende herzfrequenzsenkende Wirkung sowie eine Herabsetzung des $O_2$-Bedarfs des Herzens.

Beispielsweise wurden die Verbindungen

A = 1-[7,8-Dimethoxy-1,3,4,5-tetrahydro-2H-3-benzazepin-2-on 3-yl]-3-[N-methyl-N-(2-(3,4-dimethoxyphenyl)-ethyl)-amino]-propan-dihydrochlorid,

B = 1-[7,8-Dimethoxy-1,3-dihydro-2H-3-benzazepin-2-on-3-yl]-3-[N-methyl-N-(2-(3,4-dimethoxy-phenyl)-ethyl)-amino]-propan-hydrochlorid,

C = 1-[7,8-Dimethoxy-1,3,4,5-tetrahydro-2H-3-benzazepin-1,2-dion-3-yl]-3-[N-methyl-(2-(3,4-dimethoxy-phenyl)-ethyl)-amino]-propan-hydrochlorid,

D = 1-[7,8-Dimethoxy-1,3,4,5-tetrahydro-2H-1,3-benzodiazepin-2-on-3-yl]-3-[N-methyl-(2-(3,4-dimethoxy-phenyl)-ethyl)-amino]-propan,

E = 1-[7,8-Dimethoxy-1,3,4,5-tetrahydro-2H-3-benzazepin-2-on-3-yl]-3-[N-(2-(3,4-dimethoxy-phenyl)-ethyl)-amino]-propan-hydrochlorid,

F = 1-[7,8-Dimethoxy-1,3,4,5-tetrahydro-2H-3-benzazepin-2-on-3-yl]-3-[N-methyl-N-(2-(4-amino-3,5-dichlor-phenyl)-ethyl)-amino]-propan,

G = 1-[7,8-Dimethoxy-1,3-dihydro-2H-3-benzazepin-2-on-3-yl]-3-[N-methyl-N-(2-(4-methoxy-phenyl)-ethyl)-amino]-propan-hydrochlorid,

H = 1-[7,8-Dimethoxy-1,3,4,5-tetrahydro-2H-3-benzazepin-2-on-3-yl]-3-[N-methyl-N-(2-(4-methoxy-phenyl)-ethyl)-amino]-propan-hydrochlorid,

I = 1-[7,8-Methylendioxy-1,3,4,5-tetrahydro-2H-3-benzazepin-2-on-3-yl]-3-[N-methyl-N-(2-(3,4-dimethoxy-phenyl)-ethyl)-amino]-propan-hydrochlorid,

J = 1-[7,8-Dimethoxy-1,3,4,5-tetrahydro-2H-3-benzazepin-2-on-3-yl]-3-[N-allyl-N-(2-(3,4-dimethoxy-phenyl)-ethyl)-amino]-propan-hydrochlorid und

K = 1-[7,8-Dimethoxy-1,3-dihydro-2H-3-benzazepin-2-on-3-yl]-3-[N-methyl-N-(2-(3,4-methylendioxy-phenyl)-ethyl)-amino]-propan-hydrochlorid

im Vergleich zu

L = 1-[7,8-Dimethoxy-1,2,3,4-tetrahydro-5H-2-benzazepin-1-on-2-yl]-3-[N-methyl-N-(2-(3,4-dimethoxy-phenyl)-ethyl)-amino]-propan-hydrochlorid

auf ihre biologischen Eigenschaften wie folgt untersucht :

1. Wirkung auf die Herzfrequenz an Katzen :

Die Wirkung der zu untersuchenden Substanzen auf die Herzfrequenz wurde pro Dosis an 7 Katzen beiderlei Geschlechts mit einem durchschnittlichen Gewicht von 2,5-3,5 kg untersucht. Hierzu wurden die Katzen mit Nembutal (30 mg/kg i. p.) und Chloralose-Urethan (40 mg/ml Chloralose + 200 mg/ml Urethan nach Bedarf) narkotisiert. Die zu untersuchende Substanz wurde in wäßriger Lösung in die Vene saphena bzw. Duodenum injiziert.

Die Herzfrequenz wurde vor und nach Substanzgabe mit Hilfe eines Grass-Tachographen aus dem Elektrocardiogramm (Brustwandableitung) auf einem Grass-Polygraphen registriert :

Die nachfolgende Tabelle enthält die gefundenen Werte :

(Siehe Tabelle Seite 12 f.)

| Substanz | Dosis mg/kg | Herzfrequenz-senkung | Halbwertzeit (Minuten) |
|---|---|---|---|
| A | 1,0 i.v. | - 55 % | > 120 |
| B | 1,0 i.v. | - 45 % | > 120 |
| C | 1,0 i.v. | - 44 % | > 90 |
| D | 1,0 i.v. | - 41 % | 80 |
| E | 1,0 i.v. | - 45 % | > 90 |
| F | 1,0 i.v. | - 51 % | > 120 |
| L | 1,0 i.v. | - 8,2 % | 13 |

2. Wirkung auf die Herzfrequenz an Meerschweinchen-Vorhöfen :

Isolierte spontan schlagende Vorhöfe von Meerschweinchen beider Geschlechter mit einem Körpergewicht von 300-400 g wurden in einem Organbad in Tyrode-Lösung untersucht. Die Nährlösung wurde mit Carbogen (95 % $O_2$ + 5 % $CO_2$) versorgt und konstant bei 30 °C gehalten. Die Kontraktionen wurden isometrisch mittels eines Dehnungsmeßstreifens auf einem Grass-Polygraphen registriert. Die zu untersuchenden Substanzen wurden den Organbädern je 60 Minuten lang in verschiedenen Konzentrationen zugesetzt.

Aus den Maximaleffekten wurden Konzentrationswirkungskurven erstellt und aus diesen diejenige Konzentration bestimmt, welche die Schlagfrequenz um 30 % herabsetzte ( = EC-$60_{30}$).

Die nachfolgende Tabelle enthält die gefundenen Werte :

| Substanz | EC-$60_{30}$ [µg/ml] |
|---|---|
| A | 0.030 |
| C | 0.097 |
| D | 0.058 |
| E | 0.066 |
| F | 0.014 |
| G | 0.014 |
| H | 0.0079 |
| I | 0.063 |
| J | 0.050 |
| K | 0.014 |

3. Akute Toxizität :

Die akute Toxizität der zu untersuchenden Substanz wurde an Mäusen (Beobachtungszeit : 14 Tage) nach intravenöser und oraler Gabe bestimmt :

| Substanz | Toxizität ($LD_{50}$) | |
|---|---|---|
| A | 89 mg/kg i.v. | 1 350 mg/kg p.o. |

Aufgrund ihrer pharmakologischen Eigenschaften eignen sich die erfindungsgemäß hergestellten Verbindungen zur Behandlung von Sinustachykardien verschiedener Genese und zur Prophylaxe und Therapie ischämischer Herzerkrankungen.

Die zur Erzielung einer entsprechenden Wirkung erforderlichen Dosierung beträgt zweckmäßigerweise ein- bis zweimal täglich 0,03 bis 0,4 mg/kg Körpergewicht, vorzugsweise 0,07 bis 0,25 mg/kg Körpergewicht. Hierzu lassen sich die erfindungsgemäß hergestellten Verbindungen der allgemeinen Formel I sowie ihre physiologisch verträglichen Säureadditionssalze mit anorganischen oder organischen Säuren, gegebenenfalls in Kombination mit anderen Wirksubstanzen, zusammen mit einem oder mehreren inerten üblichen Trägerstoffen und/oder Verdünnungsmitteln, z. B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Carboxymethylcellulose oder fetthaltige Substanzen wie Hartfett oder deren geeignete Gemische, in übliche galenische Zubereitungen wie Tabletten, Dragées, Kapseln, Pulver, Suspensionen, Tropfen, Ampullen, Säfte oder Zäpfchen einarbeiten.

Die nachfolgenden Beispiele sollen die Erfindung näher erläutern :

## Herstellung der Ausgangsverbindungen :

### Beispiel A

7,8-Dimethoxy-1,3-dihydro-2H-3-benzazepin-2-on

a) 3,4-Dimethoxyphenylessigsäurechlorid

Zu einer Suspension von 549,4 g 3,4-Dimethoxyphenylessigsäure in 600 ml Methylenchlorid werden während 2 Stunden 600 ml Thionylchlorid unter Rühren zugetropft. Nach beendeter Gasentwicklung (16 Stunden) wird noch eine Stunde am Rückfluß gekocht. Nach dem Entfernen der leicht flüchtigen Komponenten wird der Rückstand im Vakuum destilliert.

Ausbeute : 486 g (80,8 % der Theorie),
Kp : 134-136 °C/1,95 m bar

b) N-(2,2-Dimethoxyethyl)-3,4-dimethoxyphenylacetamid

Unter Eiskühlung wird eine Lösung von 485,2 g 3,4-Dimethoxyphenylessigsäurechlorid in 1,1 l Methylenchlorid bei 15-20 °C zu einer Lösung von 246,2 ml Aminoacetaldehyddimethylacetal und 315 ml Triäthylamin in 2,2 l Methylenchlorid zugetropft und eine Stunde bei 16-18 °C nachgerührt. Anschließend wird mehrfach mit Wasser extrahiert, über Magnesiumsulfat getrocknet und eingedampft. Das erhaltene Öl kristallisiert langsam durch.

Ausbeute : 608 g (95 % der Theorie),
Schmelzpunkt : 66-69 °C.

c) 7,8-Dimethoxy-1,3-dihydro-2H-3-benzazepin-2-on

Eine Lösung von 600,6 g N-(2,2-Dimethoxyethyl)-3,4-dimethoxyphenylacetamid in 3 l konzentrierter Salzsäure wird mit 3 l Eisessig versetzt. Nach 17-stündigem Stehenlassen bei Raumtemperatur wird der Ansatz auf Eis gegossen. Die ausgefallenen Kristalle werden abgesaugt, mit Wasser neutral gewaschen und getrocknet.

Ausbeute : 350 g (75,4 % der Theorie),
Schmelzpunkt : 234-237 °C.

### Beispiel B

7,8-Dimethoxy-1,3,4,5-tetrahydro-2H-3-benzazepin-2-on

Eine Suspension von 21,9 g (0,1 Mol) 7,8-Dimethoxy-1,3-dihydro-2H-3-benzazepin-2-on und 1,5 g Palladium/Kohle (10 %ig) in 200 ml Eisessig wird bei 50 °C und einem Wasserstoffdruck von 5 bar hydriert. Nach Abfiltrieren des Katalysators wird das Lösungsmittel im Vakuum eingedampft und der Rückstand in Methylenchlorid aufgenommen. Nach Extraktion mit Natriumbicarbonat-Lösung und Waschen mit Wasser wird über Magnesiumsulfat getrocknet, eingeengt und über Kieselgel mit Methylenchlorid und anschließend mit steigenden Anteilen von Methanol (bis 10 %) gereinigt.

Ausbeute : 12,6 g (57 % der Theorie),
Schmelzpunkt : 188-191 °C.

### Beispiel C

7,8-Dimethoxy-2,3,4,5-tetrahydro-1H-3-benzazepin

Zu einer Suspension von 1,3 g (6 mMol) 7,8-Dimethoxy-1,3,4,5-tetrahydro-2H-3-benzazepin-2-on und 1,1 g (3 mMol) Natriumborhydrid in 20 ml Dioxan wird eine Lösung von 1,8 g Eisessig in 10 ml Dioxan getropft, 3 Stunden unter Rückfluß gekocht, eingeengt und mit Wasser zersetzt. Das Gemisch wird 2 mal mit Methylenchlorid ausgeschüttelt, der Extrakt eingeengt und der Rückstand in Ether aufgenommen. Nach Filtration wird der Ether im Vakuum entfernt.

Ausbeute : 1,1 g (92,7 % der Theorie),
Schmelzpunkt : 86-89 °C.

Beispiel D

N-[3-[N'-Methyl-N'-(2-(3,4-dimethoxy-phenyl)-ethyl)-amino]-propyl]-2-(2-amino-4,5-dimethoxy-phenyl)-acetamid

33,3 g (0,07 Mol) N-[3-[N'-Methyl-N'-(2-(3,4-dimethoxy-phenyl)-ethyl)-amino]-propyl]-2-(2-nitro-4,5-dimethoxy-phenyl)-acetamid, gelöst in 500 ml Methanol, werden bei 25 °C und einem Wasserstoffdruck von 5 bar 8 Stunden unter Anwesenheit von 10 %iger Palladium/Kohle hydriert. Nach Entfernung des Katalysators wird das Lösungsmittel im Vakuum abdestilliert.
Ausbeute : 31,5 g (100 % der Theorie), viskoses Öl.

Beispiel E

6,9-Dimethoxy-1,3-dihydro-2H-3-benzazepin-2-on

2,0 g (0,007 Mol) N-(2,2-Dimethoxyethyl)-2,5-dimethoxyphenyl-acetamid werden mit 3 ml Polyphosphorsäure übergossen und 60 Minuten bei 90 °C gerührt. Anschließend wird mit Eiswasser versetzt, das ausgefallene Produkt abgesaugt und getrocknet.
Ausbeute : 0,98 g (64 % der Theorie),
Schmelzpunkt : 188-191 °C.

Beispiel F

7,8-Dimethyl-1,3-dihydro-2H-3-benzazepin-2-on

Hergestellt analog Beispiel E aus N-(2,2-Dimethoxyethyl)-3,4-dimethyl-phenylacetamid und Polyphosphorsäure.
Ausbeute : 40,1 % der Theorie,
Schmelzpunkt : 220-224 °C.

Beispiel G

7,8-Dimethoxy-5-methyl-1,3-dihydro-3,4-benzodiazepin-2-on

19,5 g (0,082 Mol) 2-Acetyl-4,5-dimethoxyphenylessigsäure werden in 200 ml Äthanol suspendiert, mit 8,2 ml 98 %igem Hydrazinhydrat versetzt und 6 Stunden unter Rückfluß gekocht. Das Reaktionsgemisch wird im Vakuum eingeengt und über eine Kieselgel-Säule mit Methylenchlorid und 1 % Ethanol als Elutionsmittel gereinigt.
Ausbeute : 9,6 g (50 % der Theorie),
IR-Spektrum (Methylenchlorid) : 3 300 $cm^{-1}$ (NH)
2 830 $cm^{-1}$ ($OCH_3$)
1 650 $cm^{-1}$ (CO)

Beispiel H

7,8-Dimethoxy-1,3,4,5-tetrahydro-2H-3-benzazepin-2,4-dion

a) 7,8-Dimethoxy-2-amino-4-brom-1H-3-benzazepin-hydrobromid

3,7 g (0,017 Mol) 3,4-Dimethoxy-o-phenylen-diacetonitril werden in 10 ml Eisessig suspendiert und bei 20 °C mit 12 ml 30 %iger Bromwasserstoffsäure in Eisessig versetzt. 3 Stunden wird bei Raumtemperatur nachgerührt, der ausgefallene Niederschlag abgesaugt, mit Eisessig und anschließend mit Aceton/Ether gewaschen und getrocknet.
Ausbeute : 5,3 g (82,8 % der Theorie),
Schmelzpunkt : 210-211 °C (Zers.).

b) 7,8-Dimethoxy-1,3,4,5-tetrahydro-2H-3-benzazepin-2,4-dion

5,3 g (0,014 Mol) 7,8-Dimethoxy-2-amino-4-brom-1H-3-benz-azepin-hydrobromid werden in 100 ml 85 °C heißem Wasser gelöst, mit 1,3 g wasserfreiem Natriumacetat versetzt und eine Stunde auf 90 °C erhitzt. Das Reaktionsgemisch wird abgekühlt, abgesaugt, mit kaltem Wasser nachgewaschen und getrocknet.
Ausbeute : 2,9 g (88 % der Theorie),
Schmelzpunkt : 235 °C (Zers.).

Beispiel I

N-[3-(7,8-Dimethoxy-1,3,4,5-tetrahydro-2H-3-benzazepin-2-on-3-yl)-propyl]-methylamin-hydrochlorid

Hergestellt analog Beispiel B durch katalytische Hydrierung von 1-[7,8-Dimethoxy-1,3-dihydro-2H-3-benzazepin-2-on-3-yl]-3-(N-benzyl-methylamino)-propan.
Ausbeute : 87 % der Theorie,
Schmelzpunk : 110 °C (Zers.).

Beispiel J

1-Chlor-3-[N-methyl-N-(2-(3,4-dimethoxy-phenyl)-ethyl)-amino]-propan

a) 3-[N-Methyl-N-(2-(3,4-dimethoxy-phenyl)-ethyl)-amino]-propanol

Eine Mischung aus 2,1 g (0,011 Mol) N-Methyl-2-(3,4-dimethoxyphenyl)-ethyl-amin und 0,9 g (0,011 Mol) Acrylsäuremethylester wird über Nacht bei Zimmertemperatur stehen gelassen. Das hierbei entstandene Additionsprodukt wird in 40 ml Ether gelöst und während 15 Minuten unter Rühren zu einer Suspension von 0,3 g (0,008 Mol) Lithium-aluminium-hydrid in 20 ml Ether getropft. Nach 5-stündigem Kochen am Rückfluß wird abgekühlt und anschließend 30 ml gesättigte wässrige Natriumsulfatlösung zugetropft. Der ausgefallene Niederschlag wird über Kieselgur abgesaugt und das Filtrat mit Methylenchlorid ausgeschüttelt. Die organische Phase wird über Natriumsulfat getrocknet und zur Trockene eingedampft.
Ausbeute : 3 g (89,7 % der Theorie),
IR-Spektrum (Methylenchlorid) : 3 600, 3 230 $cm^{-1}$ (OH) m/e = 253 ($C_{14}H_{23}NO_3$ ; 253,35)

b) 1-Chlor-3-[N-methyl-N-(2-(3,4-dimethoxy-phenyl)-ethyl)-amino]-propan

Zu 4 g 3-[N-Methyl-N-(2-(3,4-dimethoxyphenyl)-ethyl)-amino]-propanol werden 2,75 g Benzolsulfonsäurechlorid gegeben. Nach 1/2-stündigem Stehen wird in Methylenchlorid gelöst, mit 30 %-iger Natriumhydroxid-Lösung und Wasser gewaschen, über Natriumsulfat getrocknet und im Vakuum eingeengt. Nach der Reinigung über eine Kieselgelsäule (Elutionsmittel : Methylenchlorid/Ethanol) erhält man ein farbloses Öl.
Ausbeute : 1 g (17 % der Theorie),
$C_{14}H_{22}ClNO_2$ (271,8)
Ber. : C 61,86 H 8,15 N 5,15
Gef. : C 62,00 H 8,00 N 4,63

Beispiel K

N-Methyl-N-[2-(3,4-dimethoxy-phenyl)-ethyl]-azetidinium-bromid

3 g (0,011 Mol) 3-[2-(3,4-dimethoxy-phenyl)-ethyl]-azetidinium-bromid

3 g (0,011 Mol) 3-[N-Methyl-N-(2-(3,4-dimethoxy-phenyl)-ethyl)-amino]-propanol werden in 15 ml Methylenchlorid gelöst und bei 0-5 °C eine Lösung von 2 ml Phosphortribromid in 5 ml Methylenchlorid zugetropft. Nach dem Erwärmen auf Zimmertemperatur wird 2 Stunden am Rückfluß gekocht. Es wird zur Trockene eingedampft, anschließend in Methylenchlorid gelöst, mit 2 n Natriumhydroxid und Wasser gewaschen. Die organische Phase wird über Natriumsulfat getrocknet und zur Trockene eingedampft. Nach Reinigung des Rohproduktes über eine Kieselgelsäule (Elutionsmittel : Methylenchlorid/Ethanol = 100 : 5) wird der Rückstand noch 40 Minuten auf 60-70 °C erwärmt.
Ausbeute : 1,8 g (48 % der Theorie),
Schmelzpunkt : 175-180 °C.

Beispiel L

7,8-Dimethoxy-2,3-dihydro-1H-3-benzazepin

Eine siedende Suspension von 0,8 g Lithiumaluminiumhydrid in 100 ml absolutem Dioxan wird mit 2,2 g (0,01 Mol) 7,8-Dimethoxy-1,3-dihydro-2H-3-benzazepin-2-on versetzt und anschließend 3 Stunden unter Rückfluß erhitzt. Unter Eiswasserkühlung wird mit 10 %iger Ammoniumchlorid-Lösung versetzt und der gebildete Niederschlag abgesaugt. Das Filtrat wird im Vakuum auf ein Volumen von etwa 20 ml eingeengt, der ausgefallene weiße Niederschlag abgesaugt und mit wenig Dioxan nachgewaschen.
Ausbeute : 0,9 g (43,8 % der Theorie),
Schmelzpunkt : 162-163 °C.

Herstellung der Endprodukte

Beispiel 1

1-[7,8-Dimethoxy-1,3-dihydro-2H-3-benzazepin-2-on-3-yl]-3-[N-methyl-N-(2-(3,4-dimethoxy-phenyl)-ethyl)-amino]-propan-hydrochlorid

a) 1-(7,8-Dimethoxy-1,3-dihydro-2H-3-benzazepin-2-on-3-yl)-3-chlor-propan

131,5 g (0,6 Mol) 7,8-Dimethoxy 3-dihydro-2H-3-benzazepin-2-on werden in 900 ml Dimethylsulfoxid suspendiert und unter Rühren mit 80,8 g (0,72 Mol) Kalium-tert.butylat versetzt. Nach 10 Minuten wird die erhaltene Lösung unter Kühlung mit Eiswasser zu 77 ml (0,72 Mol) 1-Brom-3-chlorpropan in 300 ml Dimethylsulfoxid getropft. Nach einer Stunde gießt man auf Eiswasser. Nach kurzer Zeit beginnt die schmierige Fällung zu kristallisieren. Der Niederschlag wird abgesaugt, in Aceton gelöst, mit Wasser nochmals ausgefällt, abgesaugt und getrocknet.
Ausbeute : 155,5 g (87,3 % der Theorie),
Schmelzpunkt : 101-103 °C.

b) 1-[7,8-Dimethoxy-1,3-dihydro-2H-3-benzazepin-2-on-3-yl]-3-[N-methyl-N-(2-(3,4-dimethoxy-phenyl)-ethyl)-amino]-propan-hydrochlorid

5,9 g (0,02 Mol) 1-(7,8-Dimethoxy-1,3-dihydro-2H-3-benzazepin-2-on-3-yl)-3-chlor-propan und 11,7 g (0,06 Mol) N-Methyl-2-(3,4-dimethoxy-phenyl)-ethylamin werden 3 Stunden auf 100 °C erhitzt, abgekühlt und in Essigester/Wasser gelöst. Die organische Phase wird abgetrennt, 3 mal mit 1 %iger Essigsäure gewaschen und 2 mal mit 2n Salzsäure ausgeschüttelt. Der salzsaure Extrakt wird ammoniakalisch gestellt und mit Methylenchlorid extrahiert. Das Lösungsmittel des Extraktes entfernt man im Vakuum, löst den Rückstand in Aceton und fällt mit etherischer Salzsäure das Hydrochlorid.
Ausbeute : 6,9 g (70,3 % der Theorie),
Schmelzpunkt : 190-191 °C (Zers.).

Beispiel 2

1-[7,8-Dimethoxy-2,3,4,5-tetrahydro-1H-3,5-benzodiazepin-2,4-dion-3-yl]-3-[N-methyl-N-(2-(3,4-dimethoxy-phenyl)-ethyl)-amino]-propan-hydrochlorid

4,6 g (0,01 Mol) N-[3-[N'-Methyl-N'-(2-(3,4-dimethoxy-phenyl)-ethyl)-amino]-propyl]-2-(2-amino-4,5-dimethoxy-phenyl)-acetamid und 3,6 g (0,022 Mol) N,N'-Carbonyldiimidazol werden in 100 ml Acetonitril 33 Stunden gekocht. Das Lösungsmittel wird im Vakuum entfernt und der Rückstand über Alumina Woelm N, Akt. III, mit Methylenchlorid und 15 % Aceton als Elutionsmittel gereinigt. Die Fraktionen werden im Vakuum eingeengt, der Rückstand in Aceton gelöst und mit etherischer Salzsäure gefällt.
Ausbeute : 1,1 g (21,7 % der Theorie),
Schmelzpunkt : 125-130 °C.

Beispiel 3

1-[7,8-Dimethoxy-1,3,4,5-tetrahydro-2H-3-benzazepin-2-on-3-yl]-3-[N-methyl-N-(2-(2-amino-3,5-dichlor-phenyl)-amino]-propan

Hergestellt analog Beispiel 1b durch Umsetzung von 1-(7,8-Dimethoxy-1,3,4,5-tetrahydro-2H-3-benzazepin-2-on-3-yl)-3-chlorpropan mit N-[2-(2-Amino-3,5-dichlor-phenyl)-ethyl]-methylamin. Öl.

UV-Spektrum (Ethanol) : λ max. 240 nm (0.34)
285 nm (0.14)
306 nm (0.10)

IR-Spektrum (Dichlormethan) : 2 810 $cm^{-1}$ N-Alkyl
2 840 $cm^{-1}$ O—$CH_3$
1 655 $cm^{-1}$ C=O
1 520 und 1 620 $cm^{-1}$ (C=C)

Beispiel 4

1-[7,8-Dimethoxy-1,3,4,5-tetrahydro-2H-3-benzazepin-2-on-3-yl]-3-[N-methyl-N-(2-(3,4-dimethoxy-phenyl)-ethyl)-amino]-propandihydrochlorid

5,8 g (0,012 7 Mol) 1-[7,8-Dimethoxy-1,3-dihydro-2H-3-benzazepin-2-on-3-yl]-3-[N-methyl-N-(2-3,4-dimethoxy-phenyl)-ethyl)-amino]-propan, gelöst in 60 ml Eisessig, werden bei Raumtemperatur und einem

Wasserstoffdruck von 5 bar in Anwesenheit von 10 %iger Palladium/Kohle 5 Stunden hydriert. Der Katalysator wird abfiltriert, das Filtrat im Vakuum eingeengt und der Rückstand in Methylenchlorid/halbgesättigter Kaliumcarbonat-Lösung aufgenommen. Die organische Phase wird mit Aktivkohle/Bleicherde behandelt, filtriert und im Vakuum eingeengt. Anschließend wird der Rückstand in Aceton gelöst und mit etherischer Salzsäure das Dihydrochlorid gefällt.

Ausbeute : 6,2 g (92 % der Theorie),

Schmelzpunkt : 137 °C (Zers.).

Bei Verwendung der äquimolaren Menge an ethanolischer Salzsäure erhält man das Hydrochlorid vom Schmelzpunkt 165-168 °C.

Beispiel 5

1-[7,8-Dimethoxy-1,3,4,5-tetrahydro-2H-3-benzazepin-2-on-3-yl]-3-[N-methyl-N-(2-(3,4-dimethoxy-phenyl)-ethyl)-amino]-propandihydrochlorid

a) 1-(7,8-Dimethoxy-1,3,4,5-tetrahydro-2H-3-benzazepin-2-on-3-yl)-3-chlor-propan

Hergestellt analog Beispiel 1a durch Umsetzung von 7,8-Dimethoxy-1,3,4,5-tetrahydro-2H-3-benzazepin-2-on mit 1-Brom-3-chlorpropan (Ausbeute : 50 % der Theorie) oder analog Beispiel 4 durch katalytische Hydrierung von 1-(7,8-Dimethoxy-1,3-dihydro-2H-3-benzazepin-2-on-3-yl)-3-chlor-propan (Ausbeute : 88 % der Theorie).

Schmelzpunkt : 84-85 °C.

b) 1-[7,8-Dimethoxy-1,3,4,5-tetrahydro-2H-3-benzazepin-2-on-3-yl]-3-[N-methyl-N-(2-(3,4-dimethoxy-phenyl)-ethyl)-amino]-propan-dihydrochlorid

Hergestellt analog Beispiel 1b durch Umsetzung von 1-(7,8-Dimethoxy-1,3,4,5-tetrahydro-2H-3-benzazepin-2-on-3-yl)-3-chlor-propan mit N-Methyl-2-(3,4-dimethoxy-phenyl)-ethylamin.

Ausbeute : 75,2 % der Theorie.

Schmelzpunkt : 135-137 °C (Zers.).

Beispiel 6

1-[7,8-Dimethoxy-2,3,4,5-tetrahydro-1H-3-benzazepin-3-yl]-3-[N-methyl-N-(2-(3,4-dimethoxy-phenyl)-ethyl)-amino]-propandihydrochlorid

2,7 g (6 mMol) 1-[7,8-Dimethoxy-1,3,4,5-tetrahydro-2H-3-benzazepin-2-on-3-yl]-3-[N-methyl-N-(2-(3,4-dimethoxy-phenyl)-ethyl)-amino]-propan werden in 100 ml absolutem Ether und 100 ml absolutem Tetrahydrofuran gelöst, mit 0,25 g Lithiumaluminiumhydrid versetzt und 3,5 Stunden unter Rückfluß gekocht. Nach dem Abkühlen wird unter Kühlung eine 10 %ige Ammoniumchlorid-Lösung zugegeben, abgesaugt und das Filtrat eingedampft. Der Rückstand wird über eine Kieselgel-Säule mit Methylenchlorid gereinigt. Die Fraktionen werden eingeengt, der Rückstand in Aceton gelöst und mit methanolischer Salzsäure das Dihydrochlorid gefällt.

Ausbeute : 1,5 g (48,5 % der Theorie),

Schmelzpunkt : 270-271 °C (Zers.).

Beispiel 7

1-[7,8-Dimethoxy-1,3-dihydro-2H-3-benzazepin-2-on-3-yl]-3-[N-methyl-N-(2-(4-methoxy-phenyl)-ethyl)-amino]-propan-hydrochlorid

Hergestellt analog Beispiel 1b durch Umsetzung von 1-[7,8-Dimethoxy-1,3-dihydro-2H-3-benzazepin-2-on-3-yl)-3-chlor-propan mit N-Methyl-2-(4-methoxy-phenyl)-ethylamin.

Ausbeute : 76,2 % der Theorie,

Schmelzpunkt : 139-142 °C.

Beispiel 8

1-[7,8-Dimethoxy-1,3,4,5-tetrahydro-2H-3-benzazepin-2-on-3-yl]-3-[N-methyl-N-(2-(4-methoxy-phenyl)-ethyl)-amino]-propan-hydrochlorid

Hergestellt analog Beispiel 4 durch katalytische Hydrierung von 1-[7,8-Dimethoxy-1,3-dihydro-2H-3-benzazepin-2-on-3-yl]-3-[N-methyl-N-(2-(4-methoxy-phenyl)-ethyl)-amino]-propan.

Ausbeute : 73,3 % der Theorie,

Schmelzpunkt : 175-177 °C.

Beispiel 9

1-[7,8-Methylendioxy-1,3-dihydro-2H-3-benzazepin-2-on-3-yl]-3-[N-methyl-N-(2-(3,4-dimethoxy-phenyl)-ethyl)-amino]-propanhydrochlorid

a) 1-(7,8-Methylendioxy-1,3-dihydro-2H-benzazepin-2-on-3-yl)-3-chlor-propan

Hergestellt analog Beispiel 1a durch Umsetzung von 7,8-Methylendioxy-1,3-dihydro-2H-3-benzazepin-2-on (Schmelzpunkt : 195 °C (Zers.)) mit 1-Brom-3-chlorpropan.
Ausbeute : 72,1 % der Theorie,
Schmelzpunkt : 75-79 °C.

b) 1-[7,8-Methylendioxy-1,3-dihydro-2H-3-benzazepin-2-on-3-yl]-3-[N-methyl-N-(2-(3,4-dimethoxy-phenyl)-ethyl)-amino]-propan-hydrochlorid

Hergestellt analog Beispiel 1b durch Umsetzung von 1-(7,8-Methylendioxy-1,3-dihydro-2H-benzazepin-2-on-3-yl)-3-chlorpropan mit N-Methyl-2-(3,4-dimethoxy-phenyl)-ethylamin.
Ausbeute : 77,2 % der Theorie,
Schmelzpunkt : 185-187 °C.

Beispiel 10

1-[7,8-Methylendioxy-1,3,4,5-tetrahydro-2H-3-benzazepin-2-on-3-yl]-3-[N-methyl-N-(2-(3,4-dimethoxy-phenyl)-ethyl)-amino]-propan-hydrochlorid

Hergestellt analog Beispiel 4 durch katalytische Hydrierung von 1-[7,8-Methylendioxy-1,3-dihydro-2H-3-benzazepin-2-on-3-yl]-3)-[N-methyl-N)-2-(3,4-dimethoxy-phenyl)-ethyl)-amino]-propan.
Ausbeute : 77,3 % der Theorie,
Schmelzpunkt : 210-212 °C.

Beispiel 11

1-[7,8-Dimethoxy-1,3,4,5-tetrahydro-2H-3-benzazepin-2-on-3-yl]-3-[N-methyl-N-(2)-3,4-dimethoxy-phenyl)-ethyl)-amino]-propan

Zu einer Mischung von 0,22 g (0,5 mMol) 1-[7,8-Dimethoxy-1,3,4,5-tetrahydro-2H-3-benzazepin-2-on-3-yl]-3-[N-(2-(3,4-dimethoxyphenyl)-ethyl)-amino]-propan, 0,5 ml 37 %iger Formalinlösung, 2 ml Acetonitril und 0,1 g Natriumcyanborhydrid werden bei Zimmertemperatur 0,05 ml Eisessig gegeben. Nach 2 Stunden werden weitere 0,05 ml Eisessig zugegeben und 1/2 Stunde nachgerührt. Die Aufarbeitung erfolgt durch Eindampfen, Aufnehmen in Methylenchlorid, Waschen mit 2 n Natronlauge und Wasser. Die über Natriumsulfat getrocknete organische Phase wird eingeengt und über Kieselgel gereinigt.
Ausbeute : 104 mg (45,5 % der Theorie), viskoses Öl,
IR-Spektrum (Methylenchlorid) : 1 645 $cm^{-1}$ (CO),
1 510 $cm^{-1}$ (aromat. C=C)
Schmelzpunkt des Dihydrochlorids : 137 °C (Zers.).

Beispiel 12

1-[7,8-Dimethoxy-1,3,4,5-tetrahydro-2H-3-benzazepin-2-on-3-yl]-3-[N-methyl-N-(2-(3,4-dimethoxy-phenyl)-ethyl)-amino]-propan-dihydrochlorid

22 g (0,075 Mol) N-[3-(7,8-Dimethoxy-1,3,4,5-tetrahydro-2H-3-benzazepin-2-on-3-yl)-propyl]-methylamin werden mit 7,2 g (0,036 Mol) 2-(3,4-Dimethoxy-phenyl)-ethylchlorid 20 Stunden lang auf 140 °C erhitzt. Nach Lösen des Reaktionsproduktes in Methylenchlorid wird die Lösung einmal mit 2 n Natronlauge und 2 mal mit Wasser gewaschen, über Natriumsulfat getrocknet und eingeengt. Der Rückstand wird durch Chromatographie an Kieselgel (Korngröße : 0,063-0,2 mm ; Elutionsmittel : Methylenchlorid/Methanol = 10 : 1) gereinigt. Das so erhaltene Produkt wird in Aceton gelöst und mit etherischer Salzsäure das Dihydrochlorid gefällt.
Ausbeute : 8,0 g (45 % der Theorie),
Schmelzpunkt : 135-136 °C (Zers.).

Beispiel 13

1-[7,8-Dimethoxy-1,3,4,5-tetrahydro-2H-3-benzazepin-2-on-3-yl]-3-[N-methyl-N-(2-(4-acetylamino-3,5-dichlor-phenyl)-ethyl)-amino]-propan

3 g (0,006 3 Mol) 1-[7,8-Dimethoxy-1,3,4,5-tetrahydro-2H-3-benzazepin-2-on-3-yl]-3-[N-methyl-N-(2-(4-amino-3,5-dichlor-phenyl)-ethyl)-amino]-propan in 50 ml Chloroform und 0,7 g (0,007 Mol) Triäthylamin werden bei Siedetemperatur portionsweise mit insgesamt ca. 0,8 ml Acetylchlorid versetzt und mehrere Stunden gekocht. Nach Abkühlen auf Raumtemperatur wird mit Wasser gewaschen. Die wässrige Phase wird mit 2 n Natronlauge stark alkalisch gestellt und mit Methylenchlorid extrahiert. Die abgetrennte organische Phase wird über Natriumsulfat getrocknet und eingeengt. Das so erhaltene Rohprodukt reinigt man durch Chromatographie an Kieselgel (Korngröße : 0,063-0,2 mm, Elutionsmittel : Methylenchlorid/Methanol = 8 : 1).

Schmelzpunkt : 144-146 °C.

## Beispiel 14

1-[7,8-Dimethoxy-1,3,4,5-tetrahydro-2H-3-benzazepin-2-on-3-yl]-3-[N-methyl-N-(2)-4-amino-3,5-dibrom-phenyl)-ethyl)-amino]-propan

Zu einer Lösung von 1 g (0,002 5 Mol) 1-[7,8-Dimethoxy-1,3,4,5-tetrahydro-2H-3-benzazepin-2-on-3-yl]-3-[N-methyl-N-(2-(4-amino-phenyl)-ethyl)-amino]-propan in 20 ml 95 %iger Essigsäure tropft man 0,8 g (0,005 Mol) Brom unter Rühren bei Raumtemperatur. Nach ca. 60 Minuten wird das Lösungsmittel am Rotationsverdampfer entfernt. Der Rückstand wird zwischen 2 n Natronlauge und Methylenchlorid verteilt. Die Methylenchloridphase wäscht man einmal mit Wasser, trocknet sie über Natriumsulfat und engt anschließend im Vakuum ein. Der erhaltene ölige Rückstand kristallisiert bei Raumtemperatur und wird zur weiteren Reinigung aus absolutem Ethanol umkristallisiert.

Schmelzpunkt : 105-110 °C.

## Beispiel 15

1-[7,8-Dimethoxy-1,3,4,5-tetrahydro-2H-3-benzazepin-2-on-3-yl]-3-[N-methyl-N-(2-(3,4-dimethoxy-phenyl)-ethyl)-amino]-propan

0,22 g (0,5 mMol) 1-[7,8-Dimethoxy-1,3,4,5-tetrahydro-2H-3-benzazepin-2-on-3-yl]-3-[N-(2-(3,4-dimethoxy-phenyl)-ethyl)-amino]-propan werden mit 0,2 ml 37 %iger Formalinlösung und 0,2 ml 100 %iger Ameisensäure 20 Minuten auf 90-100 °C erhitzt. Die Aufarbeitung erfolgt analog Beispiel 11. Viskoses Öl.

IR-Spektrum (Methylenchlorid) : 1 645 $cm^{-1}$ (CO),
1 510 $cm^{-1}$ (aromat. C=C)

Schmelzpunkt des Dihydrochlorids : 137 °C (Zers.).

## Beispiel 16

1-[7,8-Dimethoxy-1,3,4,5-tetrahydro-2H-3-benzazepin-2-on-3-yl]-3)-[N-methyl-N-(2-(3,4-dimethoxy-phenyl)-ethyl)-amino]-propan

Zu einer Lösung von 3,29 g (10,0 mMol) N-[3-(7,8-Dimethoxy-1,3,4,5-tetrahydro-2H-3-benzazepin-2-on-3-yl)-propyl]-methyl-amin-hydrochlorid und 1,8 g (10,0 mMol) 2-(3,4-Dimethoxy-phenyl)-acetaldehyd in 40 ml Ethanol werden 1,26 g (20 mMol) Natriumcyanborhydrid hinzugegeben, wobei man durch Zugabe von 2 n Salzsäure darauf achtet, daß ein pH-Wert von 6-7 eingehalten wird. Bei diesem pH-Wert wird noch weitere 48 Stunden bei Raumtemperatur gerührt. Nach Einengen der Lösung im Vakuum wird der Rückstand in verdünnter Salzsäure aufgenommen und zweimal mit Ether extrahiert. Anschließend wird die wässrige Phase alkalisch gestellt, dreimal mit Methylenchlorid extrahiert, die organische Phase eingeengt und über Kieselgel gereinigt. Öl.

IR-Spektrum (Methylenchlorid) : 1 645 $cm^{-1}$ (CO)
1 510 $cm^{-1}$ (aromat. C=C)

Schmelzpunkt des Dihydrochlorids : 137 °C (Zers.).

## Beispiel 17

1-[7,8-Dimethoxy-1,3,4,5-tetrahydro-2H-3-benzazepin-2-on-3-yl]-3-[N-methyl-N-(2-(3,4-dimethoxy-phenyl)-ethyl)-amino]-propan

Zu einer Lösung von 2,77 g (10 mMol) 3-(7,8-Dimethoxy-1,3,4,5-tetrahydro-2H-3-benzazepin-2-on-3-yl)-propionaldehyd und 1,95 g (10 mMol) N-Methyl-2-(3,4-dimethoxy-phenyl)-ethylamin in 40 ml Methanol werden 1,26 g (20 mMol) Natriumcyanborhydrid hinzugegeben, wobei man durch Zugabe von Salzsäure einen pH-Wert von 6-7 einstellt. Anschließend wird bei diesem pH-Wert noch 50 Stunden bei Raumtemperatur gerührt. Nach Einengen der Lösung im Vakuum wird der Rückstand in verdünnter Salzsäure aufgenommen und dreimal mit Ether extrahiert. Dann wird die wäßrige Phase alkalisch gestellt, dreimal

mit Methylenchlorid extrahiert, die organische Phase eingeengt und über Kieselgel gereinigt. Dabei wird ein leicht gelbliches, viskoses Öl erhalten.

IR-Spektrum (Methylenchlorid) : 1 645 $cm^{-1}$ (CO)
1 510 $cm^{-1}$ (aromat. C=C)

Schmelzpunkt des Dihydrochlorids : 137 °C (Zers.).

Beispiel 18

1-[8-Methoxy-1,3,4,5-tetrahydro-2H-3-benzazepin-2-on-3-yl]-3-[N-methyl-N-(2-(3,4-dimethoxy-phenyl)-ethyl)-amino]-propan-dihydrochlorid

a) 1-(8-Methoxy-1,3-dihydro-2H-3-benzazepin-2-on-3-yl)-3-chlorpropan

Hergestellt analog Beispiel 1a durch Umsetzung von 8-Methoxy-1,3-dihydro-2H-3-benzazepin-2-on (Schmelzpunkt : 189-190 °C) mit 1-Brom-3-chlorpropan.
Ausbeute : 23 % der Theorie,
IR-Spektrum (Methylenchlorid) : 1 655 $cm^{-1}$ (CO)

b) 1-(8-Methoxy-1,3,4,5-tetrahydro-2H-3-benzazepin-2-on-3-yl)-3-chlorpropan

Hergestellt analog Beispiel 4 durch katalytische Hydrierung von 1-(8-Methoxy-1,3-dihydro-2H-3-benzazepin-2-on-3-yl)-3-chlorpropan.
Ausbeute : 67 % der Theorie,
IR-Spektrum (Methylenchlorid) : 1 645 $cm^{-1}$ (CO)

c) 1-[8-Methoxy-1,3,4,5-tetrahydro-2H-3-benzazepin-2-on-3-yl]-3-[N-methyl-N-(2-(3,4-dimethoxy-phenyl)-ethyl)-amino]-propan-dihydrochlorid

Hergestellt analog Beispiel 5b durch Umsetzung von 1-(8-Methoxy-1,3,4,5-tetrahydro-2H-3-benzazepin-2-on-3-yl)-3-chlorpropan mit N-Methyl-2-(3,4-dimethoxy-phenyl)-ethylamin.
Ausbeute : 14 % der Theorie,
Schmelzpunkt : 118-121 °C.

Beispiel 19

1-[7,8-Dimethoxy-1,3,4,5-tetrahydro-2H-3-benzazepin-2-on-3-yl]-3-[N-methyl-N-(2-(4-amino-3-chlor-phenyl)-ethyl)-amino]-propan

Hergestellt analog Beispiel 12 aus N-[3-(7,8-Dimethoxy-1,3,4,5-tetrahydro-2H-3-benzazepin-2-on-3-yl)-propyl]-methylamin und 2-(4-Amino-3-chlor-phenyl)-ethylbromid. Öl.

IR-Spektrum (Methylenchlorid) : 3 380, 3 480 $cm^{-1}$ ($NH_2$)
1 645 $cm^{-1}$ (CO)

UV-Spektrum (Ethanol) : λ max : 238 nm (0,16)
280-290 nm (0,05)

Beispiel 20

1-[7,8-Dimethoxy-1,3,4,5-tetrahydro-2H-3-benzazepin-2-on-3-yl]-3-[N-ethyl-N-(2-(4-amino-3,5-dichlor-phenyl)-ethyl)-amino]-propan

Hergestellt analog Beispiel 12 aus N-[3-(7,8-Dimethoxy-1,3,4,5-tetrahydro-2H-3-benzazepin-2-on-3-yl)-propyl]-ethylamin und 2-(4-Amino-3,5-dichlor-phenyl)-ethylchlorid. Öl.

IR-Spektrum (Methylenchlorid) : 3 390, 3 480 $cm^{-1}$ ($NH_2$)
1 650 $cm^{-1}$ (CO)

UV-Spektrum (Ethanol) : λ max : 240 nm (0,13)
280-290 nm (0,05)

Beispiel 21

1-[7,8-Dimethoxy-1,3,4,5-tetrahydro-2H-3-benzazepin-2-on-3-yl]-3-[N-methyl-N-(2-(4-amino-3,5-dichlor-phenyl)-ethyl)-amino]-propan

Hergestellt analog Beispiel 12 aus N-[3-(7,8-Dimethoxy-1,3,4,5-tetrahydro-2H-3-benzazepin-2-on-3-yl)-propyl]-methylamin und 2-(4-Amino-3,5-dichlor-phenyl)-ethylchlorid.
Schmelzpunkt : 94-104 °C.

Beispiel 22

1-[7,8-Dimethoxy-1,3,4,5-tetrahydro-2H-3-benzazepin-2-on-3-yl]-3-[N-methyl-N-(2-(4-amino-3,5-dibrom-phenyl)-ethyl)-amino]-propan

Hergestellt analog Beispiel 12 aus N-[3-(7,8-Dimethoxy-1,3,4,5-tetrahydro-2H-3-benzazepin-2-on-3-yl)-propyl]-methylamin und 2-(4-Amino-3,5-dibrom-phenyl)-ethylchlorid.
Schmelzpunkt : 108-112 °C.

Beispiel 23

1-[7,8-Dimethoxy-1,3,4,5-tetrahydro-2H-3-benzazepin-2-on-3-yl]-3-[N-methyl-N-(2-(4-ethoxycarbonylamino-3,5-dichlor-phenyl)-ethyl)-amino]-propan

Hergestellt analog Beispiel 13 aus 1-[7,8-Dimethoxy-1,3,4,5-tetrahydro-2H-3-benzazepin-2-on-3-yl]-3-[N-methyl-N-(2-(4-amino-3,5-dichlor-phenyl-ethyl)-amino]-propan und Chlorameisensäureethylester. Öl.
IR-Spektrum (Methylenchlorid) : 3 410 $cm^{-1}$ (NH)
1 650 $cm^{-1}$ (CO—N<)
1 735 $cm^{-1}$ (CO—O—)
1 800 $cm^{-1}$ (CO—O—)
UV-Spektrum (Ethanol) : λ max : 240 nm (Schulter, 0,08)
280-290 nm (0,03)

Beispiel 24

1-[7,8-Dimethoxy-1,3,4,5-tetrahydro-2H-3-benzazepin-2-on-3-yl]-3-[N-methyl-N-(2-(4-bis-(ethoxycarbonyl)-amino-3,5-dichlorphenyl)-ethyl)-amino]-propan

Hergestellt analog Beispiel 13 aus 1-[7,8-Dimethoxy-1,3,4,5-tetrahydro-2H-3-benzazepin-2-on-3-yl]-3-[N-methyl-N-(2-(4-amino-3,5-dichlor-phenyl)-ethyl)-amino]-propan und Chlorameisensäureethylester. Öl.
IR-Spektrum (Methylenchlorid) : 1 650 $cm^{-1}$ (CO—N<)
1 730 $cm^{-1}$ (CO—O—)
1 760 $cm^{-1}$ (CO—O—)
1 800 $cm^{-1}$ (CO—O—)
UV-Spektrum (Ethanol) : λ max : 228 nm (Schulter, 0,15)
282 nm (0,03)

Beispiel 25

1-[7,8-Dimethoxy-1,3,4,5-tetrahydro-2H-3-benzazepin-2-on-3-yl]-3-[N-methyl-N-(2-(4-ethoxycarbonylamino-3-cyan-5-fluor-phenyl)-ethyl)-amino]-propan

Hergestellt analog Beipiel 13 aus 1-[7,8-Dimethoxy-1,3,4,5-tetrahydro-2H-3-benzazepin-2-on-3-yl]-3-[N-methyl-N-(2-(4-amino-3-cyan-5-fluor-phenyl)-ethyl)-amino]-propan und Chlorameisensäureethylester in Gegenwart von Triethylamin.
IR-Spektrum (Methylenchlorid) : 3 400 $cm^{-1}$ (NH)
2 830 $cm^{-1}$ ($OCH_3$)
1 730, 1 650, 1 510 $cm^{-1}$ (CO)

Beispiel 26

1-[7,8-Dimethoxy-1,3,4,5-tetrahydro-2H-3-benzazepin-2-on-3-yl]-3-[N-methyl-N-(2-(4-dimethylamino-3,5-dichlor-phenyl)-ethyl)-amino]-propan

Hergestellt analog Beispiel 11 aus 1-[7,8-Dimethoxy-1,3,4,5-tetrahydro-2H-3-benzazepin-2-on-3-yl]-3-[N-methyl-N-(2-(4-amino-3,5-dichlor-phenyl)-ethyl)-amino]-propan, Paraformaldehyd und Natriumcyanborhydrid in Methanol. Öl.
IR-Spektrum (Methylenchlorid) : 1 650 $cm^{-1}$ (CO)
UV-Spektrum (Ethanol) : λ max : 227 nm (Schulter, 0,16)
280 nm (0,12)

Beispiel 27

1-[7,8-Dimethoxy-1,3-dihydro-2H-3-benzazepin-2-on-3-yl]-3-[N-methyl-N-(2-(4-amino-3,5-dichlor-phenyl)-ethyl)-amino]-propan

Hergestellt analog Beispiel 1b aus 1-(7,8-Dimethoxy-1,3-dihydro-2H-3-benzazepin-2-on-3-yl)-3-chlor-propan und 2-(4-Amino-3,5-dichlor-phenyl)-N-methyl-ethylamin. Öl.

IR-Spektrum (Methylenchlorid) : 3 390, 3 480 $cm^{-1}$ ($NH_2$)
1 655 $cm^{-1}$ (CO)

UV-Spektrum (Ethanol) : λ max : 238 nm (Schulter, 0,25)
280 nm (0,1)
303 nm (0,12)

Beispiel 28

1-[7,8-Dimethoxy-1,3,4,5-tetrahydro-2H-3-benzazepin-2-on-3-yl]-3-[N-methyl-N-(2-(4-amino-3,5-dichlor-phenyl)-ethyl)-amino]-propan

Hergestellt analog Beispiel 4 aus 1-[7,8-Dimethoxy-1,3-dihydro-2H-3-benzazepin-2-on-3-yl]-3-[N-methyl-N-(2-(4-amino-3,5-dichlor-phenyl)-ethyl)-amino]-propan und Wasserstoff in Gegenwart von Palladium/Kohle in Eisessig.

Schmelzpunkt : 94-104 °C.

Beispiel 29

1-[7,8-Dimethoxy-1,3,4,5-tetrahydro-2H-3-benzazepin-2-on-3-yl]-3-[N-ethyl-N-(2-(4-amino-3,5-dibrom-phenyl)-ethyl)-amino]-propan

Hergestellt analog Beispiel 5b aus 1-(7,8-Dimethoxy-1,3,4,5-tetrahydro-2H-3-benzazepin-2-on-3-yl)-3-chlor-propan und 2-(4-Amino-3,5-dibrom-phenyl)-N-ethyl-ethylamin. Öl.

IR-Spektrum (Methylenchlorid) : 3 380, 3 480 $cm^{-1}$ ($NH_2$)
1 645 $cm^{-1}$ (CO)

UV-Spektrum (Ethanol) : λ max : 240 nm (Schulter, 0,13)
280-290 nm (0,04)

Beispiel 30

1-[7,8-Dimethoxy-1,3,4,5-tetrahydro-2H-3-benzazepin-2-on-3-yl]-3-[N-methyl-N-(2-(4-amino-3,5-dichlor-phenyl)-ethyl)-amino]-propan

Hergestellt analog Beispiel 5b aus 1-(7,8-Dimethoxy-1,3,4,5-tetrahydro-2H-3-benzazepin-2-on-3-yl)-3-chlor-propan und 2-(4-Amino-3,5-dichlor-phenyl)-N-methyl-ethylamin.

Schmelzpunkt : 94-104 °C.

Beispiel 31

1-[7,8-Dimethoxy-1,3,4,5-tetrahydro-2H-3-benzazepin-2-on-3-yl]-3-[N-isopropyl-N-(2-(4-amino-3,5-dichlor-phenyl)-ethyl)-amino]-propan

Hergestellt analog Beispiel 5b aus 1-(7,8-Dimethoxy-1,3,4,5-tetrahydro-2H-3-benzazepin-2-on-3-yl)-3-chlor-propan und 2-(4-Amino-3,5-dichlor-phenyl)-N-isopropyl-ethylamin.

Schmelzpunkt des Hydrochlorids : $< 90$ °C (Sinterung ab 70 °C).

IR-Spektrum (Methylenchlorid) : 3 390, 3 480 $cm^{-1}$ ($NH_2$)
1 650 $cm^{-1}$ (CO)

UV-Spektrum (Ethanol) : λ max : 238 nm (0,13)
280-290 nm (0,05)

Beispiel 32

1-[7,8-Dimethoxy-1,3,4,5-tetrahydro-2H-3-benzazepin-2-on-3-yl]-3-[N-methyl-N-(1-methyl-2-(4-amino-3,5-dichlor-phenyl)-ethyl)-amino]-propan-hydrochlorid

Hergestellt analog Beispiel 5b aus 1-(7,8-Dimethoxy-1,3,4,5-tetrahydro-2H-3-benzazepin-2-on-3-yl)-3-chlor-propan und 2-(4-Amino-3,5-dichlor-phenyl)-1-methyl-N-methyl-ethylamin.

Schmelzpunkt : 118-128 °C (Zers.).

Beispiel 33

1-[7,8-Dimethoxy-1,3,4,5-tetrahydro-2H-3-benzazepin-2-on-3-yl]-3-[N-(2-(4-amino-3,5-dichlor-phenyl)-ethyl)-amino]-propan-hydrochlorid

Hergestellt analog Beispiel 5b aus 1-(7,8-Dimethoxy-1,3,4,5-tetrahydro-2H-3-benzazepin-2-on-3-yl)-3-chlor-propan und 2-(4-Amino-3,5-dichlor-phenyl)-ethylamin.
Schmelzpunkt : 236-241 °C.

Beispiel 34

1-[7,8-Dimethoxy-1,3,4,5-tetrahydro-2H-3-benzazepin-2-on-3-yl]-3-[N-methyl-N-(2-(4-amino-3-chlor-5-methyl-phenyl)-ethyl)-amino]-propan

Hergestellt analog Beispiel 5b aus 1-(7,8-Dimethoxy-1,3,4,5-tetrahydro-2H-3-benzazepin-2-on-3-yl)-3-chlor-propan und 2-(4-Amino-3-chlor-5-methyl-phenyl)-N-methyl-ethylamin.
Schmelzpunkt : 60 °C (Sinterung, Schmelzen bei 73 °C).
IR-Spektrum (Methylenchlorid) : 3 390, 3 480 $cm^{-1}$ ($NH_2$)
1 650 $cm^{-1}$ (CO)
UV-Spektrum (Ethanol) : λ max : 237 nm (0,14)
280-290 nm (0,05)

Beispiel 35

1-[7,8-Dimethoxy-1,3,4,5-tetrahydro-2H-3-benzazepin-2-on-3-yl]-3-[N-methyl-N-(2-(3,5-dichlor-4-hydroxy-phenyl)-ethyl)-amino]-propan-hydrochlorid

Hergestellt analog Beispiel 5b aus 1-(7,8-Dimethoxy-1,3,4,5-tetrahydro-2H-3-benzazepin-2-on-3-yl)-3-chlor-propan und 2-(3,5-Dichlor-4-hydroxy-phenyl)-N-methyl-ethylamin.
Schmelzpunkt : 225 °C.

Beispiel 36

1-[7,8-Dimethoxy-1,3,4,5-tetrahydro-2H-3-benzazepin-2-on-3-yl]-3-[N-methyl-N-(2-(3-chlor-5-fluor-4-β,β,β-trifluorethylamino-phenyl)-ethyl)-amino]-propan

Hergestellt analog Beispiel 5b aus 1-(7,8-Dimethoxy-1,3,4,5-tetrahydro-2H-3-benzazepin-2-on-3-yl)-3-chlor-propan und 2-(3-Chlor-5-fluor-4-β,β,β-trifluorethylamino-phenyl)-N-methyl-ethylamin.
m/e = 545/547 ($C_{26}H_{32}ClF_4N_3O_3$ ; 546,03)

Beispiel 37

1-[7,8-Dimethoxy-1,3,4,5-tetrahydro-2H-3-benzazepin-2-on-3-yl]-3-[N-methyl-N-(2-(4-amino-3-chlor-5-fluor-phenyl)-ethyl)-amino]-propan

Hergestellt analog Beispiel 5b aus 1-(7,8-Dimethoxy-1,3,4,5-tetrahydro-2H-3-benzazepin-2-on-3-yl)-3-chlor-propan und 2-(4-Amino-3-chlor-5-fluor-phenyl)-N-methyl-ethylamin.
m/e = 463/465 ($C_{24}H_{31}ClFN_3O_3$ ; 463,99)

Beispiel 38

1-[7,8-Dimethoxy-1,3,4,5-tetrahydro-2H-3-benzazepin-2-on-3-yl]-3-[N-methyl-N-(2-(4-amino-3-chlor-5-trifluormethyl-phenyl)-ethyl)-amino]-propan

Hergestellt analog Beispiel 5b aus 1-(7,8-Dimethoxy-1,3,4,5-tetrahydro-2H-3-benzazepin-2-on-3-yl)-3-chlor-propan und 2-(4-Amino-3-chlor-5-trifluormethyl-phenyl)-N-methyl-ethylamin. Öl.
IR-Spektrum (Methylenchlorid) : 3 410, 3 510 $cm^{-1}$ ($NH_2$)
1 650 $cm^{-1}$ (CO)
1 610, 1 520 $cm^{-1}$ (C=C)
2 800 $cm^{-1}$ (N-Alkyl)
2 830 $cm^{-1}$ ($OCH_3$)
UV-Spektrum (Ethanol) : λ max : 241 nm (0,33)
285 nm (0,10)
310 nm (0,08)

Beispiel 39

1-[7,8-Dimethoxy-1,3,4,5-tetrahydro-2H-3-benzazepin-2-on-3-yl]-3-[N-methyl-N-(2-(4-amino-3-cyan-5-fluor-phenyl)-ethyl)-amino]-propan

Hergestellt analog Beispiel 5b aus 1-(7,8-Dimethoxy-1,3,4,5-tetrahydro-2H-3-benzazepin-2-on-3-yl)-3-chlor-propan und N-Methyl-(2-(4-amino-3-cyan-5-fluor-phenyl)-ethyl)-amin.

IR-Spektrum (Methylenchlorid) : 3 400, 3 490 $cm^{-1}$ ($NH_2$)
2 830 $cm^{-1}$ ($OCH_3$)
2 220 $cm^{-1}$ (CN)
1 650 $cm^{-1}$ (CO)

## Beispiel 40

1-[7,8-Dimethoxy-1,3-dihydro-2H-3-benzazepin-2-on-3-yl]-3-[N-benzyl-N-(2-(3,4-dimethoxy-phenyl)-ethyl)-amino]-propan-hydrochlorid

Hergestellt analog Beispiel 1b aus 1-(7,8-Dimethoxy-1,3-dihydro-2H-3-benzazepin-2-on-3-yl)-3-chlor-propan und 2-(3,4-Dimethoxy-phenyl)-N-benzyl-ethylamin.
Ausbeute : 51 % der Theorie,
Schmelzpunkt : 102 °C (Zers.).

## Beispiel 41

1-[7,8-Dimethoxy-1,3-dihydro-2H-3-benzazepin-2-on-3-yl]-3-[N-methyl-N-(2-(3,4-methylendioxy-phenyl)-ethyl)-amino]-propanhydrochlorid

Hergestellt analog Beispiel 1b aus 1-(7,8-Dimethoxy-1,3-dihydro-2H-3-benzazepin-2-on-3-yl)-3-chlor-propan und 2-(3,4-Methylendioxy-phenyl)-N-methyl-ethylamin.
Ausbeute : 48 % der Theorie,
Schmelzpunkt : 160-162 °C.

## Beispiel 42

1-[7,8-Dimethoxy-1,3-dihydro-2H-3-benzazepin-2-on-3-yl]-3-(N-benzyl-methylamino)-propan-hydrochlorid

Hergestellt analog Beispiel 1b aus 1-(7,8-Dimethoxy-1,3-dihydro-2H-3-benzazepin-2-on-3-yl)-3-chlor-propan und N-Methyl-benzylamin.
Ausbeute : 92 % der Theorie,
Schmelzpunkt : 208-209 °C.

## Beispiel 43

1-[7,8-Dimethoxy-1,3-dihydro-2H-3-benzazepin-2-on-3-yl]-4-[N-methyl-N-(2-(3,4-dimethoxy-phenyl)-ethyl)-amino]-butan-hydrochlorid

Hergestellt analog Beispiel 1b aus 1-(7,8-Dimethoxy-1,3-dihydro-2H-3-benzazepin-2-on-3-yl)-4-chlorbutan und N-Methyl-2-(3,4-dimethoxyphenyl)-ethylamin.
Ausbeute : 85 % der Theorie,
Schmelzpunkt : 162-164 °C.

## Beispiel 44

1-[8-Propoxy-1,3-dihydro-2H-3-benzazepin-2-on-3-yl]-2-[N-methyl-N-(2-(3,4-dimethoxy-phenyl)-ethyl)-amino]-ethan-hydrochlorid

Hergestellt analog Beispiel 1b aus 1-(8-Propoxy-1,3-dihydro-2H-3-benzazepin-2-on-3-yl)-2-chlor-ethan und N-Methyl-2-(3,4-dimethoxy-phenyl)-ethylamin.
Ausbeute : 31 % der Theorie,
Schmelzpunkt : 155-157 °C

## Beispiel 45

1-[7,8-Dimethoxy-1,3,4,5-tetrahydro-2H-3-benzazepin-2-on-3-yl]-4-[N-methyl-N-(2-(3,4-dimethoxy-phenyl)-ethyl)-amino]-butan-hydrochlorid

Hergestellt analog Beispiel 4 durch katalytische Hydrierung von 1-[7,8-Dimethoxy-1,3-dihydro-2H-3-benzazepin-2-on-3-yl]-4-[N-methyl-N-(2-(3,4-dimethoxy-phenyl)-ethyl)-amino]-butan.

Schmelzpunkt : 192-194 °C.

Beispiel 46

1-[7,8-Dimethoxy-1,3,4,5-tetrahydro-2H-3-benzazepin-2-on-3-yl]-3-[N-methyl-N-(2-(3,4-methylendioxy-phenyl)-ethyl)-amino]-propan-hydrochlorid

Hergestellt analog Beispiel 4 durch katalytische Hydrierung von 1-[7,8-Dimethoxy-1,3-dihydro-2H-3-benzazepin-2-on-3-yl]-3-[N-methyl-N-(2-(3,4-methylendioxy-phenyl)-ethyl)-amino]-propan.
Ausbeute : 72 % der Theorie,
Schmelzpunkt : 191-193 °C.

Beispiel 47

1-[7,8-Dimethoxy-1,3,4,5-tetrahydro-2H-3-benzazepin-2-on-3-yl]-3-[N-(2-(3,4-dimethoxy-phenyl)-ethyl)-amino]-propan-hydrochlorid

Hergestellt analog Beispiel 4 durch katalytische Hydrierung von 1-[7,8-Dimethoxy-1,3-dihydro-2H-3-benzazepin-2-on-3-yl]-3-[N-benzyl-N-(2-(3,4-dimethoxy-phenyl)-ethyl)-amino]-propan.
Ausbeute : 81 % der Theorie,
Schmelzpunkt : 152-154 °C.

Beispiel 48

1-[7,8-Dimethoxy-1,3,4,5-tetrahydro-2H-3-benzazepin-2-on-3-yl]-3-[N-allyl-N-(2-(3,4-dimethoxy-phenyl)-ethyl)-amino]-propan-hydrochlorid

3,1 g (0,007 Mol) 1-[7,8-Dimethoxy-1,3,4,5-tetrahydro-2H-3-benzazepin-2-on-3-yl]-3-[N-(2-(3,4-dimethoxy-phenyl)-ethyl)-amino]-propan werden mit 1,0 g (0,007 Mol) Kaliumcarbonat und 0,6 ml (0,007 Mol) Allylbromid 21 Stunden in 100 ml Chloroform am Rückfluß gekocht. Die organische Phase wird anschließend mit Wasser extrahiert, getrocknet, im Vakuum eingeengt und der erhaltene Rückstand über eine Aluminiumoxid-Säule (300 g, neutral, Aktivität III) gereinigt (Fließmittel : Methylenchlorid mit 2 % Aceton). Anschließend wird das Hydrochlorid gefällt.
Ausbeute : 40 % der Theorie,
Schmelzpunkt : 153-155 °C.

Beispiel 49

1-[7,8-Dimethoxy-1,3,4,5-tetrahydro-2H-3-benzazepin-2-on-3-yl]-3-[N-propyl-N-(2-(3,4-dimethoxy-phenyl)-ethyl)-amino]-propanhydrochlorid

Hergestellt analog Beispiel 48 aus 1-[7,8-Dimethoxy-1,3,4,5-tetrahydro-2H-3-benzazepin-2-on-3-yl]-3-[N-(2-(3,4-dimethoxyphenyl)-ethyl)-amino]-propan und 1-Brompropan.
Ausbeute : 53 % der Theorie,
Schmelzpunkt : 80 °C (Zers.).

Beispiel 50

Isomerengemisch von 1-[7,8-Dimethoxy-1,3-dihydro-2H-3-benzazepin-2-on-3-yl]-3-[N-methyl-N-(2-(2- und 4-nitro-phenyl)-ethyl)-amino]-propan

Hergestellt analog Beispiel 1b aus 1-(7,8-Dimethoxy-1,3-dihydro-2H-3-benzazepin-2-on-3-yl)-3-chlor-propan und N-Methyl-2-(4- und 2-nitro-phenyl)-ethylamin.
Ausbeute : 70 % der Theorie,
IR-Spektrum (Methylenchlorid) : 1 655 $cm^{-1}$ (CO)
1 510 und 1 345 $cm^{-1}$ ($NO_2$)
NMR-Spektrum ($CDCl_3/D_2O$) : δ = 8,10 ppm, d(J = 9Hz), 2H (aromat.), (4-Nitro-Verbindung),
δ = 7,83 ppm, d(J = 7Hz), 2H (aromat.), (2-Nitro-Verbindung).

Beispiel 51

Isomerengemisch von 1-[7,8-Dimethoxy-1,3-dihydro-2H-3-benzazepin-2-on-3-yl]-3-[N-methyl-N-(2-(2-amino- und 4-amino-phenyl)-ethyl)-amino]-propan-hydrochlorid

Hergestellt analog Beispiel 4 durch katalytische Reduktion eines Isomerengemisches von 1-[7,8-Dimethoxy-1,3-dihydro-2H-3-benzazepin-2-on-3-yl]-3-[N-methyl-N-(2-(2- und 4-nitro-phenyl)-ethyl)-amino]-propan.

Ausbeute : 34 % der Theorie,
Schmelzpunkt : 100 °C (Zers.).
IR-Spektrum (Methylenchlorid) : 1 660 $cm^{-1}$ (CO)
$C_{24}H_{31}N_3O_{35}$× HCl (446,0)
Ber. : C 64,34 H 7,65 N 9,38 Cl 7,91
Gef. : C 63,60 H 7,20 N 9,28 Cl 7,94

Beispiel 52

1-[7,8-Dimethoxy-1,3-dihydro-2H-3-benzazepin-2-on-3-yl]-3-[N-methyl-N-(2-(2-acetylamino-phenyl)-ethyl)-amino]-propan und
1-[7,8-Dimethoxy-1,3-dihydro-2H-3-benzazepin-2-on-3-yl]-3-[N-methyl-N-(2-(2-acetylamino-phenyl)-ethyl)-amino]-propan

1,8 g (0,004 Mol) eines Isomerengemisches von 1-[7,8-Dimethoxy-1,3-dihydro-2H-3-benzazepin-2-on-3-yl]-3-[N-methyl-N-(2-(2- und 4-amino-phenyl)-ethyl)-amino]-propan werden in enem Gemisch von 10 ml Eisessig und 10 ml Acetanhydrid eine Stunde bei Raumtemperatur gerührt. Anschließend wird mit Wasser versetzt, mit Natriumbicarbonat neutralisiert und mit Methylenchlorid extrahiert. Nach Trocknen der organischen Phase wird im Vakuum eingeengt und der Rückstand über Aluminiumoxid (200 g, neutral, Aktivität IV) chromatographiert (Elutionsmittel : Methylenchlorid + 1 % Methanol).
Ausbeute an 2-Acetylaminoverbindung : 0,24 g (14 % der Theorie),
Schmelzpunkt : 62-66 °C.
Ausbeute an 4-Acetylaminoverbindung : 0,5 g (28 % der Theorie),
Schmelzpunkt : 69-72 °C.

Beispiel 53

1-[7,8-Dimethoxy-1,3,4,5-tetrahydro-2H-1,3-benzodiazepin-2-on-3-yl]-3-[N-methyl-N-(2-(3,4-dimethoxy-phenyl)-ethyl)-amino]-propan

a) N-[3-[N'-Methyl-N'-(2-(3,4-dimethoxy-phenyl)-ethyl)-amino]-propyl]-2-(2-amino-4,5-dimethoxy-phenyl)-ethylamin

4,5 g (0,01 Mol) N-[3-[N'-Methyl-N'-(2-(3,4-dimethoxy-phenyl)-ethyl)-amino]-propyl]-2-(2-amino-4,5-dimethoxy-phenyl)-acetamid werden in 100 ml Tetrahydrofuran gelöst und unter Stickstoff-Atmosphäre mit 60 ml einer 1-molaren Diboran-Lösung in Tetrahydrofuran versetzt. Die klare Lösung wird 2 Tage bei Raumtemperatur stehen gelassen und dann mit 20 ml halbkonzentrierter Salzsäure unter Kühlung zersetzt. Im Vakuum wird das Tetrahydrofuran abdestilliert und der verbleibende salzsaure Rückstand mit konzentrierter Natronlauge unter Kühlung alkalisch gestellt. Das ausgefallene Öl wird in Methylenchlorid aufgenommen, die organische Lösung abgetrennt, getrocknet und im Vakuum eingeengt. Der ölige Rückstand wird über Kieselgel mit Methylenchlorid und 1 % Methanol gereinigt.
Ausbeute : 2,9 g (67,2 % der Theorie),
IR-Spektrum (Methylenchlorid) : 2 830 $cm^{-1}$ ($OCH_3$)
2 800 $cm^{-1}$ (N-Alkyl)

b) 1-[7,8-Dimethoxy-1,3,4,5-tetrahydro-2H-1,3-benzodiazepin-2-on-3-yl]-3-[N-methyl-(2-(3,4-dimethoxy-phenyl)-ethyl)-amino]-propan

Hergestellt analog Beispiel 2 durch Umsetzung von N-[3-[N'-Methyl-N'-(2-(3,4-dimethoxy-phenyl)-ethyl)-amino]-propyl]-2-(2-amino-4,5-dimethoxy-phenyl)-ethylamin mit N,N'-Carbonyldiimidazol.
Ausbeute : 0,9 g (61,6 % der Theorie),
Schmelzpunkt : 116-117 °C.

Beispiel 54

1-[7,8-Dimethoxy-5-methyl-1,3-dihydro-2H-3,4-benzodiazepin-2-on-3-yl]-3-[N-methyl-N-(2-(3,4-dimethoxy-phenyl)-ethyl)-amino]-propan-dihydrochlorid

Hergestellt analog Beispiel 1b durch Umsetzung von 1-(7,8-Dimethoxy-5-methyl-1,3-dihydro-2H-3,4-benzodiazepin-2-on-3-yl)-3-chlor-propan mit N-Methyl-2-(3,4-dimethoxy-phenyl)-ethylamin.
Ausbeute : 24,2 % der Theorie,
Schmelzpunkt : 106 °C.

Beispiel 55

1-[7,8-Dimethoxy-1,3-dihydro-2H-3-benzazepin-2-on-3-yl]-2-hydroxy-3-[N-methyl-N-(2-(3,4-dimethoxy-phenyl)-ethyl)-amino]-propan

a) 1-(7,8-Dimethoxy-1,3-dihydro-2H-3-benzazepin-2-on-3-yl)-2,3-epoxy-propan

Hergestellt analog Beispiel 1a durch Umsetzung von 7,8-Dimethoxy-1,3-dihydro-2H-3-benzazepin-2-on mit Epichlorhydrin.
Ausbeute : 94,5 % der Theorie,
IR-Spektrum (Methylenchlorid) : 2 830 $cm^{-1}$ ($OCH_3$)
1 660 $cm^{-1}$ (CO)

b) 1-[7,8-Dimethoxy-1,3-dihydro-2H-3-benzazepin-2-on-3-yl]-2-hydroxy-3-[N-methyl-N-(2-(3,4-dimethoxy-phenyl)-ethyl)-amino]-propan

8,25 g (0,03 Mol) 1-(7,8-Dimethoxy-1,3-dihydro-2H-3-benzazepin-2-on-3-yl)-2,3-epoxy-propan werden in 100 ml Methanol gelöst, mit 5,85 g (0,03 Mol) N-Methyl-2-(3,4-dimethoxy-phenyl)-ethylamin versetzt und 3 Stunden unter Rückfluß gekocht. Das Methanol wird im Vakuum abdestilliert und der Rückstand über eine Kieselgel-Säule mit Methylenchlorid + 1 % Ethanol gereinigt.
Ausbeute : 7,8 g (55,2 % der Theorie),
IR-Spektrum (Methylenchlorid) : 3 600 $cm^{-1}$ (OH)
1 650 $cm^{-1}$ (CO)
$C_{26}H_{34}N_2O_6$ (470,6)
Ber. : C 66,36 H 7,28 N 5,95
Gef. : C 66,16 H 7,26 N 5,80

Beispiel 56

1-[7,8-Dimethoxy-1,3,4,5-tetrahydro-2H-3-benzazepin-1,2-dion-3-yl]-3-[N-methyl-N-(2-(3,4-dimethoxy-phenyl)-ethyl)-amino]-propan-hydrochlorid

3,5 g Selendioxid werden bei 70 °C zu einer Mischung aus 150 ml Dioxan und 6 ml Wasser gegeben, 15 Minuten gerührt und dann mit 3 g Kieselgur und 14,8 g (0,03 Mol) 1-[7,8-Dimethoxy-1,3,4,5-tetrahydro-2H-3-benzazepin-2-on-3-yl]-3-[N-methyl-N-(2-(3,4-dimethoxy-phenyl)-ethyl)-amino]-propan-hydrochlorid versetzt. Das Gemisch wird 6 Stunden unter Rückfluß gekocht, abgekühlt und filtriert. Das Filtrat wird im Vakuum eingeengt und der Rückstand über eine Kieselgel-Säule mit Methylenchlorid + 1 % Ethanol als Elutionsmittel gereinigt. Die Fraktionen werden im Vakuum eingeengt, der Rückstand wird in Aceton gelöst und mit etherischer Salzsäure das Hydrochlorid gefällt.
Ausbeute : 13,8 g (90,7 % der Theorie),
Schmelzpunkt : 196-197 °C.

Beispiel 57

1-[7,8-Dimethoxy-1,3,4,5-tetrahydro-2H-3-benzazepin-2-on-3-yl]-2-hydroxy-3-[N-methyl-N-(2-(3,4-dimethoxy-phenyl)-ethyl)-amino]-propan

Hergestellt analog Beispiel 4 durch katalytische Hydrierung von 1-[7,8-Dimethoxy-1,3-dihydro-2H-3-benzazepin-2-on-3-yl]-2-hydroxy-3-[N-methyl-N-(2-(3,4-dimethoxy-phenyl)-ethyl)-amino]-propan, jedoch in Ethanol bei 50 bar und 70 °C mit Platinoxid als Katalysator.
Ausbeute : 37,5 % der Theorie,
IR-Spektrum (Methylenchlorid) : 3 470 $cm^{-1}$ (OH)
1 635 $cm^{-1}$ (CO)
$C_{26}H_{36}N_2O_6$ (472,6)
Ber. : C 66,08 H 7,68 N 5,93
Gef. : C 66,22 H 7,57 N 5,85

Beispiel 58

1-[7,8-Dimethoxy-1,3,4,5-tetrahydro-2H-3-benzazepin-2-on-3-yl]-3-[N-methyl-N-(2-(4-nitro-phenyl)-ethyl)-amino]-propan-hydrochlorid

Hergestellt analog Beispiel 5b durch Umsetzung von 1-[7,8-Dimethoxy-1,3,4,5-tetrahydro-2H-3-benzazepin-2-on-3-yl]-chlorpropan mit N-Methyl-2-(4-nitro-phenyl)-ethylamin.

Ausbeute : 56,5 % der Theorie,
Schmelzpunkt : 182 °C.

Beispiel 59

1-[7,8-Dimethoxy-1,3,4,5-tetrahydro-2H-3-benzazepin-2-on-3-yl]-3-[N-methyl-N-(2-(3-nitro-4-acetylamino-phenyl)-ethyl)-amino]-propan-dihydrochlorid

0,3 g (1,18 m Mol) N-[3-(7,8-Dimethoxy-1,3,4,5-tetrahydro-2H-3-benzazepin-2-on-3-yl)-propyl]-methylamin und 0,34 g (1,3 mMol) 2-(3-Nitro-4-acetylamino-phenyl)-ethylbromid werden in 5 ml Chlorbenzol und 0,1 ml Pyridin eine Stunde unter Rückfluß gekocht. Das Reaktionsgemisch wird abgekühlt und das ausgefallene Pyridinhydrobromid abgesaugt. Das Filtrat wird im Vakuum eingedampft und der Rückstand über Aluminiumoxid (neutral, Aktivität II) mit Methylenchlorid und 0,5 % Ethanol als Elutionsmittel gereinigt. Das so erhaltene Öl wird in Aceton gelöst und mit etherischer Salzsäure das Dihydrochlorid gefällt.
Ausbeute : 230 mg (57,7 % der Theorie),
Schmelzpunkt : 170 °C.

Beispiel 60

1-[7,8-Dimethoxy-1,3,4,5-tetrahydro-2H-3-benzazepin-2-on-3-yl]-3-[N-methyl-N-(2-(4-fluor-phenyl)-ethyl)-amino]-propan-dihydrochlorid

Hergestellt analog Beispiel 5b durch Umsetzung von 1-(7,8-Dimethoxy-1,3,4,5-tetrahydro-2H-3-benzazepin-2-on-3-yl)-3-chlorpropan mit N-Methyl-2-(4-fluor-phenyl)-ethylamin.
Ausbeute : 68,7 % der Theorie,
Schmelzpunkt : 203 °C.

Beispiel 61

1-[7,8-Dimethoxy-1,3,4,5-tetrahydro-2H-3-benzazepin-2-on-3-yl]-3-[N-acetyl-N-(2-(3,4-dimethoxy-phenyl)-ethyl)-amino]-propan

2 g (0,004 5 Mol) 1-[7,8-Dimethoxy-1,3,4,5-tetrahydro-2H-3-benzazepin-2-on-3-yl]-3-[N-(2-(3,4-dimethoxy-phenyl)-ethyl)-amino]-propan werden mit 15 ml Acetanhydrid versetzt und 1/2 Stunde bei Raumtemperatur gerührt. Das Gemisch wird auf Eiswasser gegossen, mit Natriumbicarbonat neutralisiert und anschließend mit Methylenchlorid extrahiert. Der organische Extrakt wird über Magnesiumsulfat getrocknet, filtriert und im Vakuum eingeengt. Der Rückstand wird über Aluminiumoxid (neutral, Aktivität II) mit Methylenchlorid als Elutionsmittel gereinigt.
Ausbeute : 1,5 g (68,5 % der Theorie),
IR-Spektrum (Methylenchlorid) : 2 830 $cm^{-1}$ ($OCH_3$)
1 640 $cm^{-1}$ (CO)
$C_{27}H_{36}N_2O_6$ (484,6)
Ber. : C 66,92 H 7,49 N 5,78
Gef. : C 66,75 H 7,44 N 5,80

Beispiel 62

1-[7,8-Dimethoxy-1,3,4,5-tetrahydro-2H-3-benzazepin-2-on-3-yl]-3-[N-ethoxycarbonyl-N-(2-(3,4-dimethoxy-phenyl)-ethyl)-amino]-propan

2,5 g (0,005 65 Mol) 1-[7,8-Dimethoxy-1,3,4,5-tetrahydro-2H-3-benzazepin-2-on-3-yl]-3-[N-(2-(3,4-dimethoxy-phenyl)-ethyl)-amino]-propan und 2,45 g (0,024 Mol) Triethylamin werden in 20 ml Methylenchlorid gelöst. Nach Zusatz von 2,6 g (0,024 Mol) Chlorameisensäureethylester wird 30 Minuten bei Raumtemperatur nachgerührt, die Lösung zweimal mit Wasser gewaschen, über Magnesiumsulfat getrocknet und im Vakuum eingeengt. Der Rückstand wird über Aluminiumoxid (neutral, Aktivität II) mit Methylenchlorid als Elutionsmittel gereinigt.
Ausbeute : 1,5 g (51,6 % der Theorie),
IR-Spektrum (Methylenchlorid) : 1 690, 1 650 $cm^{-1}$ (CO)
$C_{28}H_{38}N_2O_7$ (514,6)
Ber. : C 65,30 H 7,44 N 5,44
Gef. : C 64,19 H 7,14 N 5,27

Beispiel 63

1-[7,8-Dimethoxy-1,3,4,5-tetrahydro-2H-3-benzazepin-2-on-3-yl]-3-[N-methyl-N-(2-(2,6-dichlor-phenyl)-ethyl)-amino]-propan-dihydrochlorid

Hergestellt analog Beispiel 5b durch Umsetzung von 1-(7,8-Dimethoxy-1,3,4,5-tetrahydro-2H-3-benzazepin-2-on-3-yl)-3-chlorpropan mit N-Methyl-2-(2,6-dichlor-phenyl)-ethylamin.
Ausbeute : 70,5 % der Theorie,
Schmelzpunkt : 147 °C.

Beispiel 64

1-[7,8-Dimethoxy-1,3,4,5-tetrahydro-2H-3-benzazepin-2-on-3-yl]-3-[N-methyl-N-(2-(3,4-dichlor-phenyl)-ethyl)-amino]-propan-dihydrochlorid

Hergestellt analog Beispiel 5b durch Umsetzung von 1-(7,8-Dimethoxy-1,3,4,5-tetrahydro-2H-3-benzazepin-2-on-3-yl)-3-chlorpropan mit N-Methyl-2-(3,4-dichlor-phenyl)-ethylamin.
Ausbeute : 57,6 % der Theorie,
Schmelzpunkt : 161 °C.

Beispiel 65

1-[7,8-Dimethoxy-1,3-dihydro-2H-3-benzazepin-2-on-3-yl]-2-[N-methyl-N-(2-(3,4-dimethoxy-phenyl)-ethyl)-amino]-ethan

a) 1-(7,8-Dimethoxy-1,3-dihydro-2H-3-benzazepin-2-on-3-yl)-2-chlor-ethan

Hergestellt analog Beispiel 1a durch Umsetzung von 7,8-Dimethoxy-1,3-dihydro-2H-3-benzazepin-2-on mit 1-Chlor-2-bromethan.
Ausbeute : 20 % der Theorie,
Schmelzpunkt : 114 °C.

b) 1-[7,8-Dimethoxy-1,3-dihydro-2H-3-benzazepin-2-on-3-yl]-2-[N-methyl-N-(2-(3,4-dimethoxy-phenyl)-ethyl)-amino]-ethan

Hergestellt analog Beispiel 1b durch Umsetzung von 1-(7,8-Dimethoxy-1,3-dihydro-2H-3-benzazepin-2-on-3-yl)-2-chlorethan mit N-Methyl-2-(3,4-dimethoxy-phenyl)-ethylamin.
Ausbeute : 72 % der Theorie,
IR-Spektrum (Methylenchlorid) : 2 830 $cm^{-1}$ ($OCH_3$)
2 790 $cm^{-1}$ (N-Alkyl)
1 655 $cm^{-1}$ (CO)
NMR-Spektrum ($CDCl_3$) : δ : 2,3 ppm, s, 3H ($NCH_3$) ; 3,85 ppm, s, 12H ($OCH_3$) ; 6,15 ppm, d(J = 8Hz), 1H (olefin.) ; 6,35, d(J = 8Hz), 1H (olefin.).

Beispiel 66

1-[7,8-Dimethoxy-1,3,4,5-tetrahydro-2H-3-benzazepin-2-on-3-yl]-2-[N-methyl-N-(2-(3,4-dimethoxy-phenyl)-ethyl)-amino]-ethan-hydrochlorid.

Hergestellt analog Beispiel 4 durch katalytische Hydrierung von 1-[7,8-Dimethoxy-1,3-dihydro-2H-3-benzazepin-2-on-3-yl]-2-[N-methyl-N-(2-(3,4-dimethoxy-phenyl)-ethyl)-amino]-ethan.
Ausbeute : 59 % der Theorie,
Schmelzpunkt : 188-189 °C.

Beispiel 67

1-[7,8-Dimethoxy-1,3,4,5-tetrahydro-2H-3-benzazepin-2,4-dion-3-yl]-3-[N-methyl-N-(2-(3,4-dimethoxy-phenyl)-ethyl)-amino]-propan-hydrochlorid

a) 1-(7,8-Dimethoxy-1,3,4,5-tetrahydro-2H-3-benzazepin-2,4-dion-3-yl)-3-chlor-propan

Hergestellt analog Beispiel 1a durch Umsetzung von 7,8-Dimethoxy-1,3,4,5-tetrahydro-2H-3-benzazepin-2,4-dion (Schmelzpunkt : 235 °C (Zers.)) mit 1-Brom-3-chlorpropan.
Ausbeute : 26 % der Theorie,
IR-Spektrum (KBr) : 1 660 $cm^{-1}$ (CO)

b) 1-[7,8-Dimethoxy-1,3,4,5-tetrahydro-2H-3-benzazepin-2,4-dion-3-yl]-3-[N-methyl-N-(2-(3,4-dimethoxy-phenyl)-ethyl)-amino]-propan-hydrochlorid

Hergestellt analog Beispiel 5b durch Umsetzung von 1-(7,8-Dimethoxy-1,3,4,5-tetrahydro-2H-3-benzazepin-2,4-dion-3-yl)-3-chlor-propan mit N-Methyl-2-(3,4-dimethoxy-phenyl)-ethyl-amin.

Ausbeute : 35 % der Theorie,

Schmelzpunkt : 163-164 °C.

Beispiel 68

1-[7-Benzyloxy-8-hydroxy-1,3,4,5-tetrahydro-2H-3-benzazepin-2-on-3-yl]-3-[N-methyl-N-(2-(3,4-dimethoxy-phenyl)-ethyl)-amino]-propan

a) 1-(7,8-Dihydroxy-1,3,4,5-tetrahydro-2H-3-benzazepin-2-on-3-yl)-3-chlor-propan

8,9 g (0,03 Mol) 1-(7,8-Dimethoxy-1,3,4,5-tetrahydroxy-2H-3-benzazepin-2-on-3-yl)-3-chlor-propan werden in 100 ml Methylenchlorid gelöst und bei – 60 °C mit 2,1 ml Bortribromid versetzt. Man läßt die Reaktionstemperatur langsam auf 20 °C ansteigen und rührt bei dieser Temperatur 10 Stunden nach. Die harzige Fällung wird durch Zugabe von 100 ml Wasser und anschließendem 1-stündigem Rühren in eine kristalline Form gebracht. Der Niederschlag wird abgesaugt und mit Wasser und Methylenchlorid nachgewaschen. Zur Reinigung wird das Kristallisat mit Aceton verrührt und abgesaugt.

Ausbeute : 7,3 g (90,2 % der Theorie),

Schmelzpunkt : 177-178 °C.

b) 1-(7-Hydroxy-8-benzyloxy-1,3,4,5-tetrahydro-2H-3-benzazepin-2-on-3-yl)-3-chlor-propan und 1-(7-Benzyloxy-8-hydroxy-1,3,4,5-tetrahydro-2H-3-benzazepin-2-on-3-yl)-3-chlor-propan

19 g (0,07 Mol) 1-(7,8-Dihydroxy-1,3,4,5-tetrahydro-2H-3-benzazepin-2-on-3-yl)-3-chlor-propan und 10,6 g Kaliumcarbonat in 250 ml Dimethylsulfoxid werden mit 19,5 g (0,154 Mol) Benzylchlorid versetzt. Das Gemisch wird 2 Tage bei Raumtemperatur gerührt, auf Eiswasser gegossen und mehrmals mit Essigester extrahiert. Die organischen Extrakte werden 3 mal mit Wasser gewaschen, über Magnesiumsulfat getrocknet und im Vakuum eingeengt. Die Isomerentrennung erfolgt über Kieselgel mit Methylenchlorid und 3 % Aceton als Elutionsmittel.

Ausbeute an 7-Hydroxy-Verbindung : 6 g (23,8 % der Theorie), Schmelzpunkt : 163-165 °C,

Ausbeute an 8-Hydroxy-Verbindung : 4,5 g (17,9 % der Theorie), Schmelzpunkt : 185-186 °C.

c) 1-[7-Benzyloxy-8-hydroxy-1,3,4,5-tetrahydro-2H-3-benzazepin-2-on-3-yl]-3-[N-methyl-N-(2-(3,4-dimethoxy-phenyl)-ethyl)-amino]-propan

Hergestellt analog Beispiel 5b durch Umsetzung von 1-(7-Benzyloxy-8-hydroxy-1,3,4,5-tetrahydro-2H-3-benzazepin-2-on-3-yl)-3-chlor-propan mit N-Methyl-2-(3,4-dimethoxy-phenyl)-ethylamin.

Ausbeute : 75,4 % der Theorie,

Schmelzpunkt : 128-129 °C

Beispiel 69

1-[7-Hydroxy-8-benzyloxy-1,3,4,5-tetrahydro-2H-3-benzazepin-2-on-3-yl]-3-[N-methyl-N-(2-(3,4-dimethoxy-phenyl)-ethyl)-amino]-propan

Hergestellt analog Beispiel 5b durch Umsetzung von 1-(7-Hydroxy-8-benzyloxy-1,3,4,5-tetrahydro-2H-3-benzazepin-2-on-3-yl)-3-chlor-propan mit N-Methyl-2-(3,4-dimethoxy-phenyl)-ethylamin.

Ausbeute : 47,2 % der Theorie,

Schmelzpunkt : 157-158 °C.

Beispiel 70

1-[7,8-Dihydro-1,3,4,5-tetrahydro-2H-3-benzazepin-2-on-3-yl]-3-[N-methyl-N-(2-(3,4-dimethoxy-phenyl)-ethyl)-amino]-prnnan

0,52 g (0,001 Mol) 1-[7-Hydroxy-8-benzyloxy-1,3,4,5-tetrahydro-2H-3-benzazepin-2-on-3-yl]-3-[N-methyl-N-(2-(3,4-dimethoxy-phenyl)-ethyl)-amino]-propan werden 5 Stunden bei 20 °C und 5 bar in 100 ml Methanol und 0,2 g Palladium/Kohle (10 %ig) hydriert. Der Katalysator wird abgesaugt, das Filtrat im Vakuum eingeengt und der Rückstand über Kieselgel mit Methylenchlorid + 1 % Ethanol als Elutionsmittel gereinigt.

Ausbeute : 0,2 g (43,9 % der Theorie),

IR-Spektrum (Methylenchlorid) : 3 520 $cm^{-1}$ (OH)

1 640 $cm^{-1}$ (CO)

$C_{24}H_{32}N_2O_5 \times 1/2\ H_2O$ (455,5)

Ber.: C 63,28 H 7,74 N 6,15
Gef.: C 63,44 H 7,77 N 6,13

Beispiel 71

1-[7-Benzyloxy-8-methoxy-1,3,4,5-tetrahydro-2H-3-benzazepin-2-on-3-yl]-3-[N-methyl-N-(2-(3,4-dimethoxy-phenyl)-ethyl)-amino]-propan-dihydrochlorid

0,52 g (0,001 Mol) 1-[7-Benzyloxy-8-hydroxy-1,3,4,5-tetrahydro-2H-3-benzazepin-2-on-3-yl]-3-[N-methyl-N-(2-(3,4-dimethoxyphenyl)-ethyl)-amino]-propan werden in 30 ml Dimethylformamid gelöst und mit 50 mg 50 %iger Natriumhydrid-Dispersion (in Öl) versetzt. Das Gemisch wird 30 Minuten auf 60 °C erwärmt, mit 0,1 ml Dimethylsulfat versetzt und weitere 2 Stunden auf 60 °C erwärmt. Das Dimethylformamid wird im Vakuum abdestilliert, der Rückstand in Methylenchlorid aufgenommen, die organische Phase mit Wasser gewaschen, getrocknet und im Vakuum eingeengt. Der harzige Rückstand wird über Aluminiumoxid (neutral, Aktivität II) mit Methylenchlorid als Elutionsmittel gereinigt. Das erhaltene Öl wird in Aceton gelöst und durch Versetzen mit etherischer Salzsäure das Dihydrochlorid gefällt.
Ausbeute: 250 mg (41 % der Theorie),
Schmelzpunkt: 117-120 °C.

Beispiel 72

1-[7-Methoxy-8-benzyloxy-1,3,4,5-tetrahydro-2H-3-benzazepin-2-on-3-yl]-3-[N-methyl-N-(2-(3,4-dimethoxy-phenyl)-ethyl)-amino]-propan

Hergestellt analog Beispiel 71 durch Umsetzung von 1-[7-Hydroxy-8-benzyloxy-1,3,4,5-tetrahydro-2H-3-benzazepin-2-on-3-yl]-3-[N-methyl-N-(2-(3,4-dimethoxy-phenyl)-ethyl)-amino]-propan mit Dimethylsulfat.
Ausbeute: 70 % der Theorie,
IR-Spektrum (Methylenchlorid): 1 655 $cm^{-1}$ (CO)
NMR-Spektrum ($CDCl_3/D_2O$): δ = 2,3 ppm, s, 3H ($NCH_3$); 5,1 ppm, s, 2H (benzyl.); 6,5-6,8 ppm, m, 5H (aromat.); 7,4 ppm, s, 5H (aromat.).

Beispiel 73

1-[7-Hydroxy-8-methoxy-1,3,4,5-tetrahydro-2H-3-benzazepin-2-on-3-yl]-3-[N-methyl-N-(2-(3,4-dimethoxy-phenyl)-ethyl)-amino]-propan-hydrochlorid

Hergestellt analog Beispiel 70 aus 1-[7-Benzyloxy-8-methoxy-1,3,4,5-tetrahydro-2H-3-benzazepin-2-on-3-yl]-3-[N-methyl-N-(2-(3,4-dimethoxy-phenyl)-ethyl)-amino]-propan-hydrochlorid durch katalytische Debenzylierung.
Ausbeute: 45,2 % der Theorie,
IR-Spektrum (Methylenchlorid): 3 530 $cm^{-1}$ (OH)
2 830 $cm^{-1}$ ($OCH_3$)
1 650 $cm^{-1}$ (CO)
$C_{25}H_{34}N_2O_5 \times HCl$ (479,0)
Ber.: C 62,69 H 7,36 N 5,85
Gef.: C 63,15 H 7,57 N 5,64

Beispiel 74

1-[7-Methoxy-8-hydroxy-1,3,4,5-tetrahydro-2H-3-benzazepin-2-on-3-yl]-3-(N-methyl-N-(2-(3,4-dimethoxy-phenyl)-ethyl)-amino]-propan

Hergestellt analog Beispiel 70 aus 1-[7-Methoxy-8-benzyloxy-1,3,4,5-tetrahydro-2H-3-benzazepin-2-on-3-yl]-3-[N-methyl-N-(2-(3,4-dimethoxy-phenyl)-ethyl)-amino]-propan durch katalytische Debenzylierung.
Ausbeute: 29,4 % der Theorie,
IR-Spektrum (Methylenchlorid): 1 640 $cm^{-1}$ (CO)
$C_{25}H_{34}N_2O_5$ (442,56)
Ber.: C 67,85 H 7,74 N 6,33
Gef.: C 67,50 H 7,97 N 6,18

Beispiel 75

1-[7,8-Dimethoxy-1,3,4,5-tetrahydro-2H-3-benzazepin-2-on-3-yl]-3-[N-methyl-N-(2-(3,4-dimethyl-phenyl)-ethyl)-amino]-propan-dihydrochlorid

Hergestellt analog Beispiel 5b durch Umsetzung von 1-(7,8-Dimethoxy-1,3,4,5-tetrahydro-2H-3-benzazepin-2-on-3-yl)-3-chlor-propan mit N-Methyl-2-(3,4-dimethyl-phenyl)-ethylamin.
Ausbeute : 54,3 % der Theorie,
Schmelzpunkt : 170-172 °C

Beispiel 76

1-[7,8-Dimethoxy-1,3,4,5-tetrahydro-2H-3-benzazepin-2-on-3-yl]-3-[N-methyl-N-(2-(4-tert.butyl-phenyl)-ethyl)-amino]-propan-dihydrochlorid

Hergestellt analog Beispiel 5b durch Umsetzung von 1-(7,8-Dimethoxy-1,3,4,5-tetrahydro-2H-3-benzazepin-2-on-3-yl)-3-chlor-propan mit N-methyl-2-(4-tert. butyl-phenyl)-ethylamin.
Ausbeute : 49,4 % der Theorie,
Schmelzpunkt : 146-149 °C.

Beispiel 77

1-[7,8-Dimethoxy-1,3,4,5-tetrahydro-2H-3-benzazepin-2-on-3-yl]-3-[N-methyl-N-(2-(4-n-butoxy-phenyl)-ethyl)-amino]-propan

Hergestellt analog Beispiel 5b durch Umsetzung von 1-(7,8-Dimethoxy-1,3,4,5-tetrahydro-2H-3-benzazepin-2-on-3-yl)-3-chlor-propan mit N-Methyl-2-(4-butoxy-phenyl)-ethylamin.
Ausbeute : 55,3 % der Theorie,
Schmelzpunkt : 67-69 °C.

Beispiel 78

1-[7,8-Dimethoxy-1,3,4,5-tetrahydro-2H-3-benzazepin-2-on-3-yl]-3-[N-methyl-N-(2-(2,4,6-trimethoxy-phenyl)-ethyl)-amino]-propan

Hergestellt analog Beispiel 5b durch Umsetzung von 1-(7,8-Dimethoxy-1,3,4,5-tetrahydro-2H-3-benzazepin-2-on-3-yl)-3-chlor-propan mit N-Methyl-2-(2,4,6-trimethoxy-phenyl)-ethylamin.
Ausbeute : 57,8 % der Theorie,
IR-Spektrum (Methylenchlorid) : 1 650 $cm^{-1}$ (CO)
1 520 $cm^{-1}$ (aromat. C=C)
$C_{27}H_{38}N_2O_6 \times HCl$ (523,2)

| | | | | |
|---|---|---|---|---|
| Ber. : | C 62,00 | H 7,51 | N 5,35 | Cl 6,77 |
| Ger. : | C 61,75 | H 7,52 | N 5,18 | Cl 7,34 |

Beispiel 79

1-[7,8-Dimethyl-1,3-dihydro-2H-3-benzazepin-2-on-3-yl]-3-[N-methyl-N-(2-(3,4-dimethoxy-phenyl)-ethyl)-amino]-propan-hydrochlorid

a) 1-(7,8-Dimethyl-1,3-dihydro-2H-3-benzazepin-2-on-3-yl)-3-chlor-propan

Hergestellt analog Beispiel 1a durch Umsetzung von 7,8-Dimethyl-1,3-dihydro-2H-3-benzazepin-2-on (Schmelzpunkt : 220-224 °C) mit 1-Brom-3-chlor-propan.
Ausbeute : 99 % der Theorie,
Schmelzpunkt : 62-64 °C.

b) 1-[7,8-Dimethyl-1,3-dihydro-2H-3-benzazepin-2-on-3-yl]-3-[N-methyl-N-(2-(3,4-dimethoxy-phenyl)-ethyl)-amino]-propan-hydrochlorid

Hergestellt analog Beispiel 1b durch Umsetzung von 1-(7,8-Dimethyl-1,3-dihydro-2H-3-benzazepin-2-on-3-yl)-3-chlor-propan mit N-Methyl-2-(3,4-dimethoxy-phenyl)-ethylamin.
Ausbeute : 70,9 % der Theorie,
Schmelzpunkt : 105-107 °C.

Beispiel 80

1-[7,8-Dimethyl-1,3,4,5-tetrahydro-2H-3-benzazepin-2-on-3-yl]-3-(N-methyl-N-(2-(3,4-dimethoxy-phenyl-ethyl)-amino]-propan-dihydrochlorid

Hergestellt analog Beispiel 4 durch katalytische Hydrierung von 1-[7,8-Dimethyl-1,3-dihydro-2H-3-

benzazepin-2-on-3-yl]-3-[N-methyl-N-2-(3,4-dimethoxy-phenyl)-ethyl)-amino]-propan.
Ausbeute : 83,8 % der Theorie,
Schmelzpunkt : 154-157 °C.

Beispiel 81

1-[7,8-Dimethoxy-1,3-dihydro-2H-3-benzazepin-2-on-3-yl]-2-methyl-3-[N-methyl-N-(2-(3,4-dimethoxy-phenyl)-ethyl)-amino]-propan

a) 1-(7,8-Dimethoxy-1,3-dihydro-2H-3-benzazepin-2-on-3-yl)-2-methyl-3-chlor-propan

Hergestellt analog Beispiel 1a durch Umsetzung von 7,8-Dimethoxy-1,3-dihydro-2H-3-benzazepin-2-on mit 1-Brom-2-methyl-3-chlor-propan.
Ausbeute : 97,5 % der Theorie,
Schmelzpunkt : 45-47 °C.

b) 1-[7,8-Dimethoxy-1,3-dihydro-2H-3-benzazepin-2-on-3-yl]-2-methyl-3-[N-methyl-N-(2-(3,4-dimethoxy-phenyl)-ethyl)-amino]-propan

Hergestellt analog Beispiel 1b durch Umsetzung von 1-(7,8-Dimethoxy-1,3-dihydro-2H-3-benzazepin-2-on-3-yl)-2-methyl-3-chlor-propan mit N-Methyl-2-(3,4-dimethoxy-phenyl)-ethylamin.
Ausbeute : 36 % der Theorie,
Schmelzpunkt : 30-32 °C.

Beispiel 82

1-[7,8-Dimethoxy-1,3,4,5-tetrahydro-2H-3-benzazepin-2-on-3-yl]-2-methyl-3-[N-methyl-N-(2-(3,4-dimethoxy-phenyl)-ethyl)-amino]-propan-hydrochlorid

Hergestellt analog Beispiel 4 durch katalytische Hydrierung von 1-[7,8-Dimethoxy-1,3-dihydro-2H-3-benzazepin-2-on-3-yl]-2-methyl-3-[N-methyl-N-(2-(3,4-dimethoxy-phenyl)-ethyl)-amino]-propan.
Ausbeute : 73,7 % der Theorie,
Schmelzpunkt : 99-101 °C.

Beispiel 83

1-[7,8-dimethoxy-1,3,4,5-tetrahydro-2H-3-benzazepin-1,2-dion-3-yl]-3-[N-methyl-N-(2-(3,4-dimethoxy-phenyl)-ethyl)-amino]-propan-hydrochlorid

2,45 g (0,005 Mol) 1-[7,8-Dimethoxy-1,3,4,5-tetrahydro-2H-3-benzazepin-2-on-3-yl]-3-[N-methyl-N-(2-(3,4-dimethoxy-phenyl)-ethyl)-amino]-propan-hydrochlorid werden in 50 ml Eisessig vorgelegt und mit 3 g Natriumdichromat × 2 $H_2O$ versetzt. Das Gemisch wird 2 Stunden bei Raumtemperatur gerührt, auf Eiswasser gegossen, mit Kaliumcarbonat neutralisiert und mehrmals mit Methylenchlorid ausgeschüttelt. Die organischen Extrakte werden über Magnesiumsulfat getrocknet und im Vakuum eingeengt. Der Rückstand wird über eine Kieselgel-Säule mit Methylenchlorid + 1 % Ethanol als Elutionsmittel gereinigt. Das so erhaltene Produkt wird in Aceton gelöst und mit etherischer Salzsäure das Hydrochlorid gefällt.
Ausbeute : 1,3 g (51,3 % der Theorie),
Schmelzpunkt : 197-198 °C.

Beispiel 84

1-[7,8-Dimethoxy-1,3,4,5-tetrahydro-2H-3-benzazepin-1,2-dion-3-yl]-3-[N-methyl-N-(2-(3,4-dimethoxy-phenyl)-ethyl)-amino]-propan-hydrochlorid

1,98 g (4,0 mMol) 1-[7,8-Dimethoxy-1,3,4,5-tetrahydro-2H-3-benzazepin-2-on-3-yl]-3-[N-methyl-N-(2-(3,4-dimethoxy-phenyl)-ethyl)-amino]-propan-hydrochlorid werden in 50 ml Acetanhydrid suspendiert, mit 2,5 g Kaliumpermanganat versetzt und 20 Minuten bei 20 °C gerührt. Das Reaktionsgemisch wird auf Eiswasser gegossen und mit konz. Ammoniak ammoniakalisch gestellt. Die Braunstein-Fällung wird abgesaugt und das Filtrat mehrmals mit Methylenchlorid ausgeschüttelt. Der Extrakt wird über Magnesiumsulfat getrocknet, im Vakuum eingeengt und der Rückstand über eine Kieselgel-Säule mit Methylenchlorid + 1 % Ethanol als Elutionsmittel gereinigt. Das so erhaltene Produkt wird in Aceton gelöst und mit etherischer Salzsäure das Hydrochlorid gefällt.
Ausbeute : 0,7 g (34,5 % der Theorie),
Schmelzpunkt : 196-197 °C.

Beispiel 85

1-[6,9-Dimethoxy-1,3-dihydro-2H-3-benzazepin-2-on-3-yl]-3-[N-methyl-N-(2-(3,4-dimethoxy-phenyl)-ethyl)-amino]-propan

a) 1-(6,9-Dimethoxy-1,3-dihydro-2H-3-benzazepin-2-on-3-yl)-3-chlor-propan

Hergestellt analog Beispiel 1a durch Umsetzung von 6,9-Dimethoxy-1,3-dihydro-2H-3-benzazepin-2-on (Schmelzpunkt : 188-191 °C) mit 1-Brom-3-Chlor-propan.
Ausbeute : 27 % der Theorie,
Schmelzpunkt : 97-99 °C.

b) 1-[6,9-Dimethoxy-1,3-dihydro-2H-3-benzazepin-2-on-3-yl]-3-[N-methyl-N-(2-(3,4-dimethoxy-phenyl)-ethyl)-amino]-propan

Hergestellt analog Beispiel 1b durch Umsetzung von 1-(6,9-Dimethoxy-1,3-dihydro-2H-3-benzazepin-2-on-3-yl)-3-chlor-propan mit N-Methyl-2-(3,4-dimethoxy-phenyl)-ethylamin.
Ausbeute : 90 % der Theorie,
IR-Spektrum (Methylenchlorid) : 1 658 $cm^{-1}$ (CO)
NMR-Spektrum ($CDCl_3/D_2O$) : $\delta$ = 2,2 ppm, s, 3H ($NCH_3$) ; 3,7-3,8 ppm, 4s, 12H ($OCH_3$) ; 6,25 ppm, d (J = 9 Hz), 1H (olefin.).

Beispiel 86

1-[6,9-Dimethoxy-1,3,4,5-tetrahydro-2H-benzazepin-2-on-3-yl]-3-[N-methyl-N-(2-(3,4-dimethoxy-phenyl)-ethyl)-amino]-propan-hydrochlorid

Hergestellt analog Beispiel 4 durch katalytische Hydrierung von 1-[6,9-Dimethoxy-1,3-dihydro-2H-3-benzazepin-2-on-3-yl]-3-[N-methyl-N-(2-(3,4-dimethoxy-phenyl)-ethyl)-amino]-propan.
Ausbeute : 85 % der Theorie,
Schmelzpunkt : 73-76 °C.

Beispiel 87

1-[8,9-Dimethoxy-1,3-dihydro-2H-3-benzazepin-2-on-3-yl]-3-[N-methyl-N-(2-(3,4-dimethoxy-phenyl)-ethyl)-amino]-propan-hydrochlorid

a) 1-(8,9-Dimethoxy-1,3-dihydro-2H-3-benzazepin-2-on-3-yl)-3-chlor-propan

Hergestellt analog Beispiel 1a durch Umsetzung von 8,9-Dimethoxy-1,3-dihydro-2H-3-benzazepin-2-on (Schmelzpunkt : 165-168 °C) mit 1-Brom-3-chlorpropan.
Ausbeute : 45 % der Theorie,
Schmelzpunkt : 67-71 °C.

b) 1-[8,9-Dimethoxy-1,3-dihydro-2H-3-benzazepin-2-on-3-yl]-3-[N-methyl-N-(2-(3,4-dimethoxy-phenyl)-ethyl)-amino]-propan-hydrochlorid

Hergestellt analog Beispiel 1b durch Umsetzung von 1-(8,9-Dimethoxy-1,3-dihydro-2H-3-benzazepin-2-on-3-yl)-3-chlor-propan mit N-Methyl-2-(3,4-dimethoxy-phenyl)-ethylamin.
Ausbeute : 64 % der Theorie,
Schmelzpunkt : 64-68 °C.

Beispiel 88

1-[8,9-Dimethoxy-1,3,4,5-tetrahydro-2H-3-benzazepin-2-on-3-yl]-3-[N-methyl-N-(2-(3,4-dimethoxy-phenyl)-ethyl)-amino]-propan-hydrochlorid

Hergestellt analog Beispiel 4 durch katalytische Hydrierung von 1-[8,9-Dimethoxy-1,3-dihydro-2H-3-benzazepin-2-on-3-yl]-3-[N-methyl-N-(2-(3,4-dimethoxy-phenyl)-ethyl)-amino]-propan.
Ausbeute : 75 % der Theorie,
Schmelzpunkt : 131-133 °C.

Beispiel 89

1-[7,8-Dimethoxy-1,3,4,5-tetrahydro-2H-3-benzazepin-2-on-3-yl]-3-[N-methyl-N-(2-(3,4,5-trimethoxy-phenyl)-ethyl)-amino]-propan-dihydrochlorid

Hergestellt analog Beispiel 5b durch Umsetzung von 1-(7,8-Dimethoxy-1,3,4,5-tetrahydro-2H-3-benzazepin-2-on-3-yl)-3-chlorpropan mit N-Methyl-2-(3,4,5-trimethoxy-phenyl)-ethylamin.
Ausbeute : 44 % der Theorie,
Schmelzpunkt : 131 °C (Zersetzung).

Beispiel 90

Isomerengemisch von 1-[7,8-Dimethoxy-1,3,4,5-tetrahydro-2H-3-benzazepin-2-on-3-yl]-3-[N-methyl-N-(2-(2- und 4-nitro-phenyl)-ethyl)-amino]-propan

Hergestellt analog Beispiel 1b aus 1-[7,8-Dimethoxy-1,3,4,5-tetrahydro-2H-3-benzazepin-2-on-3-yl]-3-chlorpropan und N-Methyl-2-(2- und 4-nitro-phenyl)-ethylamin.
Ausbeute : 72 % der Theorie,
IR-Spektrum (Methylenchlorid) : 1 650 $cm^{-1}$ (CO)

Beispiel 91

Isomerengemisch von 1-[7,8-Dimethoxy-1,3,4,5-tetrahydro-2H-3-benzazepin-2-on-3-yl]-3-[N-methyl-N-(2-(2- und 4-amino-phenyl)-ethyl)-amino]-propan

Hergestellt analog Beispiel 4 durch katalytische Hydrierung eines Isomerengemisches von 1-[7,8-Dimethoxy-1,3,4,5-tetrahydro-2H-3-benzazepin-2-on-3-yl]-3-[N-methyl-N-(2-(2- und 4-nitrophenyl)-ethyl)-amino]-propan.
Ausbeute : 81 % der Theorie,
IR-Spektrum (Methylenchlorid) : 1 645 $cm^{-1}$ (CO)

Beispiel 92

1-[7,8-Dimethoxy-1,3,4,5-tetrahydro-2H-3-benzazepin-2-on-3-yl]-3-[N-methyl-N-(2-(2-acetylamino-phenyl)-ethyl)-amino]-propan-hydrochlorid und
1-[7,8-Dimethoxy-1,3,4,5-tetrahydro-2H-3-benzazepin-2-on-3-yl]-3-[N-methyl-N-(2-(4-acetylamino-phenyl)-ethyl)-amino]-propan-hydrochlorid

Hergestellt analog Beispiel 52 durch Umsetzung eines Isomerengemisches von 1-[7,8-Dimethoxy-1,3,4,5-tetrahydro-2H-3-benzazepin-2-on-3-yl]-3-[N-methyl-N-(2-(2- und 4-amino-phenyl)-ethyl)-amino]-propan mit Eisessig und Acetanhydrid.
Ausbeute an 2-Acetylaminoverbindung : 26 % der Theorie, Schmelzpunkt : 102-105 °C (Zersetzung)
Ausbeute an 4-Acetylaminoverbindung : 49 % der Theorie, Schmelzpunkt : 89-93 °C (Zersetzung).

Beispiel 93

1-[7,8-Dimethoxy-1,3,4,5-tetrahydro-2H-3-benzazepin-2-on-3-yl]-3-[N-methyl-N-(2-(4-amino-phenyl)-ethyl)-amino]-propan

4,85 g (0,010 7 Mol) 1-[7,8-Dimethoxy-1,3,4,5-tetrahydro-2H-3-benzazepin-2-on-3-yl]-3-[N-methyl-N-(2-(4-acetylamino-phenyl)-ethyl)-amino]-propan werden 34 Stunden bei 40 °C mit 80 ml methanolischer Salzsäure gerührt. Nach Einengen der Lösung wird der Rückstand in Methylenchlorid gelöst, mit Natriumbicarbonat-Lösung extrahiert und mit Wasser gewaschen. Die organische Phase wird getrocknet, im Vakuum eingeengt und anschließend das erhaltene Öl im Vakuum bei 50 °C getrocknet.
Ausbeute : 88 % der Theorie,
IR-Spektrum (Methylenchlorid) : 1 645 $cm^{-1}$ (CO)
NMR-Spektrum ($CDCl_3/D_2O$) : $\delta$ = 2,25 ppm, s, 3H ($NCH_3$) ; 3,8 ppm, s, 12H ($OCH_3$) ; 6,9 ppm, d (J = 7 Hz), 2H (aromat.).

Beispiel 94

1-[7,8-Dimethoxy-1,3,4,5-tetrahydro-2H-3-benzazepin-2-on-3-yl]-3-[N-methyl-N-(2-(4-chlor-phenyl)-ethyl)-amino]-propan-dihydrochlorid

1,01 g (0,002 45 Mol) 1-[7,8-Dimethoxy-1,3,4,5-tetrahydro-2H-3-benzazepin-2-on-3-yl]-3-yl]-3-[N-methyl-N-(2-(4-aminophenyl)-ethyl)-amino]-propan werden in 5 ml halbkonzentrierter Salzsäure gelöst, mit 0,17 g (0,002 45 Mol) Natriumnitrit diazotiert. Anschließend wird bei 55 °C gerüht, bis kein Stickstoff mehr entweicht. Die Lösung wird schwach alkalisch gestellt, mit Methylenchlorid extrahiert, getrocknet und im Vakuum eingeengt. Das erhaltene Öl wird über Aluminiumoxid (200 g, neutral, Aktivität II)

(Elutionsmittel : Methylenchlorid + 2 % Ethanol) gereinigt und aus Aceton mit etherischer Salzsaüre das Dihydrochlorid gefällt.

Ausbeute : 21 % der Theorie,
Schmelzpunkt : 148-151 °C.

Beispiel 95

1-[7,8-Dimethoxy-2,3-dihydro-1H-3-benzazepin-3-yl]-3-[N-methyl-N-(2-(3,4-dimethoxy-phenyl)-ethyl)-aminol]-propan

2,3 g (0,005 Mol) 1-[7,8-Dimethoxy-1,3-dihydro-2H-3-benzazepin-2-on-3-yl]-3-[N-methyl-N-(2,-(3,4-dimethoxy-phenyl)-ethyl)-amino]-propan werden in 150 ml absolutem Ether gelöst, mit 0,6 Lithiumaluminiumhydrid versetzt und 2 Stunden bei Raumtemperatur gerührt. Unter Eiswasserkühlung wird mit 10 %iger Ammoniumchlorid-Lösung versetzt, vom Niederschlag abgesaugt und im Vakuum das Lösungsmittel entfernt. Der ölige Rückstand wird über Aluminium-oxid (neutral, Aktivität II) mit Methylenchlorid als Elutions-mittel gereinigt.

Ausbeute : 1,6 g (72,7 % der Theorie),
IR-Spektrum (Methylenchlorid) : 2 830 $cm^{-1}$ ($OCH_3$)
2 790 $cm^{-1}$ (N-Alkyl)

$C_{26}H_{36}N_2O_4$ (440,6)
Ber. : C 70,88 H 8,24 N 6,36
Gef. : C 70,50 H 8,80 N 6,22

Beispiel 96

1-[7,8-Dimethoxy-1,3,4,5-tetrahydro-2H-3-benzazepin-2-on-3-yl]-3-[N-methyl-N-(2-(4-hydroxy-phenyl)-ethyl)-amino]-propan-hydrochlorid

1,1 g (2,67 mMol) 1-[7,8-Dimethoxy-1,3,4,5-tetrahydro-2H-3-benz-azepin-2-on-3-yl]-3-[N-methyl-N-(2-(4-amino-phenyl)-ethyl)-amino]-propan werden in 10 ml halbkonzentrierter Schwefelsäure gelöst und bei 0 °C mit 0,18 g (2,67 mMol) Natriumnitrit diazotiert. Die Lösung wird dann 20 Minuten auf dem Dampfbad erhitzt, mit Wasser verdünnt, mit Natronlauge schwach alkalisch gestellt, mit Methylenchlorid extrahiert und getrocknet. Nach Einengen im Vakuum wird das erhaltene Öl über 100 g Aluminiumoxid (neutral, Aktivität II) mit Methylenchlorid + 2 % Ethanol als Elutionsmittel gereinigt. Aus Aceton/etherischer Salzsäure wird dann das Hydrochlorid gefällt.

Ausbeute : 0,17 g (15 % der Theorie),
Schmelzpunkt : 109-112 °C (Zersetzung).

Beispiel 97

1-[7,8-Dimethoxy-1,3,4,5-tetrahydro-2H-3-benzazepin-2-on-3-yl]-3-[N-methyl-N-(2-(4-dimethylamino-phenyl)-ethyl)-amino]-propan-dihydrochlorid

Hergestellt analog Beispiel 11 durch Umsetzung von 1-[7,8-Di-methoxy-1,3,4,5-tetrahydro-2H-3-benzazepin-2-on-3-yl]-3-[N-methyl-N-(2-(4-amino-phenyl)-ethyl)-amino]-propan mit 37 %iger Formalin-Lösung und Natriumcyanborhydrid.

Ausbeute : 40 % der Theorie,
Schmelzpunkt : 193-196 °C.

Beispiel 98

1-[7,8-Dimethoxy-1,3,4,5-tetrahydro-2H-1,3-benzodiazepin-2-on-3-yl]-3-[N-methyl-N-(2-(4-amino-3,5-dichlor-phenyl)-ethyl)-amino]-propan

a) N-[3-[N'-Methyl-N'-(2-(4-amino-3,5-dichlor-phenyl)-ethyl)-amino]-propyl]-2-(2-amino-4,5-dimethoxy-phenyl)-ethylamin-hydrochlorid

Hergestellt analog Beispiel 53a aus N-[3-[N'-Methyl-N'-(2-(4-amino-3,5-dichlor-phenyl)-ethyl)-amino]-propyl]-2-(2-amino-4,5-dimethoxy-phenyl)-acetamid und Lithiumaluminium-hydrid.
Schmelzpunkt : 182-188 °C (Zers.).

b) 1-[7,8-Dimethoxy-1,3,4,5-tetrahydro-2H-1,3-benzodiazepin-2-on-3-yl]-3-[N-methyl-N-(2-(4-amino-3,5-dichlor-phenyl)-ethyl)-amino]-propan

Hergestellt analog Beispiel 53b aus N-[3-[N'-Methyl-N'-(2-(4-amino-3,5-dichlor-phenyl)-ethyl)-amino]-propyl]-2-(2-(2-amino-4,5-dimethoxy-phenyl)-ethylamin-hydrochlorid und N,N'-Carbonyldiimidazol.
Schmelzpunkt : 163-166 °C.

Beispiel 99

1-[7,8-Dimethoxy-1,3,4,5-tetrahydro-2H-3-benzazepin-1,2-dion-3-yl]-3-[N-methyl-N-(2-(4-amino-3,5-dichlorphenyl)-ethyl)-amino]-propan

Hergestellt analog Beispiel 5b aus 1-[7,8-Dimethoxy-1,3,4,5-tetrahydro-2H-3-benzazepin-2-on-3-yl]-3-[N-methyl-N-(2-(4-amino-3,5-dichlor-phenyl)-ethyl)-amino]-propan und Selendioxid.
Schmelzpunkt : 118-130 °C,
m/e = 493/495 ($C_{24}H_{29}Cl_2N_3O_4$ ; 494,43)

Beispiel 100

1-[7,8-Dimethoxy-1,3,4,5-tetrahydro-2H-3-benzazepin-2-on-3-yl]-3-[N-methyl-N-(2-(3,4-dimethoxy-phenyl)-ethyl)-amino]-propan-dihydrochlorid

1,1 g (5,0 mMol) 7,8-Dimethoxy-1,3,4,5-tetrahydro-2H-3-benzazepin-2-on werden in 20 ml Dimethylsulfoxid suspendiert und unter Rühren mit 0,6 g (5,3 mMol) Kalium-tert.butylat versetzt. Nach 10 Minuten wird die Lösung mit 2,0 g (7,4 mMol) 1-Chlor-3-[N-methyl-N-(2-(3,4-dimethoxyphenyl)-ethyl)-amino]-propan versetzt. Anschließend wird noch eine Stunde bei 50 °C gerührt, auf Wasser gegossen, mit Essigsäureethylester extrahiert und die organische Phase im Vakuum eingeengt. Der Rückstand wird über Kieselgel mit Methylenchlorid + 10 % Methanol als Elutionsmittel gereinigt und aus Aceton mit etherischer Salzsäure das Dihydrochlorid gefällt.
Schmelzpunkt : 136-137 °C.

Beispiel 101

1-[7,8-Dimethoxy-1,3,4,5-tetrahydro-2H-3-benzazepin-2-on-3-yl]-3-[N-methyl-N-(2-(3,4-dimethoxy-phenyl)-ethyl)-amino]-propan-dihydrochlorid

Hergestellt analog Beispiel 100 aus 7,8-Dimethoxy-1,3,4,5-tetrahydro-2H-3-benzazepin-2-on und N-Methyl-N-(2-(3,4-dimethoxy-phenyl)-ethyl)-azetidinium-bromid.
Schmelzpunkt : 137 °C.

Beispiel 102

1-[7,8-Dimethoxy-1,3,4,5-tetrahydro-2H-3-benzazepin-2-on-3-yl]-3-[N-methyl-N-(2-(3-amino-4-chlor-phenyl)-ethyl)-amino]-propan

Hergestellt analog Beispiel 12 durch Umsetzung von N-[3-(7,8-Dimethoxy-1,3,4,5-tetrahydro-2H-3-benzazepin-2-on-3-yl)-propyl]-methylamin und 2-(3-Amino-4-chlor-phenyl)-ethylchlorid. Öl.
UV-Spektrum (Ethanol) : λ max 238 nm (0.26)
285 nm (0.13)
304 nm (0.06)
IR-Spektrum (Dichlormethan) : 3 390 und 3 480 $cm^{-1}$ ($NH_2$)
1 650 $cm^{-1}$ (CO)
2 830 $cm^{-1}$ ($OCH_3$)
2 795 $cm^{-1}$ ($CH_3$—N <)
1 520 und 1 620 $cm^{-1}$ (C=C)

Beispiel I

Tabletten zu 10 mg 1-[7,8-Dimethoxy-1,3,4,5-tetrahydro-2H-3-benzazepin-2-on-3-yl]-3-[N-methyl-N-(2-(3,4-dimethoxy-phenyl)-ethyl)-amino]-propan-hydrochlorid

Zusammensetzung :
1 Tablette enthält :

| | |
|---|---|
| Wirksubstanz | 10,0 mg |
| Maisstärke | 57,0 mg |
| Milchzucker | 48,0 mg |
| Polyvinylpyrrolidon | 4,0 mg |
| Magnesiumstearat | 1,0 mg |
| | 120,0 mg |

Herstellungsverfahren

Der Wirkstoff, Maisstärke, Milchzucker und Polyvinylpyrrolidon werden gemischt und mit Wasser befeuchtet. Die feuchte Mischung wird durch ein Sieb mit 1,5 mm-Maschenweite gedrückt und bei ca. 45 °C getrocknet. Das trockene Granulat wird durch ein Sieb mit 1,0 mm-Maschenweite geschlagen und mit Magnesiumstearat vermischt. Die fertige Mischung preßt man auf einer Tablettenpresse mit Stempeln von 7 mm Durchmesser, die mit einer Teilkerbe versehen sind, zu Tabletten.

Tablettengewicht : 120 mg

Beispiel II

Dragées zu 5 mg 1-[7,8-Dimethoxy-1,3,4,5-tetrahydro-2H-3-benzazepin-2-on-3-yl]-3-[N-methyl-N-(2-(3,4-dimethoxy-phenyl)-ethyl)-amino]-propan-hydrochlorid

1 Dragéekern enthält :

| | |
|---|---|
| Wirksubstanz | 5,0 mg |
| Maisstärke | 41,5 mg |
| Milchzucker | 30,0 mg |
| Polyvinylpyrrolidon | 3,0 mg |
| Magnesiumstearat | 0,5 mg |
| | 80,0 mg |

Herstellungsverfahren

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45 °C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht. Die fertigen Dragées werden mit Wachs poliert.

Dragéegewicht : 130 mg

Beispiel III

Ampullen zu 5 mg 1-[7,8-Dimethoxy-1,3,4,5-tetrahydro-2H-3-benzazepin-2-on-3-yl]-3-[N-methyl-N-(2-(3,4-dimethoxy-phenyl)-ethyl)-amino]-propan-hydrochlorid

1 Ampulle enthält :

| | |
|---|---|
| Wirksubstanz | 5,0 mg |
| Sorbit | 50,0 mg |
| Wasser für Injektionszwecke ad | 2,0 ml |

Herstellungsverfahren

In einem geeigneten Ansatzgefäß wird der Wirkstoff in Wasser für Injektionszwecke gelöst und die Lösung mit Sorbit isotonisch gestellt.

Nach Filtration über einen Membranfilter wird die Lösung unter $N_2$-Begasung in gereinigte und sterilisierte Ampullen abgefüllt und 20 Minuten im strömenden Wasserdampf autoklaviert.

Beispiel IV

Suppositorien zu 15 mg 1-[7,8-Dimethoxy-1,3,4,5-tetrahydro-2H-3-benzazepin-2-on-3-yl]-3-[N-methyl-N-(2-(3,4-dimethoxy-phenyl)-ethyl)-amino]-propan-hydrochlorid

1 Zäpfchen enthält :

| | |
|---|---|
| Wirksubstanz | 0,015 g |
| Hartfett (z. B. Witepsol H 19 und W 45) | 1,685 g |
| | 1,700 g |

Herstellungsverfahren

Das Hartfett wird geschmolzen. Bei 38 °C wird die gemahlene Wirksubstanz in der Schmelze homogen dispergiert. Es wird auf 35 °C abgekühlt und in schwach vorgekühlte Suppositorienformen ausgegossen.

Beispiel V

Tropfenlösung mit 10 mg 1-[7,8-Dimethoxy-1,3,4,5-tetrahydro-2H-3-benzazepin-2-on-3-yl]-3-[N-methyl-N-(2-(3,4-dimethoxy-phenyl)-ethyl)-amino]-propan-hydrochlorid pro 5 ml

100 ml Lösung enthalten :

| | |
|---|---|
| Wirksubstanz | 0,2 g |
| Hydroxyäthylcellulose | 0,15 g |
| Weinsäure | 0,1 g |
| Sorbitlösung 70 % Trockensubstanz | 30,0 g |
| Glycerin | 10,0 g |
| Benzosäure | 0,15 g |
| Dest. Wasser ad | 100 ml |

Herstellungsverfahren

Dest. Wasser wird auf 70 °C erhitzt. Hierin wird unter Rühren Hydroxyäthylcellulose, Benzoesäure und Weinsäure gelöst. Es wird auf Raumtemperatur abgekühlt und hierbei das Glycerin und die Sorbitlösung unter Rühren zugegeben. Bei Raumtemperatur wird der Wirkstoff zugegeben und bis zur völligen Auflösung gerührt. Anschließend wird zur Entlüftung des Saftes unter Rühren evakuiert.

**Patentansprüche** (für die Vertragsstaaten : BE, CH, DE, FR, IT, LI, LU, NL, SE)

1. Benzazepinderivate der allgemeinen Formel

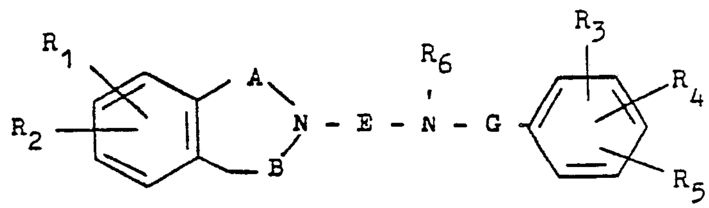

(I)

in der

A eine —$CH_2$—$CH_2$-, —CH=CH-, —NH—CO-, —$CH_2$—CO- oder
5 5

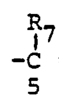
=N-Gruppe

in der $R_7$ eine Alkylgruppe mit 1 bis 3 Kohlenstoffatomen darstellt, und B die Methylen- oder Carbonylgruppe oder

A die —CO—CO-Gruppe und B die Methylengruppe,

E eine gegebenenfalls durch eine Alkylgruppe mit 1 bis 3 Kohlenstoffatomen substituierte Ethylen-, n-Propylen- oder n-Butylengruppe, eine durch eine Hydroxygruppe in 2-Stellung substituierte n-Propylengruppe oder eine durch eine Hydroxygruppe in 2- oder 3-Stellung substituierte n-Butylengruppe,

G eine gegebenenfalls durch eine Alkylgruppe mit 1 bis 3 Kohlenstoffatomen substituierte Methylen- oder Ethylengruppe,

$R_1$ und $R_2$, die gleich oder verschieden sein können, Hydroxygruppen, Alkyl-, Alkoxy- oder Phenylalkoxygruppen, in denen der Alkylteil jeweils 1 bis 3 Kohlenstoffatome enthalten kann, oder einer der Reste $R_1$ oder $R_2$ auch ein Wasserstoffatom oder $R_1$ zusammen mit $R_2$ eine Alkylendioxygruppe mit 1 oder 2 Kohlenstoffatomen,

$R_3$ und $R_4$, die gleich oder verschieden sein können, Wasserstoff- oder Halogenatome, Hydroxygruppen, Alkyl- oder Alkoxygruppen mit jeweils 1 bis 4 Kohlenstoffatomen, Trifluormethyl- oder Cyangruppen oder einer der Reste $R_3$ oder $R_4$ auch eine Nitrogruppe oder $R_3$ zusammen mit $R_4$ eine Alkylendioxygruppe mit 1 oder 2 Kohlenstoffatomen,

$R_5$ ein Wasserstoffatom, eine Alkyl-, Hydroxy-, Alkoxy-, Amino-, Alkylamino-, Dialkylamino-, Alkanoylamino-, Alkoxycarbonylamino- oder Bis(alkoxycarbonyl)aminogruppe, in denen der Alkylteil jeweils 1 bis 3 Kohlenstoffatome enthalten kann, oder eine durch eine Trifluormethylgruppe substituierte Methylamino- oder Ethylaminogruppe, und

$R_6$ ein Wasserstoffatom, eine Alkyl-, Phenylalkyl-, Alkanoyl- oder Alkoxycarbonylgruppe, in denen

der Alkylteil jeweils 1 bis 3 Kohlenstoffatome enthalten kann, oder eine Alkenylgruppe mit 3 bis 5 Kohlenstoffatomen bedeuten, und deren Säureadditionssalze, insbesondere deren physiologisch verträgliche Säureadditionssalze mit anorganischen oder organischen Säuren.

2. Benzazepinderivate der allgemeinen Formel I gemäß Anspruch 1, in der

A eine $—CH_2—CH_2$-, —CH=CH-, —NH—CO-, $—CH_2—CO$- oder

5 5

$CH_3$
|
-C = N-Gruppe
5

und B die Methylen- oder Carbonylgruppe oder

A die —CO—CO-Gruppe und B die Methylengruppe,

E die Ethylen-, n-Propylen-, n-Butylen-, 2-Methyl-n-propylen- oder 2-Hydroxy-n-propylengruppe,

G die Methylen-, Ethylen- oder 1-Methyl-ethylengruppe,

$R_1$ eine Methyl-, Hydroxy-, Benzyloxy- oder Alkoxygruppe mit 1 bis 3 Kohlenstoffatomen,

$R_2$ ein Wasserstoffatom, eine Methyl-, Hydroxy- oder Methoxygruppe oder $R_1$ und $R_2$ zusammen die Methylendioxygruppe,

$R_3$ ein Wasserstoff-, Fluor-, Chlor- oder Bromatom, eine Trifluormethyl-, Nitro-, Alkyl- oder Alkoxygruppe mit jeweils 1 bis 4 Kohlenstoffatomen,

$R_4$ ein Wasserstoff-, Chlor- oder Bromatom, eine Methyl-, Methoxy- oder Cyangruppe oder $R_3$ und $R_4$ zusammen die Methylendioxygruppe,

$R_5$ ein Wasserstoffatom, eine Hydroxy-, Methoxy-, Amino-, Acetylamino-, Ethoxycarbonylamino-, Bis(ethoxycarbonyl)amino-, Dimethylamino- oder β,β,β-Trifluorethylaminogruppe und

$R_6$ ein Wasserstoffatom, eine Alkylgruppe mit 1 bis 3 Kohlenstoffatomen, eine Allyl-, Benzyl-, Acetyl- oder Ethoxycarbonylgruppe bedeuten, und deren Säureadditionssalze, insbesondere deren physiologisch verträgliche Säureadditionssalze mit anorganischen oder organischen Säuren.

3. Benzazepinderivate der allgemeinen Formel I gemäß Anspruch 1, in der

A die $—CH_2—CH_2$-, —CH=CH- oder

$CH_3$
|
-C = N-Gruppe
5

und B die Carbonylgruppe oder

A die —CH=CH-, —NH—CO- oder —CO—CO-Gruppe und B die Methylengruppe,

5

E die n-Propylengruppe,

G die Ethylengruppe,

$R_1$ die Methoxy- oder die Hydroxygruppe,

$R_2$ die Methoxygruppe oder $R_1$ und $R_2$ zusammen die Methylendioxygruppe,

$R_3$ die Methoxy- oder Trifluormethylgruppe, ein Chlor- oder Bromatom,

$R_4$ die Methoxygruppe, ein Wasserstoff-, Chlor- oder Bromatom oder $R_3$ und $R_4$ zusammen die Methylendioxygruppe,

$R_5$ ein Wasserstoffatom, eine Amino- oder Methoxygruppe und

$R_6$ ein Wasserstoffatom, die Methyl-, Ethyl-, n-Propyl- oder Allylgruppe bedeuten, und deren physiologisch verträgliche Säureadditionssalze mit anorganischen oder organischen Säuren.

4. Benzazepinderivate der allgemeinen Formel

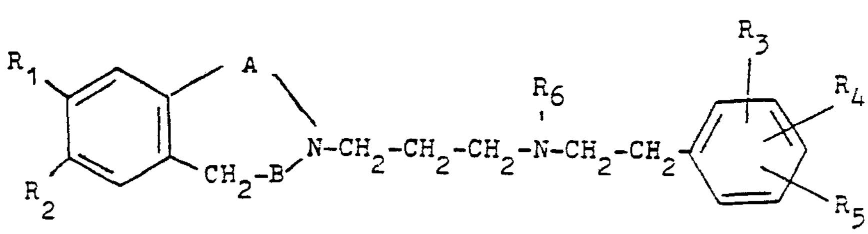

(Ia)

in der

A die $—CH_2—CH_2$-, —CH=CH- oder

$CH_3$
|
-C = N-Gruppe
5

und B die Carbonylgruppe oder

A die —NH—CO- oder —CO-CO-Gruppe und B die Methylengruppe,

5

$R_1$ und $R_2$ je eine Methoxygruppe,

$R_6$ ein Wasserstoffatom, die Methyl- oder Allylgruppe,

$R_3$ ein Wasserstoffatom oder eine Methoxygruppe in 3-Stellung,

$R_4$ eine Methoxygruppe in 4-Stellung oder $R_3$ und $R_4$ zusammen die 3,4-Methylendioxygruppe und $R_5$ ein Wasserstoffatom oder

$R_3$ in 3-Stellung eine Trifluormethylgruppe, ein Chlor- oder Bromatom,

$R_4$ in 5-Stellung ein Chlor- oder Bromatom und

$R_5$ in 4-Stellung die Aminogruppe bedeuten, und deren physiologisch verträgliche Säureadditionssalze mit anorganischen oder organischen Säuren.

5. 1-[7,8-Dimethoxy-1,3,4,5-tetrahydro-2H-3-benzazepin-2-on-3-yl]-3-[N-methyl-N-(2-(3,4-dimethoxy-phenyl)-ethyl)-amino]-propan und dessen Säureadditionssalze.

6. 1-[7,8-Dimethoxy-1,3,4,5-tetrahydro-2H-3-benzazepin-1,2-dion-3-yl]-3-[N-methyl-N-(2-(3,4-dimethoxy-phenyl)-ethyl)-amino]-propan und dessen Säureadditionssalze.

7. 1-[7,8-Dimethoxy-1,3,4,5-tetrahydro-2H-3-benzazepin-2-on-3-yl]-3-[N-methyl-N-(2-(4-amino-3,5-dichlor-phenyl)-ethyl)-amino]-propan und dessen Säureadditionssalze.

8. 1-[7,8-Dimethoxy-1,3,4,5-tetrahydro-2H-3-benzazepin-2-on-3-yl]-3-[N-methyl-N-(2-(4-methoxy-phenyl)-ethyl)-amino]-propan und dessen Säureadditionssalze.

9. Arzneimittel zur Bekämpfung von Sinustachykardien und ischämischer Herzerkrankungen, enthaltend eine Verbindung gemäß den Ansprüchen 1 bis 8 neben einem oder mehreren inerten Trägerstoffen und/oder Verdünnungsmitteln.

10. Verfahren zur Herstellung von Benzazepinderivaten der allgemeinen Formel

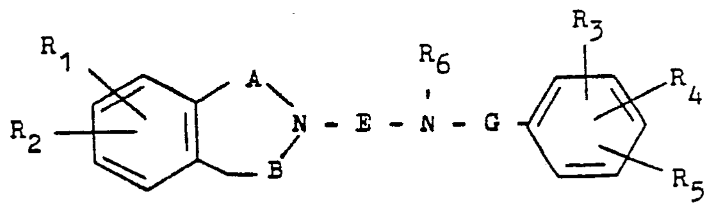

(I)

in der

A eine $—CH_2—CH_2-$, $—CH=CH-$, $—NH—CO-$, $—CH_2—CO-$ oder

5 5

$-\overset{R_7}{\overset{|}{C}}=N$-Gruppe

5

in der $R_7$ eine Alkylgruppe mit 1 bis 3 Kohlenstoffatomen darstellt, und B die Methylen- oder Carbonylgruppe oder

A die —CO—CO-Gruppe und B die Methylengruppe,

E eine gegebenenfalls durch eine Alkylgruppe mit 1 bis 3 Kohlenstoffatomen substituierte Ethylen-, n-Propylen- oder n-Butylengruppe, eine durch eine Hydroxygruppe in 2-Stellung substituierte n-Propylengruppe oder eine durch eine Hydroxygruppe in 2- oder 3-Stellung substituierte n-Butylengruppe,

G eine gegebenenfalls durch eine Alkylgruppe mit 1 bis 3 Kohlenstoffatomen substituierte Methylen- oder Ethylengruppe,

$R_1$ und $R_2$, die gleich oder verschieden sein können, Hydroxygruppen, Alkyl-, Alkoxy- oder Phenylalkoxygruppen, in denen der Alkylteil jeweils 1 bis 3 Kohlenstoffatome enthalten kann, oder einer der Reste $R_1$ oder $R_2$ auch ein Wasserstoffatom oder $R_1$ zusammen mit $R_2$ eine Alkylendioxygruppe mit 1 oder 2 Kohlenstoffatomen,

$R_3$ und $R_4$, die gleich oder verschieden sein können, Wasserstoff- oder Halogenatome, Hydroxygruppen, Alkyl- oder Alkoxygruppen mit jeweils 1 bis 4 Kohlenstoffatomen, Trifluormethyl- oder Cyangruppen oder einer der Reste $R_3$ oder $R_4$ auch eine Nitrogruppe oder $R_3$ zusammen mit $R_4$ eine Alkylendioxygruppe mit 1 oder 2 Kohlenstoffatomen,

$R_5$ ein Wasserstoffatom, eine Alkyl-, Hydroxy-, Alkoxy-, Amino-, Alkylamino-, Dialkylamino-, Alkanoylamino-, Alkoxycarbonylamino- oder Bis(alkoxycarbonyl)aminogruppe, in denen der Alkylteil jeweils 1 bis 3 Kohlenstoffatome enthalten kann, oder eine durch eine Trifluormethylgruppe substituierte Methylamino- oder Ethylaminogruppe, und

$R_6$ ein Wasserstoffatom, eine Alkyl-, Phenylalkyl-, Alkanoyl- oder Alkoxycarbonylgruppe, in denen

der Alkylteil jeweils 1 bis 3 Kohlenstoffatome enthalten kann, oder eine Alkenylgruppe mit 3 bis 5 Kohlenstoffatomen bedeuten, sowie von deren Säureadditionssalzen, insbesondere von deren physiologisch verträglichen Säureadditionssalzen mit anorganischen oder organischen Säuren dadurch gekennzeichnet, daß

a) eine Verbindung der allgemeinen Formel

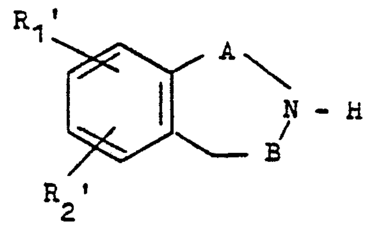

(II)

in der

A und B wie eingangs definiert sind,

$R_1'$ und $R_2'$ je eine durch einen Schutzrest geschützte Hydroxygruppe darstellen oder die für $R_1$ und $R_2$ eingangs erwähnten Bedeutungen besitzen, mit einer Verbindung der allgemeinen Formel

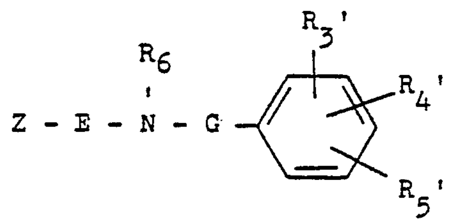

(III)

in der

$R_6$, E und G wie eingangs definiert sind,

$R_3'$ bis $R_5'$ je eine durch einen Schutzrest geschützte Hydroxygruppe darstellen oder die für $R_3$ bis $R_5$ eingangs erwähnten Bedeutungen besitzen und

Z eine nukleophile Austrittsgruppe bedeutet, oder deren gegebenenfalls im Reaktionsgemisch vorliegenden inneren quartären Salzes der allgemeinen Formel

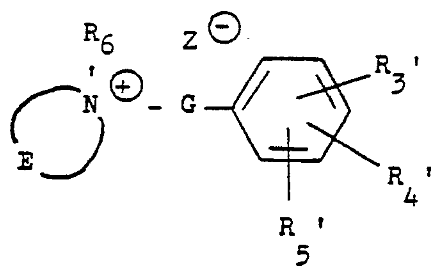

(IIIa)

in der E, G, Z, $R_3'$ bis $R_5'$ und $R_6$ wie eingangs definiert sind, umgesetzt wird und gegebenenfalls anschließende Abspaltung eines verwendeten Schutzrestes oder

b) zur Herstellung von Verbindungen der allgemeinen Formel I, in der $R_6$ ein Wasserstoffatom, eine Alkenylgruppe mit 3 bis 5 Kohlenstoffatomen, eine Alkyl- oder Phenylalkylgruppe mit jeweils 1 bis 3 Kohlenstoffatomen im Alkylteil darstellt, eine Verbindung der allgemeinen Formel

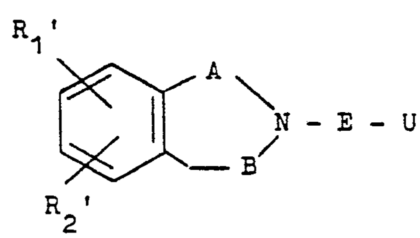

(IV)

mit einer Verbindung der allgemeinen Formel

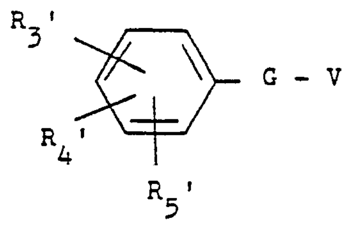

(V)

in denen

A, B, E und G wie eingangs definiert sind,

$R_1'$ bis $R_5'$ je eine durch einen Schutzrest geschützte Hydroxygruppe darstellen oder die für $R_1$ bis $R_5$ eingangs erwähnten Bedeutungen besitzen, einer der Reste U oder V die $R_6'$-NH-Gruppe darstellt, wobei $R_6'$ ein Wasserstoffatom, eine Alkenylgruppe mit 3 bis 5 Kohlenstoffatomen, eine Alkyl- oder Phenylalkylgruppe mit jeweils 1 bis 3 Kohlenstoffatomen im Alkylteil bedeutet, der andere der Reste U oder V eine nukleophile Austrittsgruppe oder der Rest U zusammen mit einem β-ständigen Wasserstoffatom des Restes E ein Sauerstoffatom und V eine $R_6'$-NH-Gruppe darstellen, wobei $R_6'$ wie vorstehend erwähnt definiert ist, umgesetzt wird und gegebenenfalls anschließende Abspaltung eines verwendeten Schutzrestes oder

c) zur Herstellung von Verbindungen der allgemeinen Formel I, in der $R_6$ ein Wasserstoffatom, eine Alkenylgruppe mit 3 bis 5 Kohlenstoffatomen, eine Alkyl- oder Phenylalkylgruppe mit jeweils 1 bis 3 Kohlenstoffatomen im Alkylteil darstellt, eine Verbindung der allgemeinen Formel

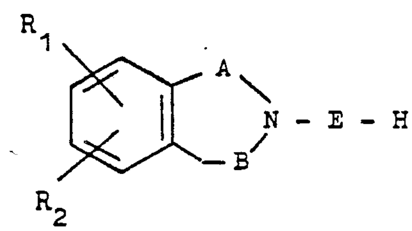

(VI)

in der A, B, E, $R_1$ und $R_2$ wie eingangs definiert sind, wobei jedoch im Rest E zwei Wasserstoffatome in einer $-CH_2-$ oder $CH_3$-Gruppe des Restes E durch ein Sauerstoffatom ersetzt sind, mit einem Amin der allgemeinen Formel

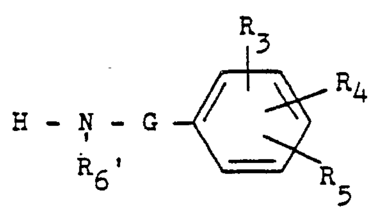

(VII)

in der G und $R_3$ bis $R_5$ wie eingangs definiert sind und $R_6'$ ein Wasserstoffatom, eine Alkenylgruppe mit 3 bis 5 Kohlenstoffatomen, eine Alkyl- oder Phenylalkylgruppe mit jeweils 1 bis 3 Kohlenstoffatomen im Alkylteil darstellt, in Gegenwart eines Reduktionsmittels umgesetzt wird oder

d) zur Herstellung von Verbindungen der allgemeinen Formel I, in der $R_6$ ein Wasserstoffatom, eine Alkenylgruppe mit 3 bis 5 Kohlenstoffatomen, eine Alkyl- oder Phenylalkylgruppe mit jeweils 1 bis 3 Kohlenstoffatomen im Alkylteil darstellt, eine Verbindung der allgemeinen Formel

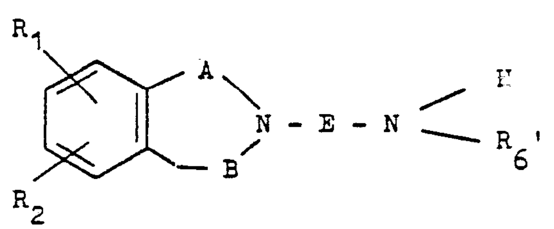

(VIII)

in der A, B, E, $R_1$ und $R_2$ wie eingangs definiert sind und $R_6'$ ein Wasserstoffatom, eine Alkenylgruppe mit 3 bis 5 Kohlenstoffatomen, eine Alkyl- oder Phenylalkylgruppe mit jeweils 1 bis 3 Kohlenstoffatomen im Alkylteil darstellt, mit einer Verbindung der allgemeinen Formel

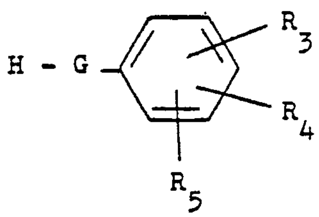

(IX)

in der G und $R_3$ bis $R_5$ wie eingangs definiert sind, wobei jedoch im Rest G zwei Wasserstoffatome in einer —$CH_2$— oder $CH_3$-Gruppe des Restes G durch ein Sauerstoffatom ersetzt sind, in Gegenwart eines Reduktionsmittels umgesetzt wird oder

e) zur Herstellung von Verbindungen der allgemeinen Formel I, in der A die —NH—CO-Gruppe, E eine gegebenenfalls durch eine Alkylgruppe mit 1 bis 3 Kohlenstoffatomen substituierte Ethylen-, n-Propylen- oder n-Butylengruppe und $R_6$ kein Wasserstoffatom darstellen, eine Verbindung der allgemeinen Formel

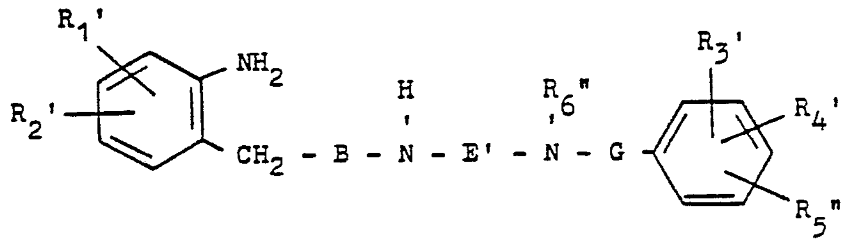

(X)

in der

B und G wie eingangs definiert sind,

E' eine gegebenenfalls durch eine Alkylgruppe mit 1 bis 3 Kohlenstoffatomen substituierte Ethylen-, n-Propylen- oder n-Butylengruppe,

$R_1'$ bis $R_4'$ je eine durch einen Schutzrest geschützte Hydroxygruppe darstellen oder die für $R_1$ bis $R_4$ eingangs erwähnten Bedeutungen besitzen,

$R_5''$ eine durch einen Schutzrest geschützte Hydroxy-, Amino- oder Alkylaminogruppe darstellt oder mit Ausnahme der Amino- oder Alkylaminogruppe die für $R_5$ eingangs erwähnten Bedeutungen besitzt und

$R_6''$ mit Ausnahme von Wasserstoff die für $R_6$ eingangs erwähnten Bedeutungen besitzt oder eine Schutzgruppe für eine Aminogruppe darstellt, mit einem Kohlensäurederivat der allgemeinen Formel

$$W{-}CO{-}W \qquad (XI)$$

in der W, die gleich oder verschieden sein können, jeweils eine nukleophile Austrittsgruppe wie ein Chlor- oder Bromatom, eine gegebenenfalls durch Halogenatome substituierte Alkoxygruppe mit 1 bis 3 Kohlenstoffatomen oder eine Imidazolyl-(2)-gruppe bedeuten, umgesetzt wird und gegebenenfalls anschließende Abspaltung eines verwendeten Schutzrestes oder

f) zur Herstellung von Verbindungen der allgemeinen Formel I, in der A die —$CH_2$—$CH_2$-Gruppe, $R_1$ und $R_2$ keine Benzyloxygruppe, $R_3$ oder $R_4$ keine Nitrogruppe und $R_6$ keine Benzylgruppe oder keine Alkenylgruppe mit 3 bis 5 Kohlenstoffatomen darstellen, eine Verbindung der allgemeinen Formel

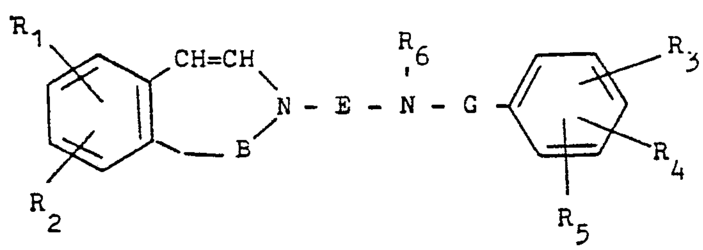

(XII)

in der $R_1$ bis $R_5$, B, E und G wie eingangs definiert sind, wobei $R_3$ oder $R_4$ keine Nitrogruppe darstellen können, falls $R_5$ eine Aminogruppe bedeutet, hydriert wird oder

g) eine Verbindung der allgemeinen Formel

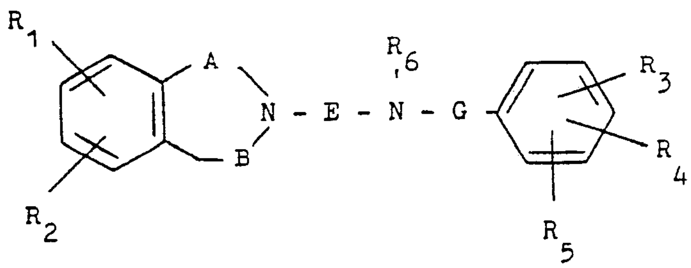

(XIII)

in der $R_1$ bis $R_6$, A, B, E und G wie eingangs definiert sind, wobei jedoch mindestens einer der Reste $R_1$ bis $R_4$ eine Hydroxygruppe oder $R_5$ eine Hydroxygruppe, eine Amino- oder Alkylaminogruppe mit 1 bis 3 Kohlenstoffatomen oder $R_6$ ein Wasserstoffatom darstellen muß, mit einer Verbindung der allgemeinen Formel

$$R_8—X \quad \text{(XIV)}$$

in der $R_8$ eine Alkylgruppe mit 1 bis 3 Kohlenstoffatomen oder auch eine Alkenylgruppe mit 3 bis 5 Kohlenstoffatomen, wenn $R_6$ ein Wasserstoffatom darstellt, oder auch eine durch eine Phenylgruppe substituierte Alkylgruppe mit 1 bis 3 Kohlenstoffatomen, wenn $R_1$ oder $R_2$ die Hydroxygruppe und/oder $R_6$ ein Wasserstoffatom darstellen, und X eine nukleophile Austrittsgruppe wie ein Halogenatom oder eine Sulfonyloxygruppe oder, falls mindestens einer der Reste $R_1$ bis $R_5$ eine Hydroxygruppe darstellt, X zusammen mit einem α-ständigen Wasserstoffatom des Restes $R_8$ eine Diazogruppe oder auch, falls $R_6$ ein Wasserstoffatom oder $R_5$ eine Amino- oder Alkylaminogruppe darstellen, ein Sauerstoffatom bedeuten, umgesetzt wird oder

h) zur Herstellung von Verbindungen der allgemeinen Formel I, in der A keine —CH=CH-Gruppe, $R_3$ ein Chlor- oder Bromatom und $R_5$ eine Amino- oder Alkylaminogruppe darstellen, eine Verbindung der allgemeinen Formel

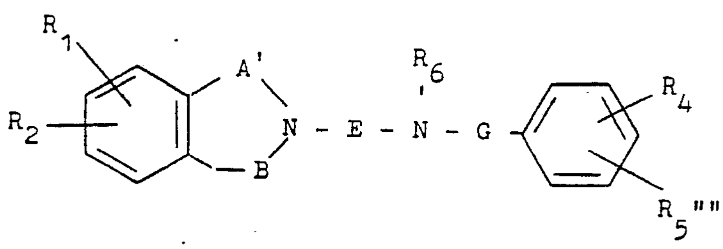

(XV)

in der

$R_1$, $R_2$, $R_4$, $R_6$, B, E und G wie eingangs definiert sind,

A' mit Ausnahme der —CH=CH-Gruppe die für A eingangs erwähnten Bedeutungen besitzt und

$R_5''''$ eine Amino- oder Alkylaminogruppe mit 1 bis 3 Kohlenstoffatomen darstellt, halogeniert wird oder

i) zur Herstellung von Verbindungen der allgemeinen Formel I, in der A die —CO—CO-Gruppe, B die Methylengruppe und E eine gegebenenfalls durch eine Alkylgruppe mit 1 bis 3 Kohlenstoffatomen substituierte Ethylen-, n-Propylen- oder n-Butylengruppe darstellen, eine Verbindung der allgemeinen Formel

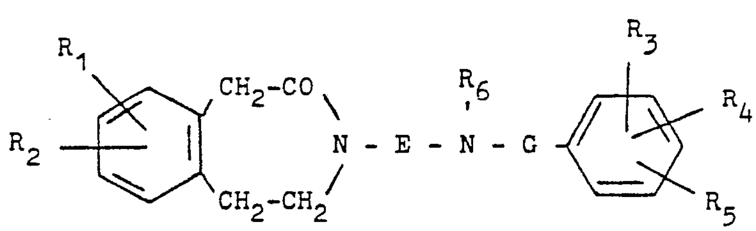

(XVI)

in der $R_1$ bis $R_6$, E und G wie eingangs definiert sind, oxidiert wird und gewünschtenfalls anschließend eine erhaltene Verbindung der allgemeinen Formel I, in der B die Carbonylgruppe darstellt, mittels Reduktion in eine entsprechende Verbindung der allgemeinen Formel I, in der B die Methylengruppe darstellt, übergeführt wird und/oder

eine erhaltene Verbindung der allgemeinen Formel I, in der

A keine $-CH=CH-$ oder

$-\overset{R_7}{\overset{|}{C}}=N$-Gruppe

und $R_6$ eine Benzyl- oder 1-Phenylethylgruppe darstellen, mittels katalytischer Hydrierung in eine entsprechende Verbindung der allgemeinen Formel I, in der $R_6$ ein Wasserstoffatom darstellt, übergeführt wird und/oder

eine erhaltene Verbindung der allgemeinen Formel I, in der $R_3$ oder $R_4$ eine Nitrogruppe darstellt, mittels Reduktion in eine entsprechende Verbindung der allgemeinen Formel I, in der $R_5$ eine Aminogruppe darstellt, übergeführt wird und/oder

eine erhaltene Verbindung der allgemeinen Formel I, in der $R_6$ ein Wasserstoffatom und/oder $R_5$ eine Aminogruppe darstellt, mittels Acylierung in eine entsprechende Verbindung der allgemeinen Formel I, in der $R_6$ eine Alkanoyl- oder Alkoxycarbonylgruppe und/oder $R_5$ eine Alkanoyl-amino-, Alkoxycarbonylamino- oder Bis(alkoxycarbonyl-aminogruppe) darstellen, wobei der Alkylteil jeweils 1 bis 3 Kohlenstoffatome enthalten kann, übergeführt wird und/oder

eine erhaltene Verbindung der allgemeinen Formel I, in der $R_5$ eine Aminogruppe, $R_6$ kein Wasserstoffatom, $R_3$ und $R_4$ jeweils keine Cyangruppe darstellen, über eine entsprechendes Diazoniumsalz in eine entsprechende Verbindung der allgemeinen Formel I, in der $R_5$ ein Wasserstoffatom, eine Hydroxy- oder Alkoxygruppe oder $R_5$ ein Wasserstoffatom und $R_3$ ein Halogenatom oder die Cyangruppe bedeuten, übergeführt wird und/oder

eine erhaltene Verbindung der allgemeinen Formel I in ihre Säureadditionssalze, insbesondere in ihre physiologisch verträglichen Salze mit anorganischen oder organischen Säuren übergeführt wird.

11. Verfahren zur Herstellung eines Arzneimittels, dadurch gekennzeichnet, daß eine Verbindung der allgemeinen Formel I oder ein physiologisch verträgliches Säureadditionssalz hiervon auf nichtchemischem Wege in einen oder mehrere inerte Trägerstoffe und/oder Verdünnungsmitteln eingearbeitet wird.

**Patentansprüche** (für den Vertragsstaat AT)

1. Verfahren zur Herstellung von Benzazepinderivaten der allgemeinen Formel

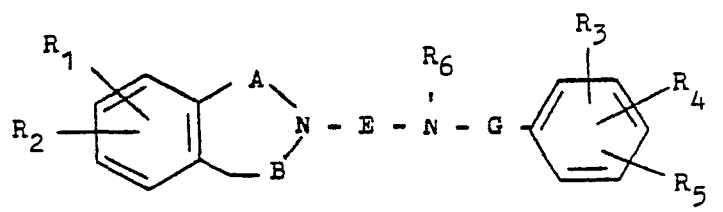

(I)

in der

A eine $-CH_2-CH_2-$, $-CH=CH-$, $-NH-CO-$, $-CH_2-CO-$ oder
5 5

$-\overset{R_7}{\overset{|}{C}}=N$-Gruppe
5

in der $R_7$ eine Alkylgruppe mit 1 bis 3 Kohlenstoffatomen darstellt, und B die Methylen- oder Carbonylgruppe oder

A die $-CO-CO-$Gruppe und B die Methylengruppe,

E eine gegebenenfalls durch eine Alkylgruppe mit 1 bis 3 Kohlenstoffatomen substituierte Ethylen-, n-Propylen- oder n-Butylengruppe, eine durch eine Hydroxygruppe in 2-Stellung substituierte n-Propylengruppe oder eine durch eine Hydroxygruppe in 2- oder 3-Stellung substituierte n-Butylengruppe,

G eine gegebenenfalls durch eine Alkylgruppe mit 1 bis 3 Kohlenstoffatomen substituierte Methylen- oder Ethylengruppe,

$R_1$ und $R_2$, die gleich oder verschieden sein können, Hydroxygruppen, Alkyl-, Alkoxy- oder Phenylalkoxygruppen, in denen der Alkylteil jeweils 1 bis 3 Kohlenstoffatome enthalten kann, oder einer der Reste $R_1$ oder $R_2$ auch ein Wasserstoffatom oder $R_1$ zusammen mit $R_2$ eine Alkylendioxygruppe mit 1 oder 2 Kohlenstoffatomen,

$R_3$ und $R_4$, die gleich oder verschieden sein können, Wasserstoff- oder Halogenatome, Hydroxygruppen, Alkyl- oder Alkoxygruppen mit jeweils 1 bis 4 Kohlenstoffatomen, Trifluormethyl- oder Cyangruppen oder einer der Reste $R_3$ oder $R_4$ auch eine Nitrogruppe oder $R_3$ zusammen mit $R_4$ eine Alkylendioxygruppe mit 1 oder 2 Kohlenstoffatomen,

$R_5$ ein Wasserstoffatom, eine Alkyl-, Hydroxy-, Alkoxy-, Amino-, Alkylamino-, Dialkylamino-, Alkanoylamino-, Alkoxycarbonylamino- oder Bis(alkoxycarbonyl)aminogruppe, in denen der Alkylteil jeweils 1 bis 3 Kohlenstoffatome enthalten kann, oder eine durch eine Trifluormethylgruppe substituierte Methylamino- oder Ethylaminogruppe, und

$R_6$ ein Wasserstoffatom, eine Alkyl-, Phenylalkyl-, Alkanoyl- oder Alkoxycarbonylgruppe, in denen der Alkylteil jeweils 1 bis 3 Kohlenstoffatome enthalten kann, oder eine Alkenylgruppe mit 3 bis 5 Kohlenstoffatomen bedeuten, sowie von deren Säureadditionssalzen, insbesondere von deren physiologisch verträglichen Säureadditionssalzen mit anorganischen oder organischen Säuren dadurch gekennzeichnet, daß

a) eine Verbindung der allgemeinen Formel

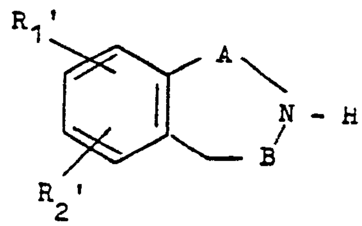

(II)

in der

A und B wie eingangs definiert sind,

$R_1'$ und $R_2'$ je eine durch einen Schutzrest geschützte Hydroxygruppe darstellen oder die für $R_1$ und $R_2$ eingangs erwähnten Bedeutungen besitzen, mit einer Verbindung der allgemeinen Formel

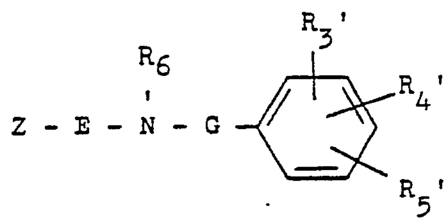

(III)

in der

$R_6$, E und G wie eingangs definiert sind,

$R_3'$ bis $R_5'$ je eine durch einen Schutzrest geschützte Hydroxygruppe darstellen oder die für $R_3$ bis $R_5$ eingangs erwähnten Bedeutungen besitzen und

Z eine nukleophile Austrittsgruppe bedeutet, oder deren gegebenenfalls im Reaktionsgemisch vorliegenden inneren quartären Salzes der allgemeinen Formel

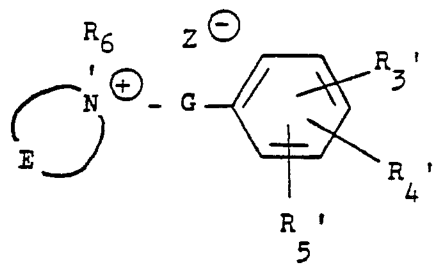

(IIIa)

in der E, G, Z, $R_3'$ bis $R_5'$ und $R_6$ wie eingangs definiert sind, umgesetzt wird und gegebenenfalls anschließende Abspaltung eines verwendeten Schutzrestes oder

b) zur Herstellung von Verbindungen der allgemeinen Formel I, in der $R_6$ ein Wasserstoffatom, eine Alkenylgruppe mit 3 bis 5 Kohlenstoffatomen, eine Alkyl- oder Phenylalkylgruppe mit jeweils 1 bis 3 Kohlenstoffatomen im Alkylteil darstellt, eine Verbindung der allgemeinen Formel

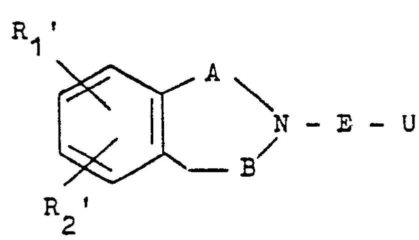

(IV)

mit einer Verbindung der allgemeinen Formel

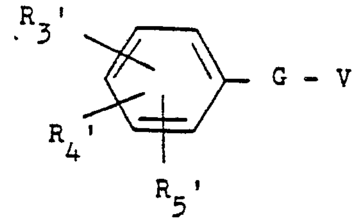

(V)

in denen

A, B, E und G wie eingangs definiert sind,

$R_1'$ bis $R_5'$ je eine durch einen Schutzrest geschützte Hydroxygruppe darstellen oder die für $R_1$ bis $R_5$ eingangs erwähnten Bedeutungen besitzen,

einer der Reste U oder V die $R_6'$ —NH-Gruppe darstellt, wobei $R_6'$ ein Wasserstoffatom, eine Alkenylgruppe mit 3 bis 5 Kohlenstoffatomen, eine Alkyl- oder Phenylalkylgruppe mit jeweils 1 bis 3 Kohlenstoffatomen im Alkylteil bedeutet,

der andere der Reste U oder V eine nukleophile Austrittsgruppe oder

der Rest U zusammen mit einem β-ständigen Wasserstoffatom des Restes E ein Sauerstoffatom und V eine $R_6'$ —NH-Gruppe darstellen, wobei $R_6'$ wie vorstehend erwähnt definiert ist, umgesetzt wird und gegebenenfalls anschließende Abspaltung eines verwendeten Schutzrestes oder

c) zur Herstellung von Verbindungen der allgemeinen Formel I, in der $R_6$ ein Wasserstoffatom, eine Alkenylgruppe mit 3 bis 5 Kohlenstoffatomen, eine Alkyl- oder Phenylalkylgruppe mit jeweils 1 bis 3 Kohlenstoffatomen im Alkylteil darstellt, eine Verbindung der allgemeinen Formel

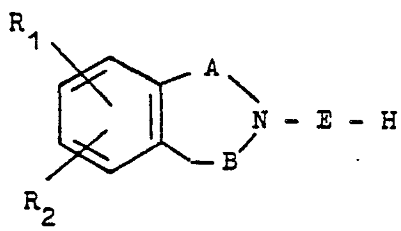

(VI)

in der A, B, E, $R_1$ und $R_2$ wie eingangs definiert sind, wobei jedoch im Rest E zwei Wasserstoffatome in einer $—CH_2$- oder $CH_3$-Gruppe des Restes E durch ein Sauerstoffatom ersetzt sind, mit einem Amin der allgemeinen Formel

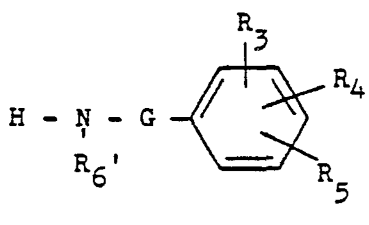

(VII)

in der

G und $R_3$ bis $R_5$ wie eingangs definiert sind und $R_6'$ ein Wasserstoffatom, eine Alkenylgruppe mit 3 bis 5 Kohlenstoffatomen, eine Alkyl- oder Phenylalkylgruppe mit jeweils 1 bis 3 Kohlenstoffatomen im Alkylteil darstellt, in Gegenwart eines Reduktionsmittels umgesetzt wird oder

d) zur Herstellung von Verbindungen der allgemeinen Formel I, in der $R_6$ ein Wasserstoffatom, eine Alkenylgruppe mit 3 bis 5 Kohlenstoffatomen, eine Alkyl- oder Phenylalkylgruppe mit jeweils 1 bis 3 Kohlenstoffatomen im Alkylteil darstellt, eine Verbindung der allgemeinen Formel

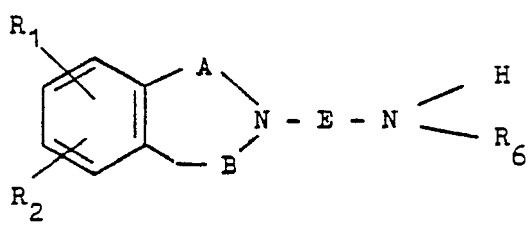

(VIII)

in der

A, B, E, $R_1$ und $R_2$ wie eingangs definiert sind und $R_6'$ ein Wasserstoffatom, eine Alkenylgruppe mit 3

bis 5 Kohlenstoffatomen, eine Alkyl- oder Phenylalkylgruppe mit jeweils 1 bis 3 Kohlenstoffatomen im Alkylteil darstellt, mit einer Verbindung der allgemeinen Formel

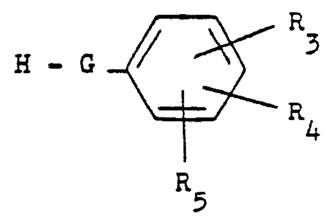

(IX)

in der G und $R_3$ bis $R_5$ wie eingangs definiert sind, wobei jedoch im Rest G zwei Wasserstoffatome in einer —$CH_2$- oder $CH_3$-Gruppe des Restes G durch ein Sauerstoffatom ersetzt sind, in Gegenwart eines Reduktionsmittels umgesetzt wird oder

e) zur Herstellung von Verbindungen der allgemeinen Formel I, in der A die —NH—CO-Gruppe, E eine gegebenenfalls durch eine Alkylgruppe mit 1 bis 3 Kohlenstoffatomen substituierte Ethylen-, n-Propylen- oder n-Butylengruppe und $R_6$ kein Wasserstoffatom darstellen, eine Verbindung der allgemeinen Formel

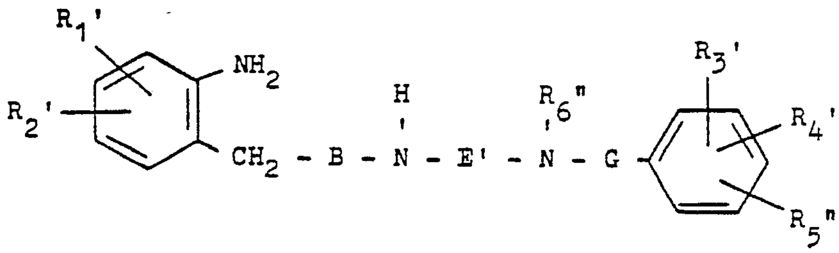

(X)

in der

B und G wie eingangs definiert sind,

E' eine gegebenenfalls durch eine Alkylgruppe mit 1 bis 3 Kohlenstoffatomen substituierte Ethylen-, n-Propylen- oder n-Butylengruppe,

$R_1'$ bis $R_4'$ je eine durch einen Schutzrest geschützte Hydroxygruppe darstellen oder die für $R_1$ bis $R_4$ eingangs erwähnten Bedeutungen besitzen,

$R_5''$ eine durch einen Schutzrest geschützte Hydroxy-, Amino- oder Alkylaminogruppe darstellt oder mit Ausnahme der Amino- oder Alkylaminogruppe die für $R_5$ eingangs erwähnten Bedeutungen besitzt und

$R_6''$ mit Ausnahme von Wasserstoff die für $R_6$ eingangs erwähnten Bedeutungen besitzt oder eine Schutzgruppe für eine Aminogruppe darstellt, mit einem Kohlensäurederivat der allgemeinen Formel

W—CO—W (XI)

in der W, die gleich oder verschieden sein können, jeweils eine nukleophile Austrittsgruppe wie ein Chlor- oder Bromatom, eine gegebenenfalls durch Halogenatome substituierte Alkoxygruppe mit 1 bis 3 Kohlenstoffatomen oder eine Imidazolyl-(2)-gruppe bedeuten, umgesetzt wird und gegebenenfalls anschließende Abspaltung eines verwendeten Schutzrestes oder

f) zur Herstellung von Verbindungen der allgemeinen Formel I, in der A die —$CH_2$—$CH_2$-Gruppe, $R_1$ und $R_2$ keine Benzyloxygruppe, $R_3$ oder $R_4$ keine Nitrogruppe und $R_6$ keine Benzylgruppe oder keine Alkenylgruppe mit 3 bis 5 Kohlenstoffatomen darstellen, eine Verbindung der allgemeinen Formel

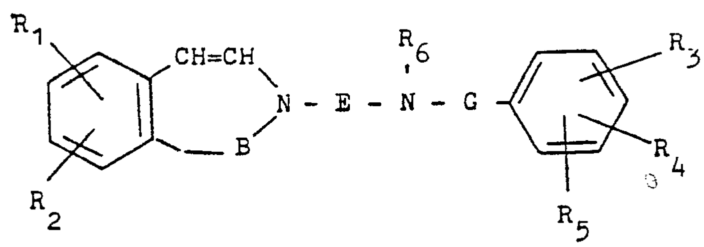

(XII)

in der $R_1$ bis $R_5$, B, E und G wie eingangs definiert sind, wobei $R_3$ oder $R_4$ keine Nitrogruppe darstellen können, falls $R_5$ eine Aminogruppe bedeutet, hydriert wird oder

g) eine Verbindung der allgemeinen Formel

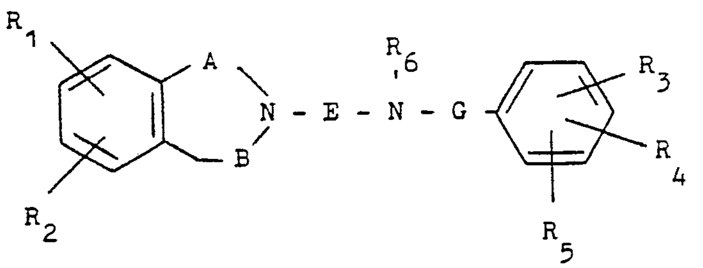

(XIII)

in der $R_1$ bis $R_6$, A, B, E und G wie eingangs definiert sind, wobei jedoch mindestens einer der Reste $R_1$ bis $R_4$ eine Hydroxygruppe oder $R_5$ eine Hydroxygruppe, eine Amino- oder Alkylaminogruppe mit 1 bis 3 Kohlenstoffatomen oder $R_6$ ein Wasserstoffatom darstellen muß, mit einer Verbindung der allgemeinen Formel

$$R_8—X, \qquad (XIV)$$

in der $R_8$ eine Alkylgruppe mit 1 bis 3 Kohlenstoffatomen oder auch eine Alkenylgruppe mit 3 bis 5 Kohlenstoffatomen, wenn $R_6$ ein Wasserstoffatom darstellt, oder auch eine durch eine Phenylgruppe substituierte Alkylgruppe mit 1 bis 3 Kohlenstoffatomen, wenn $R_1$ oder $R_2$ die Hydroxygruppe und/oder $R_6$ ein Wasserstoffatom darstellen, und X eine nukleophile Austrittsgruppe wie eine Halogenatom oder eine Sulfonyloxygruppe oder, falls mindestens einer der Reste $R_1$ bis $R_5$ eine Hydroxygruppe darstellt, X zusammen mit einem α-ständigen Wasserstoffatom des Restes $R_8$ eine Diazogruppe oder auch, falls $R_6$ ein Wasserstoffatom oder $R_5$ eine Amino- oder Alkylaminogruppe darstellen, ein Sauerstoffatom bedeuten, umgesetzt wird oder

h) zur Herstellung von Verbindungen der allgemeinen Formel I, in der A keine —CH=CH-Gruppe, $R_3$ ein Chlor- oder Bromatom und $R_5$ eine Amino- oder Alkylaminogruppe darstellen, eine Verbindung der allgemeinen Formel

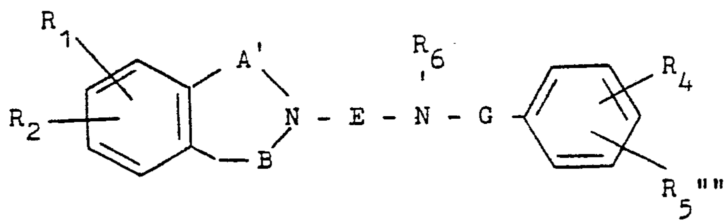

(XV)

in der

$R_1$, $R_2$, $R_4$, $R_6$, B, E und G wie eingangs definiert sind,

A' mit Ausnahme der —CH=CH-Gruppe die für A eingangs erwähnten Bedeutungen besitzt und

$R_5''''$ eine Amino- oder Alkylaminogruppe mit 1 bis 3 Kohlenstoffatomen darstellt, halogeniert wird oder

i) zur Herstellung von Verbindungen der allgemeinen Formel I, in der A die —COCO-Gruppe, B die Methylengruppe und E eine gegebenenfalls durch eine Alkylgruppe mit 1 bis 3 Kohlenstoffatomen substituierte Ethylen-, n-Propylen- oder n-Butylengruppe darstellen, eine Verbindung der allgemeinen Formel

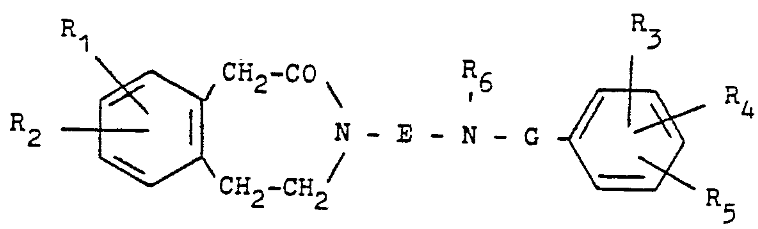

(XVI)

in der $R_1$ bis $R_6$, E und G wie eingangs definiert sind, oxidiert wird

und gewünschtenfalls anschließend eine erhaltene Verbindung der allgemeinen Formel I, in der B die Carbonylgruppe darstellt, mittels Reduktion in eine entsprechende Verbindung der allgemeinen Formel I, in der B die Methylengruppe darstellt, übergeführt wird und/oder

eine erhaltene Verbindung der allgemeinen Formel I, in der

A keine —CH=CH- oder

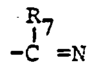-Gruppe

und $R_6$ eine Benzyl- oder 1-Phenylethylgruppe darstellen, mittels katalytischer Hydrierung in eine entsprechende Verbindung der allgemeinen Formel I, in der $R_6$ ein Wasserstoffatom darstellt, übergeführt wird und/oder

eine erhaltene Verbindung der allgemeinen Formel I, in der $R_3$ oder $R_4$ eine Nitrogruppe darstellt, mittels Reduktion in eine entsprechende Verbindung der allgemeinen Formel I, in der $R_5$ eine Aminogruppe darstellt, übergeführt wird und/oder

eine erhaltene Verbindung der allgemeinen Formel I, in der $R_6$ ein Wasserstoffatom und/oder $R_5$ eine Aminogruppe darstellt, mittels Acylierung in eine entsprechende Verbindung der allgemeinen Formel I, in der $R_6$ eine Alkanoyl- oder Alkoxycarbonylgruppe und/oder $R_5$ eine Alkanoylamino-, Alkoxycarbonylamino- oder Bis(alkoxycarbonylaminogruppe) darstellen, wobei der Alkylteil jeweils 1 bis 3 Kohlenstoffatome enthalten kann, übergeführt wird und/oder

eine erhaltene Verbindung der allgemeinen Formel I, in der $R_5$ eine Aminogruppe, $R_6$ kein Wasserstoffatom, $R_3$ und $R_4$ jeweils keine Cyangruppe darstellen, über ein entsprechendes Diazoniumsalz in eine entsprechende Verbindung der allgemeinen Formel I, in der $R_5$ ein Wasserstoffatom, eine Hydroxy- oder Alkoxygruppe oder $R_5$ ein Wasserstoffatom und $R_3$ ein Halogenatom oder die Cyangruppe bedeuten, übergeführt wird und/oder

eine erhaltene Verbindung der allgemeinen Formel I in ihre Säureadditionssalze, insbesondere in ihre physiologisch verträglichen Salze mit anorganischen oder organischen Säuren übergeführt wird.

2. Verfahren gemäß Anspruch 1 zur Herstellung von neuen Benzazepinderivaten der allgemeinen Formel

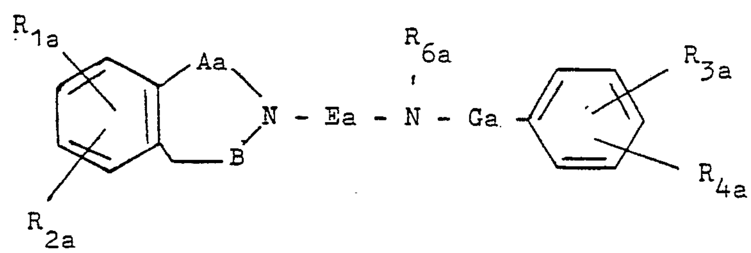

(Ia)

in der

Aa die $—CH_2—CH_2$-, —CH=CH- oder—NH—CO-Gruppe,

5

B die Methylen- oder Carbonylgruppe,

Ea die Ethylen-, n-Propylen- oder n-Butylengruppe,

Ga die Ethylengruppe,

$R_{1a}$ eine Alkoxygruppe mit 1 bis 3 Kohlenstoffatomen,

$R_{2a}$ bis $R_{4a}$, die gleich oder verschieden sein können, jeweils Wasserstoffatome oder Alkoxygruppen mit 1 bis 3 Kohlenstoffatomen oder

$R_{1a}$ zusammen mit $R_{2a}$ und/oder $R_{3a}$ zusammen mit $R_{4a}$ je eine Methylendioxygruppe und

$R_{6a}$ ein Wasserstoffatom, eine gegebenenfalls durch eine Phenylgruppe substituierte Alkylgruppe mit 1 bis 3 Kohlenstoffatomen oder die Allylgruppe bedeuten, und von deren Säureadditionssalze, insbesondere von deren physiologisch verträglichen Säureadditionssalzen mit anorganischen oder organischen Säuren, dadurch gekennzeichnet, daß

b) eine Verbindung der allgemeinen Formel

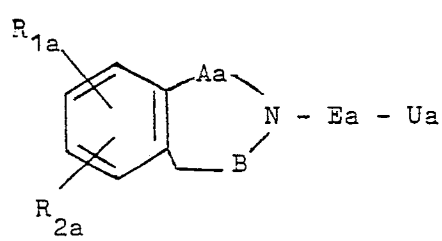

(IVa)

mit einer Verbindung der allgemeinen Formel

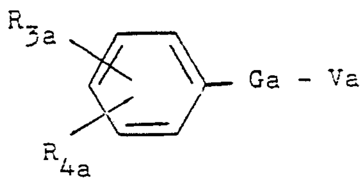

(Va)

in denen $R_{1a}$ bis $R_{4a}$, Aa, Ea und Ga wie eingangs definiert sind, einer der Reste Ua oder Va die $R_{6a}$-NH-Gruppe darstellt, wobei $R_{6a}$ wie eingangs definiert ist, und der andere der Reste Ua oder Va eine nukleophile Austrittsgruppe wie ein Halogenatom oder eine Sulfonyloxygruppe darstellt, umgesetzt wird oder

e) zur Herstellung einer Verbindung der allgemeinen Formel Ia, in der Aa die —NH—CO-Gruppe und $R_{6a}$ kein Wasserstoffatom darstellt, eine Verbindung der allgemeinen Formel

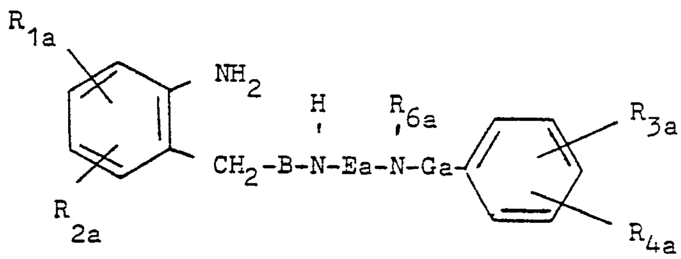

(Xa)

in der $R_{1a}$ bis $R_{4a}$, B, Ea, Ga und $R_{6a}$ mit Ausnahme des Wasserstoffatoms wie eingangs definiert sind, mit einem Kohlensäurederivat der allgemeinen Formel

$$Wa—CO—Wa, \qquad (XIa)$$

in der Wa, die gleich oder verschieden sein können, jeweils eine nukleophile Austrittsgruppe bedeuten, umgesetzt wird oder

f) zur Herstellung einer Verbindung der allgemeinen Formel Ia, in der Aa die $—CH_2—CH_2$-Gruppe darstellt, eine Verbindung der allgemeinen Formel

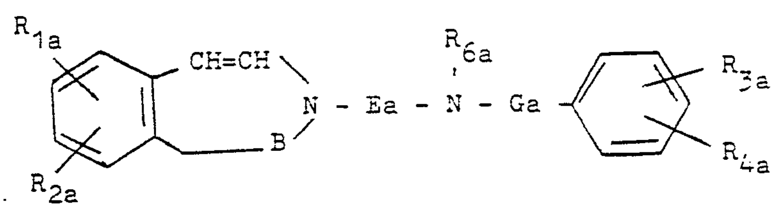

(XIIa)

in der $R_{1a}$ bis $R_{4a}$, $R_{6a}$, B, Ea und Ga wie eingangs definiert sind, in Gegenwart eines Hydrierungskatalysators und in einem Lösungsmittel hydriert wird oder

g) eine Verbindung der allgemeinen Formel

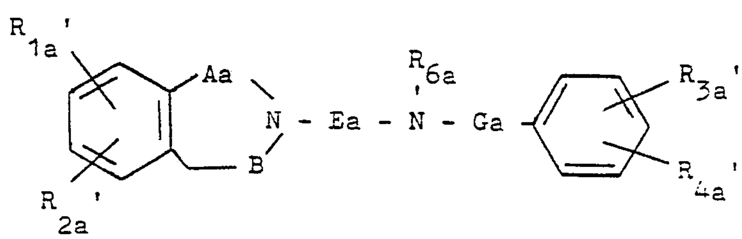

(XIIIa)

in der

$R_{6a}$, A, B, Ea und Ga wie eingangs definiert sind und

$R_{1a}'$ bis $R_{4a}'$ je eine Hydroxygruppe darstellen oder die für $R_{1a}$ bis $R_{4a}$ eingangs erwähnten Bedeutungen besitzen, wobei jedoch mindestens einer der Reste $R_{1a}'$ bis $R_{4a}'$ eine Hydroxygruppe oder $R_{6a}$ ein Wasserstoffatom darstellen muß, mit einer Verbindung der allgemeinen Formel

$$R_{8a}—X, \qquad (XIVa)$$

in der

$R_{8a}$ eine gegebenenfalls durch eine Phenylgruppe substituierte Alkylgruppe mit 1 bis 3 Kohlenstoffatomen und

X eine nukleophile Austrittsgruppe wie ein Halogenatom oder eine Sulfonyloxygruppe, oder, falls mindestens einer der Reste $R_{1a}'$ bis $R_{4a}'$ eine Hydroxygruppe darstellt, zusammen mit einem α-ständigen Wasserstoffatom des Restes $R_{8a}$ eine Diazogruppe oder auch ein Sauerstoffatom, falls $R_{6a}$ ein Wasserstoffatom darstellt, bedeuten, umgesetzt wird

und gewünschtenfalls anschließend eine so erhaltene Verbindung der allgemeinen Formel Ia, in der B die Carbonylgruppe darstellt, mittels Reduktion in eine entsprechende Verbindung der allgemeinen Formel Ia, in der B die Methylengruppe darstellt, übergeführt wird und/oder

eine so erhaltene Verbindung der allgemeinen Formel Ia, in der $R_{6a}$ eine Benzyl- oder 1-Phenyläthylgruppe derstellt, mittels katalytischer Hydrierung in eine entsprechende Verbindung der allgemeinen Formel Ia, in der $R_{6a}$ ein Wasserstoffatom darstellt, übergeführt wird und/oder

eine erhaltene Verbindung der allgemeinen Formel Ia in ihr Säureadditionssalz, insbesondere in ihr physiologisch verträgliches Säureadditionssalz mit einer anorganischen oder organischen Säure übergeführt wird.

Priorität : 19. Mai 1981 (P 31 19 874.0)

3. Verfahren gemäß den Ansprüchen 1 und 2, dadurch gekennzeichnet, daß die Umsetzung in einem Lösungsmittel durchgeführt wird.

4. Verfahren gemäß den Ansprüchen 1a, 1b, 1e, 2b, 2e und 3, dadurch gekennzeichnet, daß die Umsetzung in Gegenwart einer Base und bei Temperaturen zwischen 0 und 150 °C, vorzugsweise jedoch bei der Siedetemperatur des verwendeten Lösungsmittels, durchgeführt wird.

5. Verfahren gemäß den Ansprüchen 1a, 1b und 3, dadurch gekennzeichnet, daß ein verwendeter Schutzrest hydrolytisch in Gegenwart einer Säure oder Base oder ein als Schutzrest verwendeter Benzylrest hydrogenolytisch abgespalten wird.

6. Verfahren gemäß den Ansprüchen 1c, 1d und 3, dadurch gekennzeichnet, daß zur Herstellung von Verbindungen der allgemeinen Formel I, in der $R_6$ ein Wasserstoffatom darstellt, die Umsetzung in Gegenwart von Wasserstoff und einem Hydrierungskatalysator durchgeführt wird.

7. Verfahren gemäß den Ansprüchen 1c, 1d und 3, dadurch gekennzeichnet, daß die Umsetzung in Gegenwart eines komplexen Metallhydrids wie Lithium- oder Natriumcyanborhydrid bei einem pH-Wert von 6-7 und bei Raumtemperatur durchgeführt wird.

8. Verfahren gemäß den Ansprüchen 1g, 2g und 3, dadurch gekennzeichnet, daß die Umsetzung in Gegenwart eines säurebindenden Mittels und bei Temperaturen zwischen 0 und 150 °C, vorzugsweise jedoch bei Temperaturen zwischen 20 und 80 °C, durchgeführt wird, wenn X eine nukleophile Austrittsgruppe darstellt.

9. Verfahren gemäß den Ansprüchen 1g, 2g und 3, dadurch gekennzeichnet, daß die Umsetzung mit einer Diazoverbindung der allgemeinen Formel XIV bei Temperaturen zwischen 0 und 30 °C durchgeführt wird, wenn mindestens einer der Reste $R_1$ bis $R_5$ eine Hydroxygruppe darstellt.

10. Verfahren gemäß den Ansprüchen 1g, 2g und 3, dadurch gekennzeichnet, daß die Umsetzung mit einem Aldehyd der allgemeinen Formel XIV in Gegenwart eines Reduktionsmittels bei Temperaturen zwischen 0 und 120 °C durchgeführt wird, wenn $R_6$ ein Wasserstoffatom und/oder $R_5$ eine Amino- oder Alkylaminogruppe darstellen.

11. Verfahren zur Herstellung eines Arzneimittels, dadurch gekennzeichnet, daß eine Verbindung der allgemeinen Formel I oder ein physiologisch verträgliches Säureadditionssalz hiervon auf nichtchemischem Wege in einen oder mehrere inerte Trägerstoffe und/oder Verdünnungsmitteln eingearbeit wird.

**Claims** (for the Contracting States : BE, CH, DE, FR, IT, LI, LU, NL, SE)

1. Benzazepine derivatives of general formula

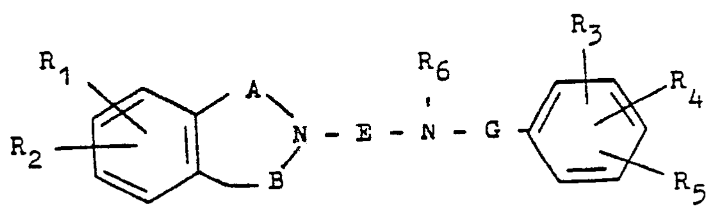

(I)

wherein

A represents a $—CH_2—CH_2$-, —CH=CH-, —NH—CO-, $—CH_2—CO$- or

5 5

$-\overset{R_7}{\overset{|}{C}}$ =N-group,

5

wherein $R_7$ represents an alkyl group containing 1 to 3 carbon atoms, and B represents a methylene or carbonyl group or

A represents a —CO—CO group and B represents a methylene group,

E represents an ethylene, n-propylene or n-butylene group optionally substituted by an alkyl group containing 1 to 3 carbon atoms, an n-propylene group substituted by a hydroxy group in the 2 position or an n-butylene group substituted by a hydroxy group in the 2 or 3 position,

G represents a methylene or ethylene group optionally substituted by an alkyl group with 1 to 3 carbon atoms,

$R_1$ and $R_2$, which may be the same or different, represent hydroxy groups, alkyl, alkoxy or phenylalkoxy groups wherein the alkyl part may contain 1 to 3 carbon atoms, or one of the groups $R_1$ or $R_2$ may also represent a hydrogen atom or $R_1$ together with $R_2$ may represent an alkylenedioxy group with 1 or 2 carbon atoms,

$R_3$ and $R_4$, which may be the same or different, represent hydrogen or halogen atoms, hydroxy groups, alkyl or alkoxy groups each having 1 to 4 carbon atoms, trifluoromethyl or cyano groups or one of the groups $R_3$ or $R_4$ may also represent a nitro group or $R_3$ together with $R_4$ may represent an alkylenedioxy group with 1 or 2 carbon atoms,

$R_5$ represents a hydrogen atom, an alkyl, hydroxy, alkoxy, amino, alkylamino, dialkylamino, alkanoylamino, alkoxycarbonylamino or bis(alkoxycarbonyl)amino group wherein the alkyl part may contain 1 to 3 carbon atoms, or a methylamino or ethylamino group substituted by a trifluoromethyl group, and

$R_6$ represents a hydrogen atom, an alkyl, phenylalkyl, alkanoyl or alkoxycarbonyl group wherein the alkyl part may contain from 1 to 3 carbon atoms, or an alkenyl group with 3 to 5 carbon atoms, and the addition salts thereof, particularly the physiologically acceptable acid addition salts thereof with inorganic or organic acids.

2. Benzazepine derivatives of general formula I as claimed in claim 1, wherein

A represents a $—CH_2—CH_2$-, —CH=CH-, —NH—CO-, $—CH_2—CO$- or

5 5

$CH_3$
|
-C = N-group
5

and B represents a methylene or carbonyl group or

A represents a —CO—CO-group and B represents a methylene group,

E represents an ethylene, n-propylene, n-butylene, 2-methyl-n-propylene or 2-hydroxy-n-propylene group,

G represents a methylene, ethylene or 1-methyl-ethylene group,

$R_1$ represents a methyl, hydroxy, benzyloxy or alkoxy group with 1 to 3 carbon atoms,

$R_2$ represents a hydrogen atom, a methyl, hydroxy or methoxy group or

$R_1$ and $R_2$ together represent the methylenedioxy group,

$R_3$ represents a hydrogen, fluorine, chlorine or bromine atom or a trifluoromethyl, nitro, alkyl or alkoxy group each having 1 to 4 carbon atoms,

$R_4$ represents a hydrogen, chlorine or bromine atom or a methyl, methoxy or cyano group or $R_3$ and $R_4$ together represent the methylenedioxy group,

$R_5$ represents a hydrogen atom or a hydroxy, methoxy, amino, acetylamino, ethoxycarbonylamino, bis(ethoxycarbonyl)amino, dimethylamino or β,β,β-trifluoroethylamino group and

$R_6$ represents a hydrogen atom, an alkyl group with 1 to 3 carbon atoms, an allyl, benzyl, acetyl or ethoxycarbonyl group, and the acid addition salts thereof, particularly the physiologically acceptable acid addition salts thereof with inorganic or organic acids.

3. Benzazepine derivatives of general formula I as claimed in claim 1, wherein

A represents a $—CH_2—CH_2$-, —CH=CH- or

$CH_3$
|
-C = N-group
5

and B represents a carbonyl group or

A represents a —CH=CH-, —NH—CO- or —CO—CO-group and B represents a methylene group,

5

E represents an n-propylene group,

G represents the ethylene group,

$R_1$ represents a methoxy or hydroxy group,

$R_2$ represents a methoxy group or $R_1$ and $R_2$ together represent a methylenedioxy group,

$R_3$ represents a methoxy or trifluoromethyl group or a chlorine or bromine atom,

$R_4$ represents a methoxy group, a hydrogen, chlorine or bromine atom or $R_3$ and $R_4$ together represent a methylenedioxy group,

$R_5$ represents a hydrogen atom or an amino or methoxy group and

$R_6$ represents a hydrogen atom or a methyl, ethyl, n-propyl or allyl group, and the physiologically acceptable acid addition salts thereof with inorganic or organic acids.

4. Benzazepine derivatives of general formula

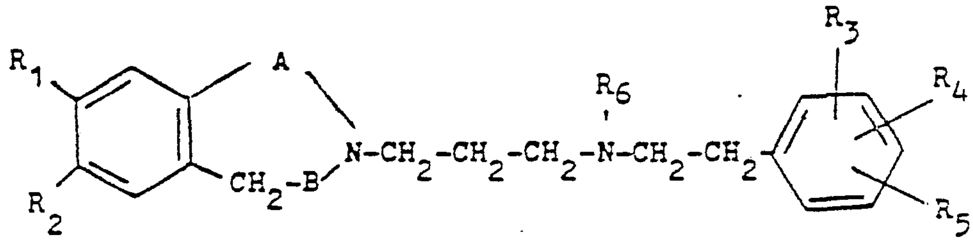

wherein

A represents a $—CH_2—CH_2$-, —CH=CH- or

$CH_3$

$-C = N$-group

5

and B represents a carbonyl group or

A represents a —NH—CO- or —COCO-group and B represents a methylene group,

5

$R_1$ and $R_2$ each represent a methoxy group,

$R_6$ represents a hydrogen atom or a methyl or allyl group,

$R_3$ represents a hydrogen atom or a methoxy group in the 3 position,

$R_4$ represents a methoxy group in the 4 position or $R_3$ and $R_4$ together represent a 3,4-methylenedioxy group and

$R_5$ represents a hydrogen atom or

$R_3$ in the 3 position represents a trifluoromethyl group or a chlorine or bromine atom,

$R_4$ in the 5 position represents a chlorine or bromine atom and

$R_5$ in the 4 position represents an amino group, and the physiologically acceptable acid addition salts thereof with inorganic or organic acids.

5. 1-[7,8-Dimethoxy-1,3,4,5-tetrahydro-2H-3-benzazepin-2-on-3-yl]-3-[N-)methyl-N-(2-(3,4-dimethoxyphenyl)-ethyl)-amino]-propane and the acid addition salts thereof.

6. 1-[7,8-Dimethoxy-1,3,4,5-tetrahydro-2H-3-benzazepin-1,2-dion-3-yl]-3-[N-methyl-N-(2-(3,4-dimethoxy-phenyl)-ethyl)-amino]-propane and the acid addition salts thereof.

7. 1-[7,8-Dimethoxy-1,3,4,5-tetrahydro-2H-3-benzazepin-2-on-3-yl]-3-[N-methyl-N-(2-(4-amino-3,5-dichloro-phenyl)-ethyl)-amino]-propane and the acid addition salts thereof.

8. 1-[7,8-Dimethoxy-1,3,4,5-tetrahydro-2H-3-benzazepin-2-on-3-yl]-3-[N-methyl-N-(2-(4-methoxyphenyl)-ethyl)-amino]-propane and the acid addition salts thereof.

9. Pharmaceutical compositions for controlling sinus tachycardia and ischaemic heart disease, containing a compound as claimed in claims 1 to 8 together with one or more inert carriers and/or diluents.

10. Process for preparing benzazepine derivatives of general formula

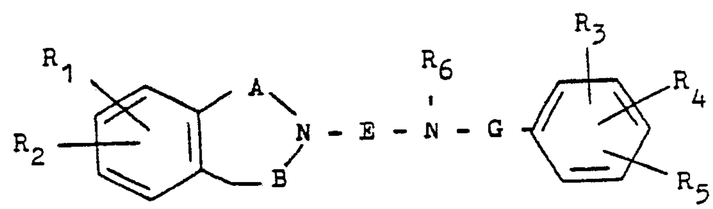

(I)

wherein

A represents a $—CH_2—CH_2$-, —CH=CH-, —NH—CO-, $—CH_2—CO$- or

5 5

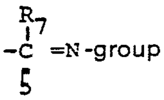

wherein $R_7$ represents an alkyl group containing 1 to 3 carbon atoms, and B represents a methylene or carbonyl group or

A represents a —CO—CO group and B represents a methylene group,

E represents an ethylene, n-propylene or n-butylene group optionally substituted by an alkyl group containing 1 to 3 carbon atoms, an n-propylene group substituted by a hydroxy group in the 2 position or an n-butylene group substituted by a hydroxy group in the 2 or 3 position,

G represents a methylene or ethylene group optionally substituted by an alkyl group with 1 to 3 carbon atoms,

$R_1$ and $R_2$, which may be the same or different, represent hydroxy groups, alkyl, alkoxy or phenylalkoxy groups wherein the alkyl part may contain 1 to 3 carbon atoms, or one of the groups $R_1$ or $R_2$ may also represent a hydrogen atom or $R_1$ together with $R_2$ may represent an alkylenedioxy group with 1 or 2 carbon atoms,

$R_3$ and $R_4$, which may be the same or different, represent hydrogen or halogen atoms, hydroxy groups, alkyl or alkoxy groups each having 1 to 4 carbon atoms, trifluoromethyl or cyano groups or one of the groups $R_3$ or $R_4$ may also represent a nitro group or $R_3$ together with $R_4$ may represent an alkylenedioxy group with 1 or 2 carbon atoms,

$R_5$ represents a hydrogen atom, an alkyl, hydroxy, alkoxy, amino, alkylamino, dialkylamino, alkanoylamino, alkoxycarbonylamino or bis(alkoxycarbonyl)amino group wherein the alkyl part may contain 1 to 3 carbon atoms, or a methylamino or ethylamino group substituted by a trifluoromethyl group, and

$R_6$ represents a hydrogen atom, an alkyl, phenylalkyl, alkanoyl or alkoxycarbonyl group wherein the alkyl part may contain from 1 to 3 carbon atoms, or an alkenyl group with 3 to 5 carbon atoms, and the addition salts thereof, particularly the physiologically acceptable acid addition salts thereof with inorganic or organic acids, characterised in that

a) a compound of general formula

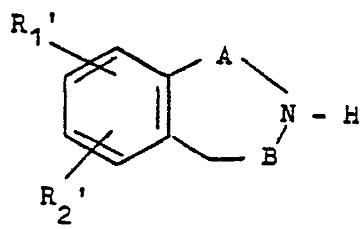

(II)

wherein

A and B are as hereinbefore defined,

$R_1'$ and $R_2'$ each represent a hydroxy group protected by a protecting group or have the meanings given for $R_1$ and $R_2$ hereinbefore, is reacted with a compound of general formula

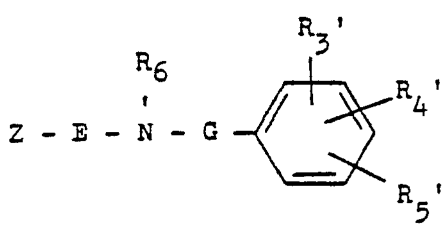

(III)

wherein

$R_6$, E and G are as hereinbefore defined, $R_3'$ to $R_5'$ represent hydroxy groups protected by a protecting group or have the meanings given for $R_3$ to $R_5$ hereinbefore and

Z represents a nucleophilically exchangeable group, or the internal quaternary salts thereof, optionally present in the reaction mixture, of general formula

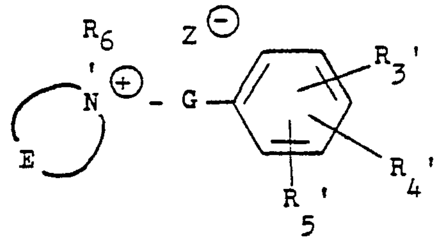

(IIIa)

wherein E, G, Z, $R_3'$ to $R_5'$ and $R_6$ are as hereinbefore defined, and subsequently, if desired, the protecting group used is split off or

b) in order to prepare compounds of general formula I wherein $R_6$ represents a hydrogen atom, an alkenyl group with 3 to 5 carbon atoms, an alkyl or phenylalkyl group with 1 to 3 carbon atoms in the alkyl part, a compound of general formula

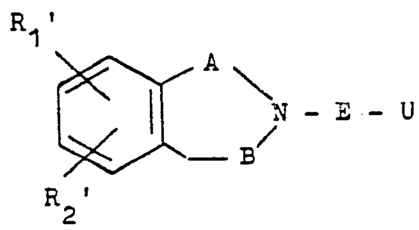

(IV)

is reacted with a compound of general formula

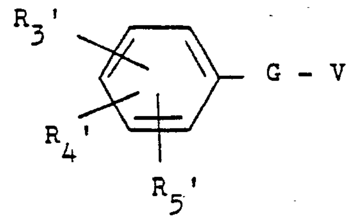

(V)

wherein

A, B, E and G are as hereinbefore defined, $R_1'$ to $R_5'$ represent hydroxy groups protected by protecting groups or have the meanings given for $R_1$ to $R_5$ hereinbefore, one of the groups U or V represents the $R_6'$—NH group in which $R_6'$ represents a hydrogen atom, an alkenyl group with 3 to 5 carbon atoms or an alkyl or phenylalkyl group with 1 to 3 carbon atoms in the alkyl part, the other group U or V represents a nucleophilically exchangeable group or the group U together with a hydrogen atom of the group E in the β position represents an oxygen atom and V represents an $R_6'$—NH group in which $R_6'$ is as hereinbefore defined, and subsequently, if desired, any protecting group used is split off or

c) in order to prepare compounds of general formula I wherein $R_6$ represents a hydrogen atom, an alkenyl group with 3 to 5 carbon atoms, an alkyl or phenylalkyl group with 1 to 3 carbon atoms in the alkyl part, a compound of general formula

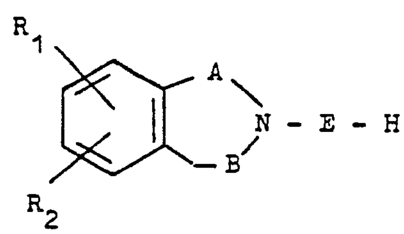

(VI)

wherein A, B, E, $R_1$ and $R_2$ are as hereinbefore defined, but in the group E two hydrogen atoms in a $—CH_2—$ or $CH_3$ group of the group E are replaced by an oxygen atom, is reacted with an amine of general formula

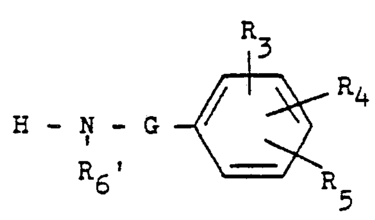

(VII)

wherein G and $R_3$ to $R_5$ are as hereinbefore defined and $R_6'$ represents a hydrogen atom, an alkenyl group with 3 to 5 carbon atoms, an alkyl or phenylalkyl group with 1 to 3 carbon atoms in the alkyl part, in the presence of a reducing agent or

d) in order to prepare compounds of general formula I wherein $R_6$ represents a hydrogen atom, an alkenyl group with 3 to 5 carbon atoms, an alkyl or phenylalkyl group with 1 to 3 carbon atoms in the alkyl part, a compound of general formula

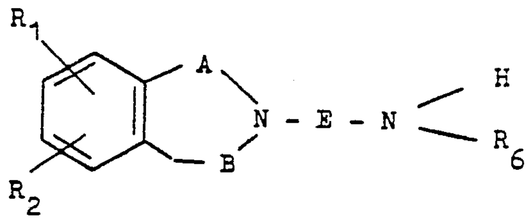

(VIII)

wherein A, B, E, $R_1$ and $R_2$ are as hereinbefore defined and $R_6$' represents a hydrogen atom, an alkenyl group with 3 to 5 carbon atoms, an alkyl or phenylalkyl group with 1 to 3 carbon atoms in the alkyl part, is reacted with a compound of general formula

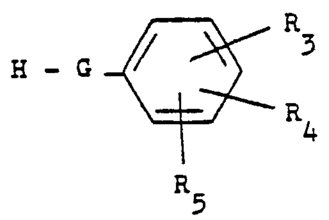

(IX)

wherein G and $R_3$ to $R_5$ are as hereinbefore defined, but in the group G two hydrogen atoms in a —$CH_2$— or $CH_3$ group of the group G are replaced by an oxygen atom, in the presence of a reducing agent or

e) in order to prepare compounds of general formula I wherein A represents an —NH—CO-group, E represents an ethylene, n-propylene or n-butylene group optionally substituted by an alkyl group with 1 to 3 carbon atoms and $R_6$ does not represent a hydrogen atom, a compound of general formula

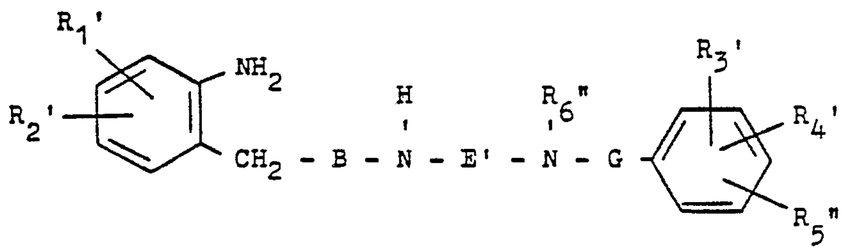

(X)

wherein

B and G are as hereinbefore defined,

E' represents an ethylene, n-propylene or n-butylene group optionaly substituted by an alkyl group with 1 to 3 carbon atoms,

$R_1$' to $R_4$' each represent a hydroxy group protected by a protecting group or have the meanings given for $R_1$ to $R_4$ hereinbefore,

$R_5$" represents a hydroxy, amino or alkylamino group protected by a protecting group or has the meanings given for $R_5$ hereinbefore, with the exception of the amino or alkylamino group, and

$R_6$" has the meanings given for $R_6$ hereinbefore with the exception of hydrogen or represents a protecting group for an amino group, is reacted with a carbonic acid derivative of general formula

W—CO—W (XI)

wherein W, which may be the same or different, in each case represents a nucleophilically exchangeable group such as a chlorine or bromine atom, an alkoxy group with 1 to 3 carbon atoms optionally substituted by halogen atoms or an imidazolyl-(2) group, and subsequently, if desired, any protecting group used is split off or

f) in order to prepare compounds of general formula I wherein A represents a —$CH_2$—$CH_2$-group, $R_1$ and $R_2$ do not represent benzyloxy groups, $R_3$ or $R_4$ does not represent a nitro group and $R_6$ does not represent a benzyl group or an alkenyl group with 3 to 5 carbon atoms, a compound of general formula

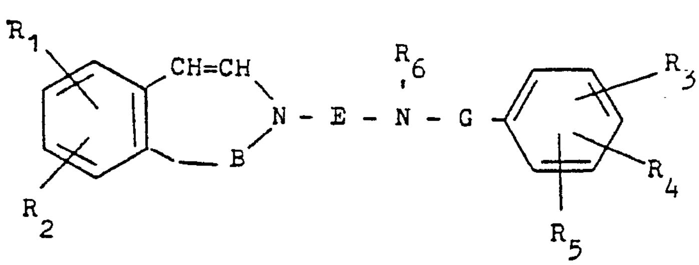

(XII)

wherein $R_1$ to $R_5$, B, E and G are as hereinbefore defined, but $R_3$ or $R_4$ cannot represent a nitro group if $R_5$ represents an amino group, is hydrogenated or

g) a compound of general formula

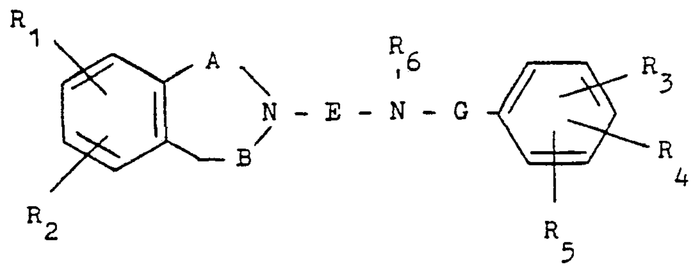

(XIII)

wherein $R_1$ to $R_6$, A, B, E and G are as hereinbefore defined, but at least one of the groups $R_1$ to $R_4$ must represent a hydroxy group or $R_5$ represents a hydroxy group or an amino or alkylamino group with 1 to 3 carbon atoms or $R_6$ must represent a hydrogen atom, is reacted with a compound of general formula

$$R_8—X \qquad (XIV)$$

wherein $R_8$ represents an alkyl group with 1 to 3 carbon atoms or an alkenyl group with 3 to 5 carbon atoms if $R_6$ represents a hydrogen atom, or an alkyl group with 1 to 3 carbon atoms substituted by a phenyl group, if $R_1$ or $R_2$ represents a hydroxy group and/or $R_6$ represents a hydrogen atom, and X represents a nucleophilically exchangeable group such as a halogen atom or a sulphonyloxy group or, if at least one of the groups $R_1$ to $R_5$ represents a hydroxy group, X together with a hydrogen atom in the α position of the group $R_8$ represents a diazo group or, if $R_6$ represents a hydrogen atom or $R_5$ represents an amino or alkylamino group, it may also represent an oxygen atom, or

h) in order to prepare compounds of general formula I wherein A does not represent a —CH=CH-group, $R_3$ represents a chlorine or bromine atom and $R_5$ represents an amino or alkylamino group, a compound of general formula

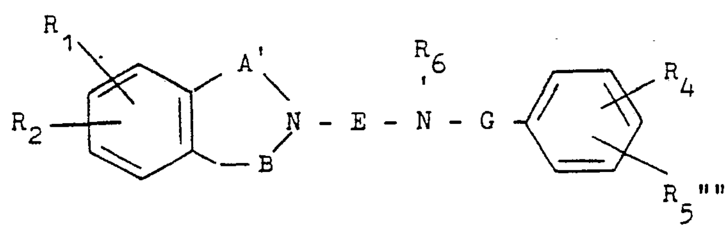

(XV)

wherein

$R_1$, $R_2$, $R_4$, $R_6$, B, E and G are as hereinbefore defined,

A' has the meanings given for A hereinbefore, with the exception of the —CH=CH-group and

$R_5''''$ represents an amino or alkylamino group with 1 to 3 carbon atoms, is halogenated or

i) in order to prepare compounds of general formula I wherein A represents a —COCO-group, B represents a methylene group and E represents an ethylene, n-propylene or n-butylene group optionally substituted by an alkyl group with 1 to 3 carbon atoms, a compound of general formula

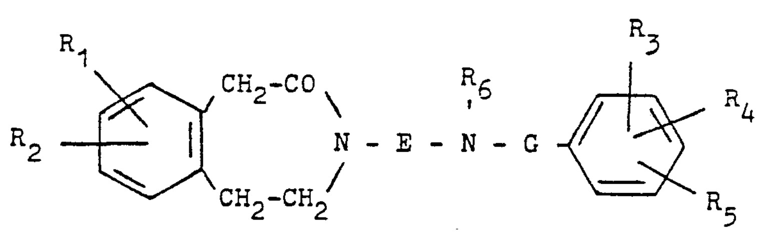

(XVI)

wherein $R_1$ to $R_6$, E and G are as hereinbefore defined, is oxidised

and subsequently, if desired, a compound of general formula I obtained wherein B represents a carbonyl group is converted by reduction into a corresponding compound of general formula I wherein B represents a methylene group, and/or

if a compound of general formula I is obtained wherein A does not represent a —CH=CH- or

$$-\overset{R_7}{\overset{|}{C}}=N\text{-group}$$

and $R_6$ represents a benzyl or 1-phenyl-ethyl group, this may be converted by catalytic hydrogenation into a corresponding compound of general formula I wherein $R_6$ represents a hydrogen atom, and/or

a compound of general formula I obtained wherein $R_3$ or $R_4$ represents a nitro group may be converted by reduction into a corresponding compound of general formula I wherein $R_5$ represents an amino group, and/or

a compound of general formula I obtained wherein $R_6$ represents a hydrogen atom and/or $R_5$ represents an amino group may be converted by acylation into a corresponding compound of general formula I wherein $R_6$ represents an alkanoyl or alkoxycarbonyl group and/or $R_5$ represents an alkanoylamino, alkoxycarbonylamino or bis(alkoxycarbonyl)amino group, wherein the alkyl part may contain from 1 to 3 carbon atoms, and/or

a compound of general formula I obtained wherein $R_5$ represents an amino group, $R_6$ does not represent a hydrogen atom, $R_3$ and $R_4$ do not represent cyano groups, may be converted via a corresponding diazonium salt into a corresponding compound of general formula I wherein $R_5$ represents a hydrogen atom or a hydroxy or alkoxy group or $R_5$ represents a hydrogen atom and $R_3$ represents a halogen atom or a cyano group, and/or

a compound of general formula I may be converted into the acid addition salts thereof, particularly the physiologically acceptable salts thereof with inorganic or organic acids.

11. Process for preparing a pharmaceutical composition, characterised in that a compound of general formula I or a physiologically acceptable acid addition salt thereof is incorporated, by a non-chemical method, in one or more inert carriers and/or diluents.

**Claims** (for the Contracting State AT)

1. Process for preparing benzazepine derivatives of general formula

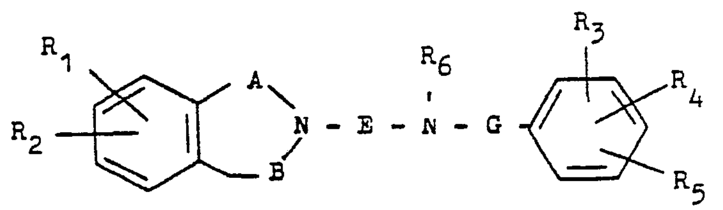

(I)

wherein

A represents a $-CH_2-CH_2-$, $-CH=CH-$, $-NH-CO-$, $-CH_2-CO-$ or

5 5

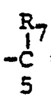

=N-group

wherein $R_7$ represents an alkyl group containing 1 to 3 carbon atoms, and B represents a methylene or carbonyl group or

A represents a $-CO-CO$ group and B represents a methylene group,

E represents an ethylene, n-propylene or n-butylene group optionally substituted by an alkyl group containing 1 to 3 carbon atoms, an n-propylene group substituted by a hydroxy group in the 2 position or an n-butylene group substituted by a hydroxy group in the 2 or 3 position,

G represents a methylene or ethylene group optionally substituted by an alkyl group with 1 to 3 carbon atoms,

$R_1$ and $R_2$, which may be the same or different, represent hydroxy groups, alkyl, alkoxy or phenylalkoxy groups wherein the alkyl part may contain 1 to 3 carbon atoms, or one of the groups $R_1$ or $R_2$ may also represent a hydrogen atom or $R_1$ together with $R_2$ may represent an alkylenedioxy group with 1 or 2 carbon atoms,

$R_3$ and $R_4$, which may be the same or different, represent hydrogen or halogen atoms, hydroxy groups, alkyl or alkoxy groups each having 1 to 4 carbon atoms, trifluoromethyl or cyano groups or one of the groups $R_3$ or $R_4$ may also represent a nitro group or $R_3$ together with $R_4$ may represent an alkylenedioxy group with 1 or 2 carbon atoms,

$R_5$ represents a hydrogen atom, an alkyl, hydroxy, alkoxy, amino, alkylamino, dialkylamino, alkanoylamino, alkoxycarbonylamino or bis(alkoxycarbonyl)amino group wherein the alkyl part may contain 1 to 3 carbon atoms, or a methylamino or ethylamino group substituted by a trifluoromethyl group, and

$R_6$ represents a hydrogen atom, an alkyl, phenylalkyl, alkanoyl or alkoxycarbonyl group wherein the alkyl part may contain from 1 to 3 carbon atoms, or an alkenyl group with 3 to 5 carbon atoms, and the addition salts thereof, particularly the physiologically acceptable acid addition salts thereof with inorganic or organic acids, characterised in that

a) a compound of general formula

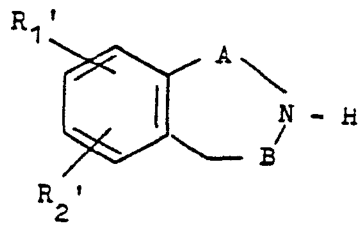

(II)

wherein

A and B are as hereinbefore defined, $R_1$' and $R_2$' each represent a hydroxy group protected by a protecting group or have the meanings given for $R_1$ and $R_2$ hereinbefore, is reacted with a compound of general formula

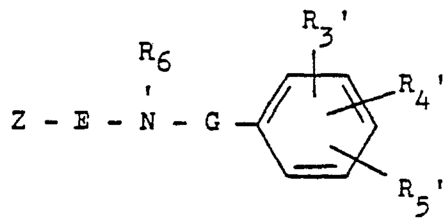

(III)

wherein

$R_6$, E and G are as hereinbefore defined, $R_3$' to $R_5$' represent hydroxy groups protected by a protecting group or have the meanings given for $R_3$ to $R_5$ hereinbefore and

Z represents a nucleophilically exchangeable group, or the internal quaternary salts thereof, optionally present in the reaction mixture, of general formula

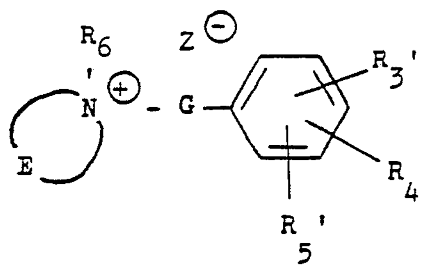

(IIIa)

wherein E, G, Z, $R_3$' to $R_5$' and $R_6$ are as hereinbefore defined, and subsequently, if desired, the protecting group used is split off or

b) in order to prepare compounds of general formula I wherein $R_6$ represents a hydrogen atom, an alkenyl group with 3 to 5 carbon atoms, an alkyl or phenylalkyl group with 1 to 3 carbon atoms in the alkyl part, a compound of general formula

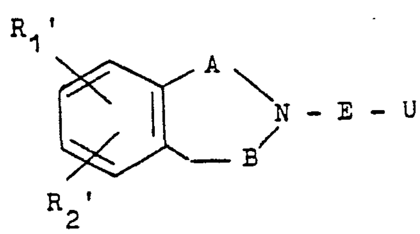

(IV)

is reacted with a compound of general formula

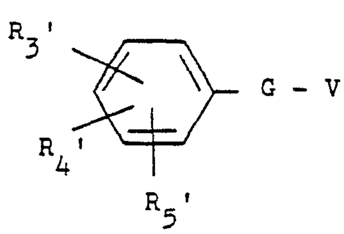

(V)

wherein

A, B, E and G are as hereinbefore defined, $R_1$' to $R_5$' represent hydroxy groups protected by protecting groups or have the meanings given for $R_1$ to $R_5$ hereinbefore, one of the groups U or V represents the $R_6$'—NH group in which $R_6$' represents a hydrogen atom, an alkenyl group with 3 to 5 carbon atoms or an alkyl or phenylalkyl group with 1 to 3 carbon atoms in the alkyl part, the other group U or V represents a nucleophilically exchangeable group or the group U together with a hydrogen atom of the group E in the β position represents an oxygen atom and V represents an $R_6$'—NH group in which $R_6$' is as hereinbefore defined, and subsequently, if desired, any protecting group used is split off or

c) in order to prepare compounds of general formula I wherein $R_{6'}$ represents a hydrogen atom, an alkenyl group with 3 to 5 carbon atoms, an alkyl or phenylalkyl group with 1 to 3 carbon atoms in the alkyl part, a compound of general formula

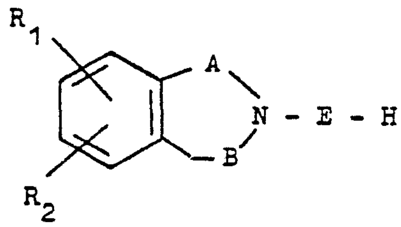

(VI)

wherein A, B, E, $R_1$ and $R_2$ are as hereinbefore defined, but in the group E two hydrogen atoms in a $—CH_2—$ or $CH_3$ group of the group E are replaced by an oxygen atom, is reacted with an amine of general formula

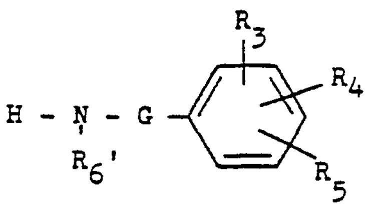

(VII)

wherein

G and $R_3$ to $R_5$ are as hereinbefore defined and $R_6$' represents a hydrogen atom, an alkenyl group with 3 to 5 carbon atoms, an alkyl or phenylalkyl group with 1 to 3 carbon atoms in the alkyl part, in the presence of a reducing agent or

d) in order to prepare compounds of general formula I wherein $R_6$ represents a hydrogen atom, an alkenyl group with 3 to 5 carbon atoms, an alkyl or phenylalkyl group with 1 to 3 carbon atoms in the alkyl part, a compound of general formula

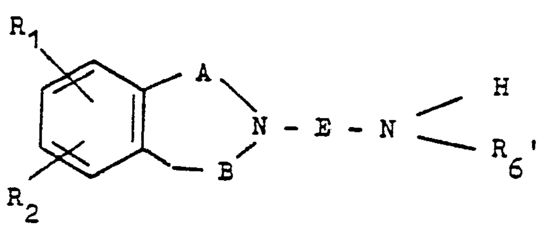

(VIII)

wherein

A, B, E, $R_1$ and $R_2$ are as hereinbefore defined and $R_6$' represents a hydrogen atom, an alkenyl group with 3 to 5 carbon atoms, an alkyl or phenylalkyl group with 1 to 3 carbon atoms in the alkyl part, is reacted with a compound of general formula

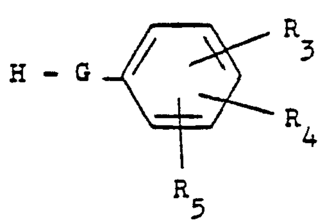

(IX)

wherein G and $R_3$ to $R_5$ are as hereinbefore defined, but in the group G two hydrogen atoms in a $—CH_2$- or $CH_3$ group of the group G are replaced by an oxygen atom, in the presence of a reducing agent or

e) in order to prepare compounds of general formula I wherein A represents an —NH—CO-group, E represents an ethylene, n-propylene or n-butylene group optionally substituted by an alkyl group with 1 to 3 carbon atoms and $R_6$ does not represent a hydrogen atom, a compound of general formula

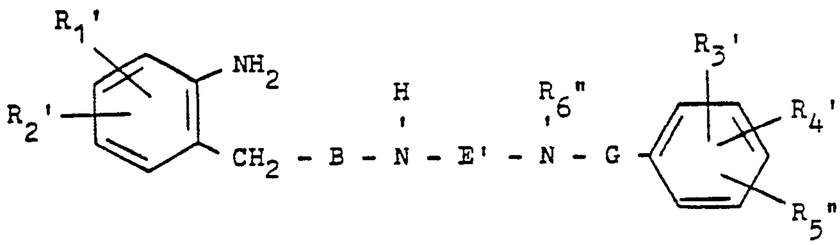

(X)

wherein

B and G are as hereinbefore defined, E' represents an ethylene, n-propylene or n-butylene group optionally substituted by an alkyl group with 1 to 3 carbon atoms,

$R_1$' to $R_4$' each represent a hydroxy group protected by a protecting group or have the meanings given for $R_1$ to $R_4$ hereinbefore,

$R_5$" represents a hydroxy, amino or alkylamino group protected by a protecting group or has the meanings given for $R_5$ hereinbefore, with the exception of the amino or alkylamino group, and

$R_6$" has the meanings given for $R_6$ hereinbefore with the exception of hydrogen or represents a protecting group for an amino group, is reacted with a carbonic acid derivative of general formula

$$W—CO—W \quad (XI)$$

wherein W, which may be the same or different, in each case represents a nucleophilically exchangeable group such as a chlorine or bromine atom, an alkoxy group with 1 to 3 carbon atoms optionally substituted by halogen atoms optionally substituted by halogen atoms or an imidazolyl-(2) group, and subsequently, if desired, any protecting group used is split off or

f) in order to prepare compounds of general formula I wherein A represents a $—CH_2—CH_2$- group, $R_1$ and $R_2$ do not represent benzyloxy groups, $R_3$ or $R_4$ does not represent a nitro group and $R_6$ does not represent a benzyl group or an alkenyl group with 3 to 5 carbon atoms, a compound of general formula

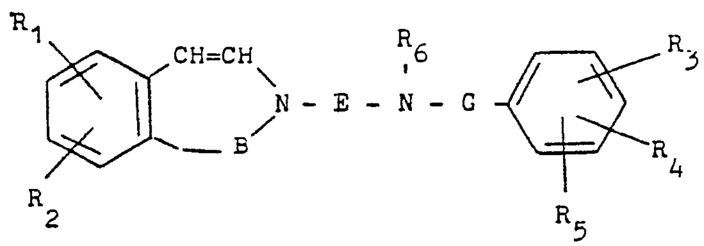

(XII)

wherein $R_1$ to $R_5$, B, E and G are as hereinbefore defined, but $R_3$ or $R_4$ cannot represent a nitro group if $R_5$ represents an amino group, is hydrogenated or

g) a compound of general formula

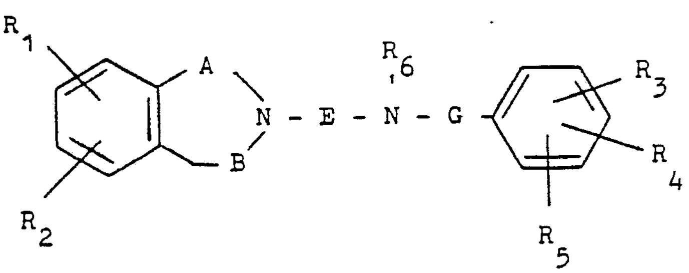

(XIII)

wherein $R_1$ to $R_6$, A, B, E and G are as hereinbefore defined, but at least one of the groups $R_1$ to $R_4$ must represent a hydroxy group or $R_5$ represents a hydroxy group or an amino or alkylamino group with 1 to 3 carbon atoms or $R_6$ must represent a hydrogen atom, is reacted with a compound of general formula

$$R_8—X \quad (XIV)$$

wherein $R_8$ represents an alkyl group with 1 to 3 carbon atoms or an alkenyl group with 3 to 5 carbon

atoms if $R_6$ represents a hydrogen atom, or an alkyl group with 1 to 3 carbon atoms substituted by a phenyl group, if $R_1$ or $R_2$ represents a hydroxy group and/or $R_6$ represents a hydrogen atom, and X represents a nucleophilically exchangeable group such as a halogen atom or a sulphonyloxy group or, if at least one of the groups $R_1$ to $R_5$ represents a hydroxy group, X together with a hydrogen atom in the $\alpha$ position of the group $R_8$ represents a diazo group or, if $R_6$ represents a hydrogen atom or $R_5$ represents an amino or alkylamino group, it may also represent an oxygen atom, or

h) in order to prepare compounds of general formula I wherein A does not represent a —CH=CH-group, $R_3$ represents a chlorine or bromine atom and $R_5$ represents an amino or alkylamino group, a compound of general formula

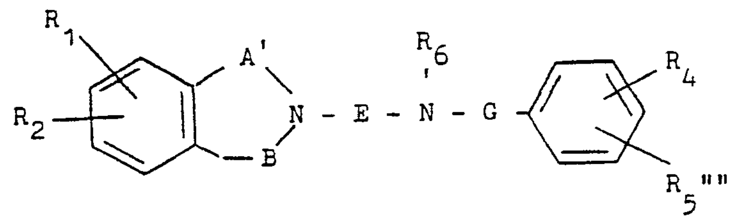

(XV)

wherein

$R_1$, $R_2$, $R_4$, $R_6$, B, E and G are as hereinbefore defined,

A′ has the meanings given for A hereinbefore, with the exception of the —CH=CH-group and

$R_5''''$ represents an amino or alkylamino group with 1 to 3 carbon atoms, is halogenated or

i) in order to prepare compounds of general formula I wherein A represents a —COCO-group, B represents a methylene group and E represents an ethylene, n-propylene or n-butylene group optionally substituted by an alkyl group with 1 to 3 carbon atoms, a compound of general formula

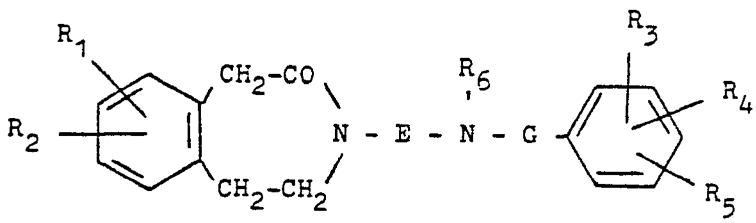

(XVI)

wherein $R_1$ to $R_6$, E and G are as hereinbefore defined, is oxidised

and subsequently, if desired, a compound of general formula I obtained wherein B represents a carbonyl group is converted by reduction into a corresponding compound of general formula I wherein B represents a methylene group, and/or

if a compound of general formula I is obtained wherein A does not represent a —CH=CH- or

$$-\overset{R_7}{\overset{|}{C}}=N\text{-group}$$

and $R_6$ represents a benzyl or 1-phenylethyl group, this may be converted by catalytic hydrogenation into a corresponding compound of general formula I wherein $R_6$ represents a hydrogen atom, and/or

a compound of general formula I obtained wherein $R_3$ or $R_4$ represents a nitro group may be converted by reduction into a corresponding compound of general formula I wherein $R_5$ represents an amino group, and/or

a compound of general formula I obtained wherein $R_6$ represents a hydrogen atom and/or $R_5$ represents an amino group may be converted by acylation into a corresponding compound of general formula I wherein $R_6$ represents an alkanoyl or alkoxycarbonyl group and/or $R_5$ represents an alkanoylamino, alkoxycarbonylamino or bis(alkoxycarbonyl)amino group, wherein the alkyl part may contain from 1 to 3 carbon atoms, and/or

a compound of general formula I obtained wherein $R_5$ represents an amino group, $R_6$ does not represent a hydrogen atom, $R_3$ and $R_4$ do not represent cyano groups, may be converted via a corresponding diazonium salt into a corresponding compound of general formula I wherein $R_5$ represents a hydrogen atom or a hydroxy or alkoxy group or $R_5$ represents a hydrogen atom and $R_3$ represents a halogen atom or a cyano group, and/or

a compound of general formula I may be converted into the acid addition salts thereof, particularly the physiologically acceptable salts thereof with inorganic or organic acids.

2. Process as claimed in claim 1 for the preparation of new benzazepine derivatives of general formula

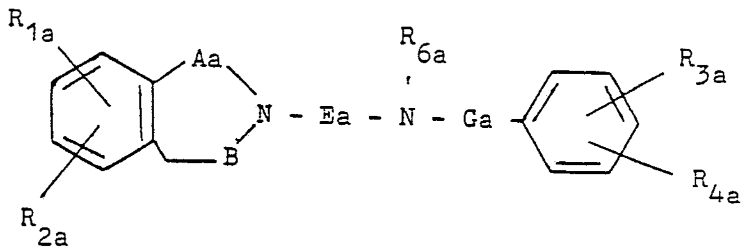

(Ia)

wherein

Aa represents a $-CH_2-CH_2-$, $-CH=CH-$ or $-NH-CO$-group
5

B represents a methylene or carbonyl group,

Ea represents an ethylene, n-propylene or n-butylene group,

Ga represents an ethylene group,

$R_{1a}$ represents an alkoxy group with 1 to 3 carbon atoms,

$R_{2a}$ to $R_{4a}$, which may be the same or different, represent hydrogen atoms or alkoxy groups with 1 to 3 carbon atoms or

$R_{1a}$ together with $R_{2a}$ and/or $R_{3a}$ together with $R_{4a}$ represent methylenedioxy groups and

$R_{6a}$ represents a hydrogen atom, an alkyl group with 1 to 3 carbon atoms optionally substituted by a phenyl group, or the allyl group, and the acid addition salts thereof, particularly the physiologically acceptable acid addition salts thereof with inorganic or organic acids, characterised in that

b) a compound of general formula

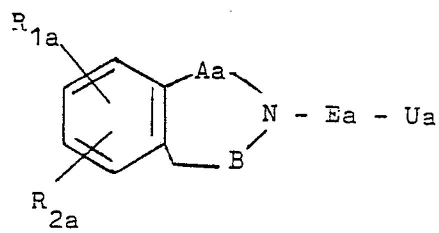

(IVa)

is reacted with a compound of general formula

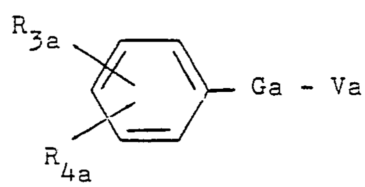

(Va)

wherein

$R_{1a}$ to $R_{4a}$, Aa, B, Ea and Ga are as hereinbefore defined, one of the groups Ua or Va represents the $R_{6a}-NH$-group wherein $R_{6a}$ is as hereinbefore defined, and the other group Ua or Va represents a nucleophilically exchangeable group such as a halogen atom or a sulphonyloxy group, or

e) in order to prepare a compound of general formula Ia wherein Aa represents a $-NH-CO$-group and $R_{6a}$ does not represent a hydrogen atom, a compound of general formula

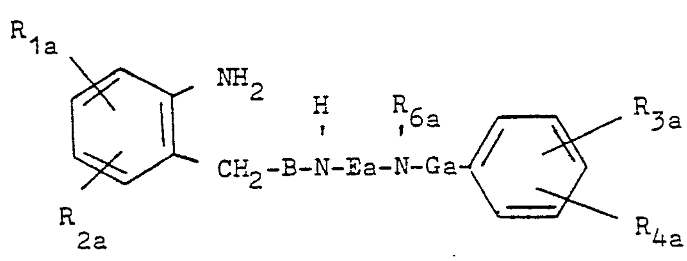

(Xa)

wherein $R_{1a}$ to $R_{4a}$, B, Ea, Ga and $R_{6a}$ have the meanings given hereinbefore, with the exception of the hydrogen atom, is reacted with a carbonic acid derivative of general formula

Wa—CO—Wa (XIa)

wherein Wa, which may be the same or different, represent nucleophilically exchangeable groups, or

f) in order to prepare a compound of general formula Ia wherein Aa represents a $-CH_2-CH_2$-group, a compound of general formula

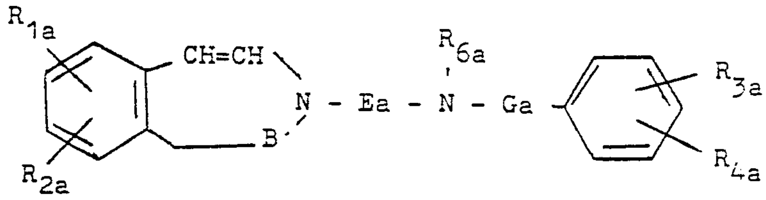

(XIIa)

wherein $R_{1a}$ to $R_{4a}$, $R_{6a}$, B, Ea and Ga are as hereinbefore defined, is hydrogenated in the presence of a hydrogenation catalyst and in a solvent or

g) a compound of general formula

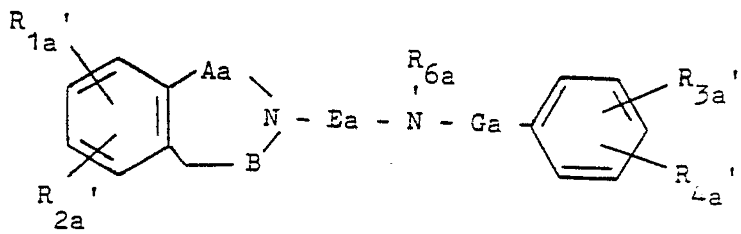

(XIIIa)

wherein

$R_{6a}$, A, B, Ea and Ga are as hereinbefore defined and

$R_{1a}'$ to $R_{4a}'$ each represent hydroxy groups or have the meanings given for $R_{1a}$ to $R_{4a}$ hereinbefore, but at least one of the groups $R_{1a}'$ to $R_{4a}'$ must represent a hydroxy group or $R_{6a}$ must represent a hydrogen atom,

is reacted with a compound of general formula

$R_{8a}$—X (XIVa)

wherein

$R_{8a}$ represents an alkyl group with 1 to 3 carbon atoms optionally substituted by a phenyl group and

X represents a nucleophilically exchangeable group such as a halogen atom or a sulphonyloxy group or, if at least one of the groups $R_{1a}'$ to $R_{4a}'$ represents a hydroxy group, X together with a hydrogen atom in the α position of the group $R_{8a}$ may represent a diazo group or an oxygen atom, if $R_{6a}$ represents a hydrogen atom,

and subsequently, if desired, a compound of general formula Ia thus obtained wherein B represents a carbonyl group, is converted by reduction into a corresponding compound of general formula Ia wherein B represents a methylene group, and/or

a compound of general formula Ia thus obtained wherein $R_{6a}$ represents a benzyl or 1-phenylethyl group, is converted by catalytic hydrogenation into a corresponding compound of general formula Ia wherein $R_{6a}$ represents a hydrogen atom, and/or

a compound of general formula Ia thus obtained may be converted into an acid addition salt thereof, particularly a physiologically acceptable acid addition salt with an inorganic or organic acid.

Priority : 19th May 1981 (P 31 19 874.0)

3. Process as claimed in claims 1 and 2, characterised in that the reaction is carried out in a solvent.

4. Process as claimed in claims 1a, 1b, 1e, 2b, 2e and 3, characterised in that the reaction is carried out in the presence of a base and at temperatures of between 0 and 150 °C, but preferably at the boiling temperature of the solvent used.

5. Process as claimed in claims 1a, 1b and 3, characterised in that any protecting group used is split off by hydrolysis in the presence of an acid or base or any benzyl group used as a protecting group is split off by hydrogenolysis.

6. Process as claimed in claims 1c, 1d and 3, characterised in that in order to prepare compounds of general formula I wherein $R_6$ represents a hydrogen atom, the reaction is carried out in the presence of hydrogen and a hydrogenation catalyst.

7. Process as claimed in claims 1c, 1d and 3, characterised in that the reaction is carried out in the presence of a complex metal hydride such as lithium or sodium cyanoborohydride at a pH value of from 6 to 7 and at ambient temperature.

8. Process as claimed in claims 1g, 2g and 3, characterised in that the reaction is carried out in the presence of an acid-binding agent and at temperatures of between 0 and 150 °C, but preferably at temperatures of between 20 and 80 °C, if X represents a nucleophilically exchangeable group.

9. Process as claimed in claims 1g, 2g and 3, characterised in that the reaction with a diazo compound of general formula XIV is carried out at temperatures of between 0 and 30 °C if at least one of the groups $R_1$ to $R_5$ is a hydroxy group.

10. Process as claimed in claims 1g, 2g and 3, characterised in that the reaction with an aldehyde of general formula XIV is carried out in the presence of a reducing agent at temperatures of between 0 and 120 °C if $R_6$ represents a hydrogen atom and/or $R_5$ represents an amino or alkylamino group.

11. Process for the preparation of a pharmaceutical composition, characterised in that a compound of general formula I or a physiologically acceptable acid addition salt thereof is incorporated, by a non-chemical method, in one or more inert carriers and/or diluents.

**Revendications** (pour les Etats contractants : BE, CH, DE, FR, IT, LI, LU, NL, SE)

1. Dérivés de benzazépine de formule générale

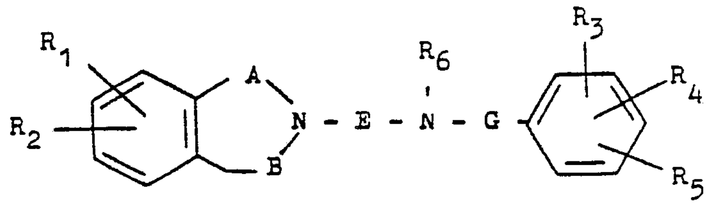

(I)

dans laquelle

A représente un groupe $-CH_2-CH_2-$, $-CH=CH-$, —NH—CO—, $-CH_2-CO-$ ou
5 5

$R_7$
|
-C =N-,
5

dans lequel $R_7$ représente un groupe alcoyle avec 1 à 3 atomes de carbone, et B représente le groupe méthylène ou carbonyle, ou

A représente le groupe —CO—CO— et B le groupe méthylène,

E représente un groupe éthylène, n-propylène ou n-butylène éventuellement substitué par un groupe alcoyle avec 1 à 3 atomes de carbone, ou représente un groupe n-propylène substitué par un groupe hydroxy en position 2 ou représente un groupe n-butylène substitué par un groupe hydroxy en position 2 ou 3,

G représente un groupe méthylène ou éthylène éventuellement substitué par un groupe alcoyle avec 1 à 3 atomes de carbone,

$R_1$ et $R_2$, qui peuvent être identiques ou différents, représentent des groupes hydroxy, des groupes alcoyle, alcoxy ou phénylalcoxy, dans lesquels la partie alcoyle peut à chaque fois contenir 1 à 3 atomes de carbone, ou l'un des radicaux $R_1$ ou $R_2$ représente également un atome d'hydrogène ou $R_1$ ensemble avec $R_2$ représente un groupe alcoylènedioxy avec 1 ou 2 atomes de carbone,

$R_3$ et $R_4$, qui peuvent être identiques ou différents, représentent des atomes d'hydrogène ou d'halogène, des groupes hydroxy, des groupes alcoyle ou alcoxy avec chacun 1 à 4 atomes de carbone, des groupes trifluorométhyle ou cyano ou l'un des radicaux $R_3$ ou $R_4$ représente également un groupe nitro, ou $R_3$ ensemble avec $R_4$ représente un groupe alcoylènedioxy avec 1 ou 2 atomes de carbone,

$R_5$ représente un atome d'hydrogène, un groupe alcoyle, hydroxy, alcoxy, amino, alcoylamino, dialcoylamino, alcanoylamino, alcoxycarbonylamino ou bis(alcoxycarbonyl)-amino, dans lesquels la partie alcoyle peut à chaque fois contenir 1 à 3 atomes de carbone, ou représente un groupe méthylamino ou éthylamino substitué par un groupe trifluorométhyle, et,

$R_6$ représente un atome d'hydrogène, un groupe alcoyle, phénylalcoyle, alcanoyle ou alcoxycarbonyle, dans lesquels la partie alcoyle peut à chaque fois contenir 1 à 3 atomes de carbone, ou représente un groupe alcényle avec 3 à 5 atomes de carbone, et leurs sels d'addition d'acides, en particulier leurs sels d'addition d'acides physiologiquemnt supportables avec des acides minéraux ou organiques.

2. Dérivés de benzazépine de formule générale I selon la revendication 1, dans laquelle :

A représente un groupe $-CH_2-CH_2-$, $-CH=CH-$, —NH—CO—, $-CH_2-CO-$ ou
5 5

$CH_3$
|
-C = N-
5

et B représente le groupe méthylène ou carbonyle, ou

A représente le groupe —CO—CO— et B le groupe méthylène,

E représente le groupe éthylène, n-propylène, n-butylène, 2-méthyl-n-propylène ou 2-hydroxy-n-propylène,

G représente le groupe méthylène, éthylène ou 1-méthyléthylène,

$R_1$ représente un groupe méthyle, hydroxy, benzyloxy ou alcoxy avec 1 à 3 atomes de carbone,

$R_2$ représente un atome d'hydrogène, un groupe méthyle, hydroxy ou méthoxy, ou $R_1$ et $R_2$ représentent ensemble le groupe méthylènedioxy,

$R_3$ représente un atome d'hydrogène, de fluor, de chlore ou de brome, un groupe trifluorométhyle, nitro, alcoyle ou alcoxy avec chacun 1 à 4 atomes de carbone,

$R_4$ représente un atome d'hydrogène, de chlore ou de brome, un groupe méthyle, méthoxy ou cyano, ou $R_3$ et $R_4$ représentent ensemble le groupe méthylènedioxy,

$R_5$ représente un atome d'hydrogène, un groupe hydroxy, méthoxy, amino, acétylamino, éthoxycarbonylamino, bis(éthoxycarbonyl)amino, diméthylamino ou β,β,β-trifluoroéthylamino et

$R_6$ représente un atome d'hydrogène, un groupe alcoyle avec 1 à 3 atomes de carbone, un groupe allyle, benzyle, acétyle ou éthoxycarbonyle, et leurs sels d'addition d'acides, en particulier leurs sels d'addition d'acides physiologiquement supportables avec des acides minéraux ou organiques.

3. Dérivés de benzazépine de formule générale I selon la revendication 1, dans laquelle

A représente le groupe —$CH_2$—$CH_2$—, —CH=CH— ou

$CH_3$
|
-C = N-
5

et B représente le groupe carbonyle, ou

A représente le groupe —CH=CH—, —NH—CO— ou —CO—CO— et B le groupe méthylène,
5

E représente le groupe n-propylène,

G représente le groupe éthylène,

$R_1$ représente le groupe méthoxy ou hydroxy,

$R_2$ représente le groupe méthoxy ou $R_1$ et $R_2$ représentent ensemble le groupe méthylènedioxy,

$R_3$ représente le groupe méthoxy ou trifluorométhyle, un atome de chlore ou de brome,

$R_4$ représente le groupe méthoxy, un atome d'hydrogène, de chlore ou de brome ou $R_3$ et $R_4$ représentent ensemble le groupe méthylènedioxy,

$R_5$ représente un atome d'hydrogène, un groupe amino ou méthoxy et

$R_6$ représente un atome d'hydrogène, le groupe méthyle, éthyle, n-propyle ou allyle, et leurs sels d'addition d'acides physiologiquement supportables avec des acides minéraux ou organiques.

4. Dérivés de benzazépine de formule générale

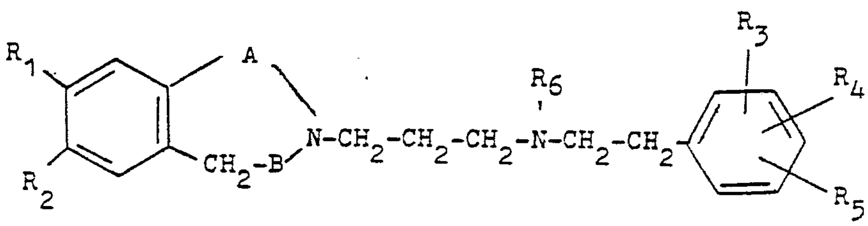

(Ia)

dans laquelle

A représente le groupe —$CH_2$—$CH_2$—, —CH=CH— ou

$CH_3$
|
-C = N-
5

et B représente le groupe carbonyle, ou

A représente le groupe —NH—CO— ou —COCO— et B représente le groupe méthylène,
5

$R_1$ et $R_2$ représentent chacun un groupe méthoxy,

$R_6$ représente un atome d'hydrogène, le groupe méthyle ou allyle,

$R_3$ représente un atome d'hydrogène ou un groupe méthoxy en position 3,

$R_4$ représente un groupe méthoxy en position 4 ou $R_3$ et $R_4$ représentent ensemble le groupe 3,4-méthylène-dioxy, et

$R_5$ représente un atome d'hydrogène ou

$R_3$ en position 3 représente un groupe trifluorométhyle, un atome de chlore ou de brome,

$R_4$ en position 5 représente un atome de chlore ou de brome et

$R_5$ en position 4 représente le groupe amino, et leurs sels d'addition d'acides physiologiquement supportables avec des acides minéraux ou organiques.

5. Le 1-[7,8-diméthoxy-1,3,4,5-tétrahydro-2H-3-benzazépine-2-one-3-yl]-3-[N-méthyl-N-(2-(3,4-diméthoxyphényl)-éthyl)-amino]-propane et ses sels d'addition d'acides.

6. Le 1-[7,8-diméthoxy-1,3,4,5-tétrahydro-2H-3-benzazépine-1,2-dione-3-yl]-3-[N-méthyl-N-(2-(3,4-diméthoxyphényl)-éthyl-amino]-propane et ses sels d'addition d'acides.

7. Le 1-[7,8-diméthoxy-1,3,4,5-tétrahydro-2H-3-benzazépine-2-one-3-yl]-3-[N-méthyl-N-(2-(4-amino-3,5-di-chlorophényl)-éthyl)-amino]-propane et ses sels d'addition d'acides.

8. Le 1-[7,8-diméthoxy-1,2,3,5-tétrahydro-2H-3-benzazépine-2-one-3-yl]-3-[N-méthyl-N-(2-(4-méthoxyphényl)-éthyl)-amino]-propane et ses sels d'additon d'acides.

9. Médicament pour lutter contre les tachycardies sinusales et les maladies ischémiques du cœur, contenant un composé selon les revendications 1 à 8, conjointement à un ou plusieurs excipients et/ou diluants inertes.

10. Procédé pour la préparation de dérivés de benzazépine de formule générale

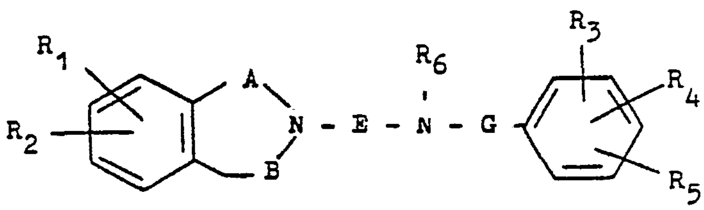

(I)

dans laquelle

A représente un groupe $—CH_2—CH_2—$, $—CH=CH—$, $—NH—CO—$, $—CH_2—CO—$ ou

5 5

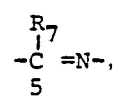

dans lequel $R_7$ représente un groupe alcoyle avec 1 à 3 atomes de carbone, et B représente le groupe méthylène ou carbonyle, ou

A représente le groupe —CO—CO— et B le groupe méthylène,

E représente un groupe éthylène, n-propylène ou n-butylène éventuellement substitué par un groupe alcoyle avec 1 à 3 atomes de carbone, ou représente un groupe n-propylène substitué par un groupe hydroxy en position 2, ou représente un groupe n-butylène substitué en position 2 ou 3 par un groupe hydroxy,

G représente un groupe méthylène ou éthylène éventuellement substitué par un groupe alcoyle avec 1 à 3 atomes de carbone,

$R_1$ et $R_2$, qui peuvent être identiques ou différents, représentent des groupes hydroxy, alcoyle, alcoxy ou phényl-alcoxy, dans lesquels la partie alcoyle peut à chaque fois contenir 1 à 3 atomes de carbone, ou l'un des radicaux $R_1$ ou $R_2$ représente également un atome d'hydrogène ou $R_1$ ensemble avec $R_2$ représente un groupe alcoylènedioxy avec 1 ou 2 atomes de carbone,

$R_3$ et $R_4$, qui peuvent être identiques ou différents, représentent des atomes d'hydrogène ou d'halogène, des groupes hydroxy, des groupes alcoyle ou alcoxy avec chacun 1 à 4 atomes de carbone, des groupes trifluorométhyle ou cyano ou l'un des radicaux $R_3$ ou $R_4$ représente également un groupe nitro, ou $R_3$ ensemble avec $R_4$ représente un groupe alcoylènedioxy avec 1 ou 2 atomes de carbone,

$R_5$ représente un atome d'hydrogène, un groupe alcoyle, hydroxy, alcoxy, amino, alcoylamino, dialcoylamino, alcanoylamino, alcoxycarbonylamino, ou bis(alcoxycarbonyl)-amino, dans lesquels la partie alcoyle peut à chaque fois contenir 1 à 3 atomes de carbone, ou représente un groupe méthylamino ou éthylamino substitué par un groupe trifluorométhyle, et

$R_6$ représente un atome d'hydrogène, un groupe alcoyle, phénylalcoyle, alcanoyle ou alcoxycarbonyle dans lesquels la partie alcoyle peut à chaque fois contenir 1 à 3 atomes de carbone, ou représente un groupe alcényle avec 3 à 5 atomes de carbone, ainsi que de leurs sels d'addition d'acides, en particulier de leurs sels d'addition d'acides physiologiquement supportables avec des acides minéraux ou organiques, caractérisé en ce que

a) on fait réagir un composé de formule générale

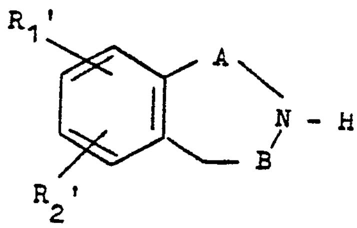

(II)

dans laquelle

A et B sont définis comme au début,

$R_1'$ et $R_2'$ représentent chacun un groupe hydroxy protégé par un groupe protecteur ou possèdent les significations mentionnées au début pour $R_1$ et $R_2$, avec un composé de formule générale

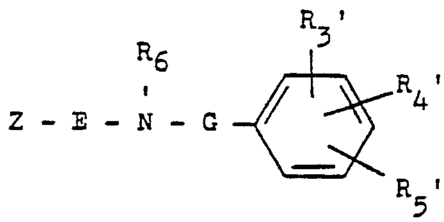

(III)

dans laquelle

$R_6$, E et G sont définis comme au début,

$R_3'$ et $R_5'$ représentent chacun un groupe hydroxy protégé par un groupe protecteur ou possèdent les significations mentionnées au début pour $R_3$ à $R_5$, et

Z représente un groupe partant nucléophile, ou son sel quaternaire interne éventuellement présent dans le mélange réactionnel de formule générale

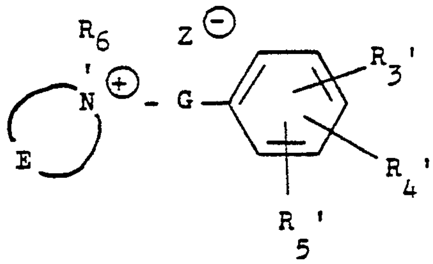

(IIIa)

dans laquelle E, G, Z, $R_3'$ à $R_5'$ et $R_6$ sont définis comme au début, et éventuellement clivage subséquent d'un radical protecteur utilisé ou

b) pour la préparation de composés de formule générale I, dans laquelle $R_6$ représente un atome d'hydrogène, un groupe alcényle avec 3 à 5 atomes de carbone, un groupe alcoyle ou phénylalcoyle avec chacun 1 à 3 atomes de carbone dans la partie alcoyle, on fait réagir un composé de formule générale

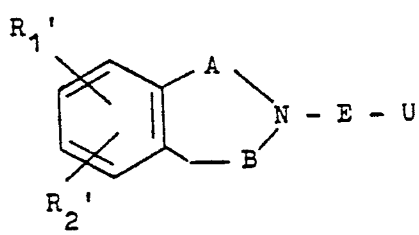

(IV)

avec un composé de formule générale

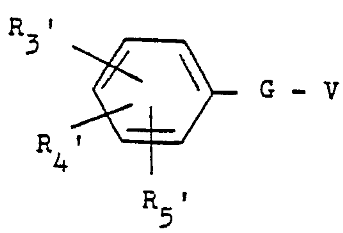

(V)

dans lequel

A, B, E et G sont définis comme au début,

$R_1'$ à $R_5'$ représentent chacun un groupe hydroxy protégé par un radical protecteur, ou possèdent les significations mentionnées au début pour $R_1$ à $R_5$, l'un des radicaux U ou V représente le groupe $R_6'$—NH—, $R_6'$ représentant un atome d'hydrogène, un groupe alcényle avec 3 à 5 atomes de carbone, un groupe alcoyle ou phénylacoyle avec chacun 1 à 3 atomes de carbone dans la partie alcoyle, l'autre des radicaux U ou V représente un groupe partant nucléophile ou le radical U ensemble avec un atome d'hydrogène du radical E en position β représente un atome d'oxygène et V représente un groupe $R_6'$—NH—, $R_6'$ étant défini comme indiqué plus haut, et éventuellement clivage subséquent d'un radical protecteur utilisé ou

c) pour la préparation de composés de formule générale I, dans laquelle $R_6$ représente un atome d'hydrogène, un groupe alcényle avec 3 à 5 atomes de carbone, un groupe alcoyle ou phénylalcoyle avec chacun 1 à 3 atomes de carbone dans la partie alcoyle, on fait réagir un composé de formule générale

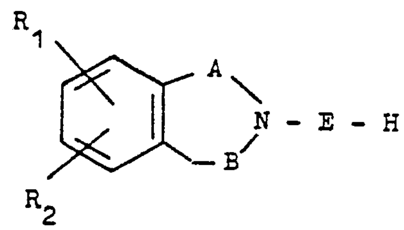

(VI)

dans laquelle A, B, E, $R_1$ et $R_2$ sont définis comme au début, dans le radical E toutefois 2 atomes d'hydrogène dans un groupe $—CH_2—$ ou $CH_3—$ du radical E étant remplacés par un atome d'oxygène, avec une amine de formule générale

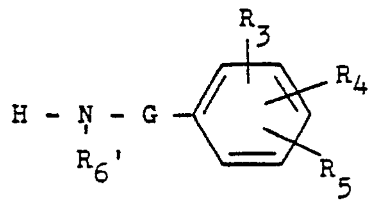

(VII)

dans laquelle G et $R_3$ à $R_5$ sont définis comme au début et $R_6'$ représente un atome d'hydrogène, un groupe alcényle avec 3 à 5 atomes de carbone, un groupe alcoyle ou phénylalcoyle avec chacun 1 à 3 atomes de carbone dans la partie alcoyle en présence d'un agent de réduction ou

d) pour la préparation de composés de formule générale I, dans laquelle $R_6$ représente un atome d'hydrogène, un groupe alcényle avec 3 à 5 atomes de carbone, un groupe alcoyle ou phénylalcoyle avec chacun 1 à 3 atomes de carbone dans la partie alcoyle, on fait réagir un composé de formule générale

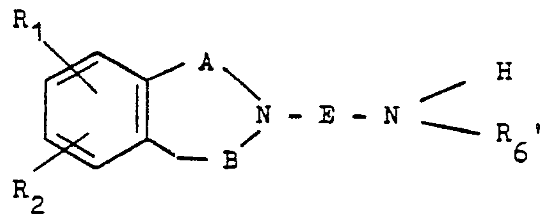

(VIII)

dans laquelle A, B, E, $R_1$ et $R_2$ sont définis comme au début et $R_6'$ représente un atome d'hydrogène, un groupe alcényle avec 3 à 5 atomes de carbone, un groupe alcoyle ou phénylalcoyle avec chacun 1 à 3 atomes de carbone dans la partie alcoyle, avec un composé de formule générale

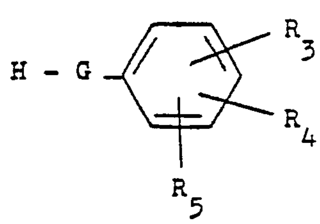

(IX)

dans laquelle G et $R_3$ à $R_5$ sont définis comme au début, dans le radical G toutefois 2 atomes d'hydrogène dans un groupe $—CH_2—$ ou $CH_3—$ du radical G étant remplacés par un atome d'oxygène, en présence d'un agent de réduction ou

e) pour la préparation de composés de formule générale I, dans laquelle A représente le groupe —NH—CO—, E représente un groupe éthylène, n-propylène ou n-butylène éventuellement substitué par un groupe alcoyle avec 1 à 3 atomes de carbone, et $R_6$ ne représente pas un atome d'hydrogène, on fait réagir un composé de formule générale :

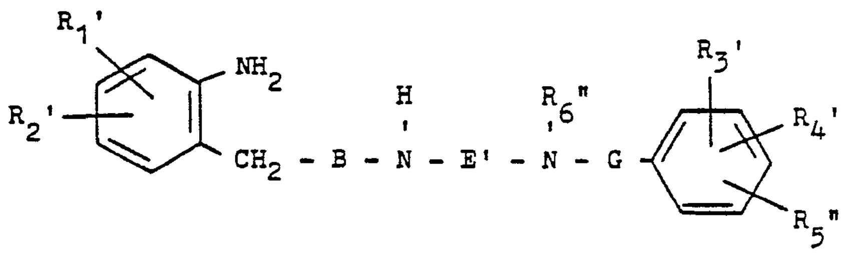

(X)

dans laquelle

B et G sont définis comme au début,

E' représente un groupe éthylène, n-propylène ou n-butylène éventuellement substitué par un groupe alcoyle avec 1 à 3 atomes de carbone,

$R_1'$ à $R_4'$ représentent chacun un groupe hydroxy protégé par un radical protecteur ou possèdent les significations mentionnées au début pour $R_1$ à $R_4$,

$R_5''$ représente un groupe hydroxy, amino ou alcoylamino protégé par un radical protecteur ou possède les significations mentionnées au début pour $R_5$ à l'exception du groupe amino ou alcoylamino, et

$R_{6''}$ possède les significations mentionnées au début pour $R_6$ à l'exception de l'hydrogène, ou représente un groupe protecteur pour un groupe amino, avec un dérivé d'acide carboxylique de formule générale

W—CO—W (XI)

dans laquelle W, qui peuvent être identiques ou différents, représentent chacun un groupe partant nucléophile tel qu'un atome de chlore ou de brome, un groupe alcoxy avec 1 à 3 atomes de carbone éventuellement substitué par des atomes d'halogène, ou un groupe imidazolyle-(2) et éventuellement clivage subséquent d'un radical protecteur utilisé, ou

f) pour la préparation de composés de formule générale I, dans laquelle A représente le groupe-$CH_2$-$CH_2$-, $R_1$ et $R_2$ ne représentent pas le groupe benzyloxy, $R_3$ ou $R_4$ ne représentent pas le groupe nitro et $R_6$ ne représente pas le groupe benzyle ou ne représente pas un groupe alcényle avec 3 à 5 atomes de carbone, on hydrogène un composé de formule générale

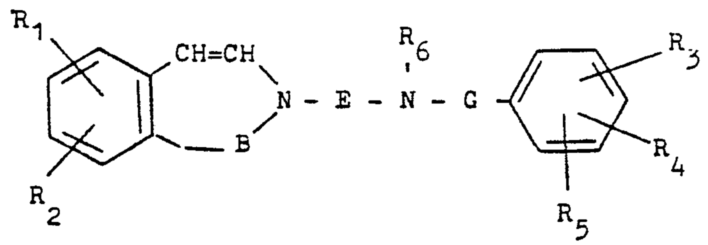

(XII)

dans laquelle $R_1$ à $R_5$, B, E et G sont définis comme au début, $R_3$ ou $R_4$ ne pouvant représenter un groupe nitro, dans le cas où $R_5$ représente un groupe amino, ou

g) on fait réagir un composé de formule générale

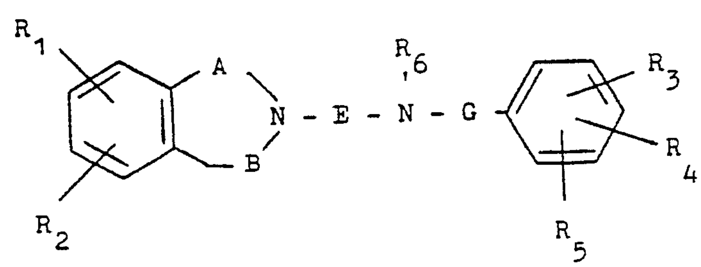

(XIII)

dans laquelle $R_1$ à $R_6$, A, B, E et G sont définis comme au début, toutefois au moins l'un des radicaux $R_1$ à

$R_4$ devant représenter un groupe hydroxy ou $R_5$ devant représenter un groupe hydroxy, un groupe amino ou alcoylamino avec 1 à 3 atomes de carbone, ou $R_6$ devant représenter un atome d'hydrogène, avec un composé de formule générale

$R_8$—X (XIV)

dans laquelle $R_8$ représente un groupe alcoyle avec 1 à 3 atomes de carbone ou également un groupe alcényle avec 3 à 5 atomes de carbone, lorsque $R_6$ représente un atome d'hydrogène, ou également un groupe alcoyle avec 1 à 3 atomes de carbone substitué par un groupe phényle lorsque $R_1$ ou $R_2$ représentent le groupe hydroxy et/ou $R_6$ représente un atome d'hydrogène, et X représente un groupe partant nucléophile tel qu'un atome d'halogène ou un groupe sulfonyloxy ou lorsqu'au moins l'un des radicaux $R_1$ à $R_5$ représente un groupe hydroxy, X ensemble avec un atome d'hydrogène du radical $R_8$ en position α représente un groupe diazo ou également, lorsque $R_6$ représente un atome d'hydrogène ou $R_5$ représente un groupe amino ou alcoylamino, un atome d'oxygène ou

h) pour la préparation de composés de formule générale I, dans laquelle A ne représente pas un groupe —CH=CH—, $R_3$ représente un atome de chlore ou de brome et $R_5$ représente un groupe amino ou alcoylamino, on halogène un composé de formule générale

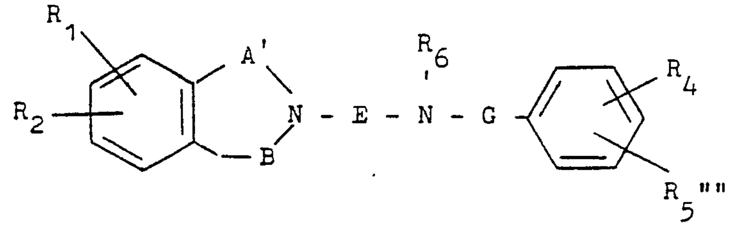

(XV)

dans laquelle

$R_1$, $R_2$, $R_4$, $R_6$, B, E et G sont définis comme au début, A' possède les significations mentionnées au début pour A à l'exception du groupe —CH=CH— et

$R_5''''$ représente un groupe amino ou alcoylamino avec 1 à 3 atomes de carbone ou

i) pour la préparation de composés de formule générale I, dans laquelle A représente le groupe —COCO—, B représente le groupe méthylène et E représente un groupe éthylène, n-propylène ou n-butylène éventuellement substitué par un groupe alcoyle avec 1 à 3 atomes de carbone, on oxyde un composé de formule générale

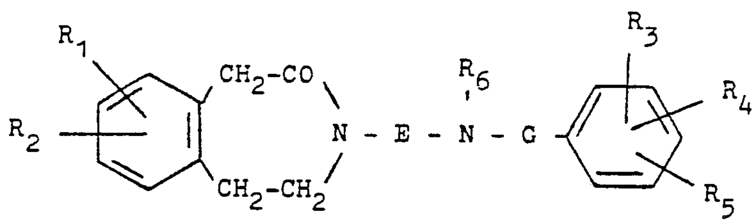

(XVI)

dans laquelle $R_1$ à $R_6$, E et G sont définis comme au début,

et si on le désire ensuite on transforme un composé obtenu de formule générale I, dans laquelle B représente le groupe carbonyle, au moyen d'une réduction en un composé correspondant de formule générale I, dans laquelle B représente le groupe méthylène, et/ou

on transforme un composé obtenu de formule générale I, dans laquelle A ne représente pas un groupe —CH=CH— ou

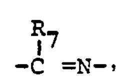

et $R_6$ représente un groupe benzyle ou 1-phényléthyle, au moyen d'une hydrogénation catalytique en un composé correspondant de formule générale I, dans laquelle $R_6$ représente un atome d'hydrogène, et/ou

on transforme un composé obtenu de formule générale I, dans laquelle $R_3$ ou $R_4$ représente un groupe nitro, au moyen d'une réduction en un composé correspondant de formule générale I, dans laquelle $R_5$ représente un groupe amino et/ou

on transforme un composé obtenu de formule générale I, dans laquelle $R_6$ représente un atome d'hydrogène et/ou $R_5$ représente un groupe amino, au moyen d'une acylation en un composé correspondant de formule générale I, dans laquelle $R_6$ représente un groupe alcanoyle ou alcoxycarbonyle et/ou $R_5$ représente un groupe alcanoylamino, alcoxycarbonylamino ou bis (alcoxycarbonylamino), la partie alcoyle pouvant à chaque fois contenir 1 à 3 atomes de carbone, et/ou

on transforme un composé obtenu de formule générale I, dans laquelle $R_5$ représente un groupe amino, $R_6$ ne représente pas un atome d'hydrogène, $R_3$ et $R_4$ chacun ne représentent pas un groupe cyano, par l'intermédiaire d'un sel de diazonium correspondant, en un composé correspondant de formule générale I, dans laquelle $R_5$ représente un atome d'hydrogène, un groupe hydroxy ou alcoxy ou $R_5$ représente un atome d'hydrogène et $R_3$ un atome d'halogène ou le groupe cyano et/ou

on transforme un composé obtenu de formule générale I en ses sels d'addition d'acides, en particulier en ses sels physiologiquement supportables avec des acides minéraux ou organiques.

11. Procédé pour la préparation d'un médicament, caractérisé en ce qu'un composé de formule générale I ou un sel d'addition d'acide physiologiquement supportable de celui-ci est introduit par voie non chimique dans un ou plusieurs excipients et/ou diluants inertes.

**Revendications** (pour l'Etat contractant AT)

1. Procédé pour la préparation de dérivés de benzazépine de formule générale

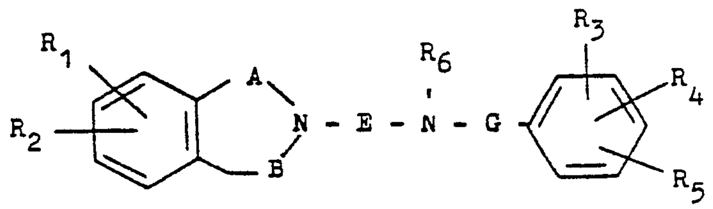

(I)

dans laquelle

A représente un groupe $—CH_2—CH_2—$, $—CH=CH—$, $—NH—CO—$, $—CH_2—CO—$ ou

5 · 5

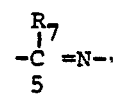

dans lequel $R_7$ représente un groupe alcoyle avec 1 à 3 atomes de carbone, et B représente le groupe méthylène ou carbonyle ou

A représente le groupe $—CO—CO—$ et B le groupe méthylène,

E représente un groupe éthylène, n-propylène ou n-butylène éventuellement substitué par un groupe alcoyle avec 1 à 3 atomes de carbone, ou représente un groupe n-propylène substitué par un groupe hydroxy en position 2, ou représente un groupe n-butylène substitué par un groupe hydroxy en position 2 ou 3,

G représente un groupe méthylène ou éthylène éventuellement substitué par un groupe alcoyle avec 1 à 3 atomes de carbone,

$R_1$ et $R_2$, qui peuvent être identiques ou différents, représentent des groupes hydroxy, des groupes alcoyle, alcoxy ou phénylalcoxy, dans lesquels la partie alcoyle peut à chaque fois contenir 1 à 3 atomes de carbone, ou l'un des radicaux $R_1$ ou $R_2$ représente également un atome d'hydrogène ou $R_1$ ensemble avec $R_2$ représente un groupe alcoylènedioxy avec 1 ou 2 atomes de carbone,

$R_3$ et $R_4$, qui peuvent être identiques ou différents, représentent des atomes d'hydrogène ou d'halogène, des groupes hydroxy, des groupes alcoyle ou alcoxy avec chacun 1 à 4 atomes de carbone, des groupes trifluorométhyle ou cyano ou l'un des radicaux $R_3$ ou $R_4$ représente également un groupe nitro ou $R_3$ ensemble avec $R_4$ représente un groupe alcoylènedioxy avec 1 ou 2 atomes de carbone,

$R_5$ représente un atome d'hydrogène, un groupe alcoyle, hydroxy, alcoxy, amino, alcoylamino, dialcoylamino, alcanoylamino, alcoxycarbonylamino, ou bis(alcoxycarbonyl)-amino, dans lesquels la partie alcoyle peut à chaque fois contenir 1 à 3 atomes de carbone, ou représente un groupe méthylamino ou éthylamino substitué par un groupe trifluorométhyle, et

$R_6$ représente un atome d'hydrogène, un groupe alcoyle, phénylalcoyle, alcanoyle ou alcoxycarbonyle, dans lesquels la partie alcoyle peut à chaque fois contenir 1 à 3 atomes de carbone, ou représente un groupe alcényle avec 3 à 5 atomes de carbone, ainsi que de leurs sels d'addition d'acides, en particulier de leurs sels d'addition d'acides physiologiquement supportables avec des acides minéraux ou organiques, caractérisé en ce que

a) on fait réagir un composé de formule générale

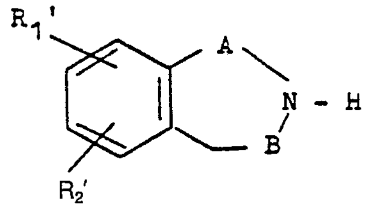

(II)

dans laquelle

A et B sont définis comme au début,

$R_1'$ et $R_2'$ représentent chacun un groupe hydroxy protégé par un radical protecteur ou possèdent les significations mentionnées au début pour $R_1$ et $R_2$, avec un composé de formule générale

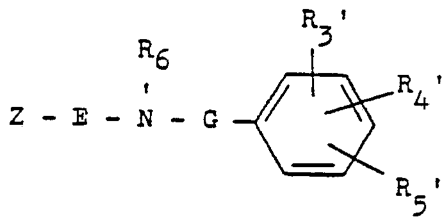

(III)

dans laquelle

$R_6$, E et G sont définis comme au début,

$R_3'$ à $R_5'$ représentent chacun un groupe hydroxy protégé par un radical protecteur, ou possèdent les significations mentionnées au début pour $R_3$ à $R_5$, et

Z représente un groupe partant nucléophile, ou son sel quaternaire interne éventuellement présent dans le mélange réactionnel de formule générale :

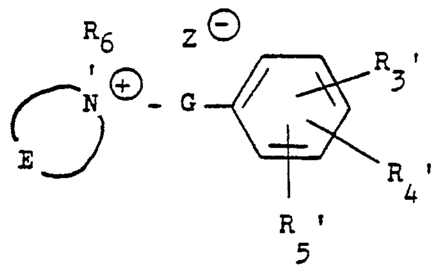

(IIIa)

dans laquelle E, G, Z, $R_3'$ à $R_5'$ et $R_6$ sont définis comme au début, et éventuellement clivage subséquent d'un radical protecteur utilisé, ou

b) pour la préparation de composés de formule générale I, dans laquelle $R_6$ représente un atome d'hydrogène, un groupe alcényle avec 3 à 5 atomes de carbone, un groupe alcoyle ou phénylalcoyle avec chacun 1 à 3 atomes de carbone dans la partie alcoyle, on fait réagir un composé de formule générale

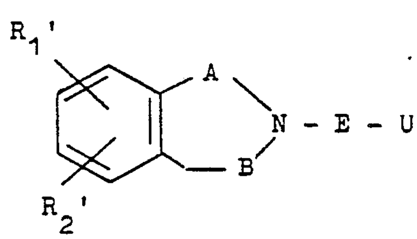

(IV)

avec un composé de formule générale

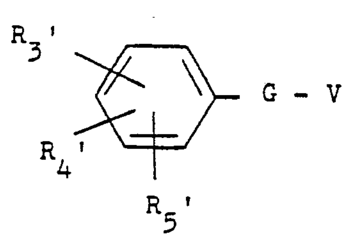

(V)

dans lesquelles

A, B, E et G sont définis comme au début,

$R_1'$ à $R_5'$ représentent chacun un groupe hydroxy protégé par un radical protecteur, ou possèdent les significations mentionnées au début pour $R_1$ à $R_5$, l'un des radicaux U ou V représente le groupe $R_6'$—NH—, $R_6'$ représentant un atome d'hydrogène, un groupe alcényle avec 3 à 5 atomes de carbone, un groupe alcoyle ou phénylalcoyle avec chacun 1 à 3 atomes de carbone dans la partie alcoyle, l'autre des radicaux U et V représente un groupe partant nucléophile ou le radical U ensemble avec un atome d'hydrogène du radical E en position β représente un atome d'oxygène et V représente un groupe R6'—NH—, $R_6'$ étant défini comme indiqué plus haut, et éventuellement clivage subséquent d'un radical protecteur utilisé ou

c) pour la préparation de composés de formule générale I, dans laquelle $R_6$ représente un atome d'hydrogène, un groupe alcényle avec 3 à 5 atomes de carbone, un groupe alcoyle ou phénylalcoyle avec chacun 1 à 3 atomes de carbone dans la partie alcoyle, on fait réagir un composé de formule générale

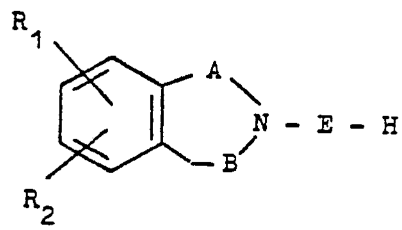

(VI)

dans laquelle A, B, E, $R_1$ et $R_2$ sont définis comme au début, dans le radical E cependant 2 atomes d'hydrogène dans un groupe $—CH_2—$ ou $CH_3—$ du radical E étant remplacés par un atome d'oxygène, avec une amine de formule générale

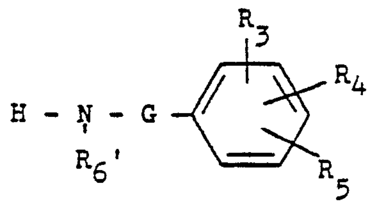

(VII)

dans laquelle

G et $R_3$ à $R_5$ sont définis comme au début et

$R_6'$ représente un atome d'hydrogène, un groupe alcényle avec 3 à 5 atomes de carbone, un groupe alcoyle ou phénylalcoyle avec chacun 1 à 3 atomes de carbone dans la partie alcoyle, en présence d'un agent de réduction ou

d) pour la préparation de composés de formule générale I, dans laquelle $R_6$ représente un atome d'hydrogène, un groupe alcényle avec 3 à 5 atomes de carbone, un groupe alcoyle ou phénylalcoyle avec chacun 1 à 3 atomes de carbone dans la partie alcoyle, on fait réagir un composé de formule générale

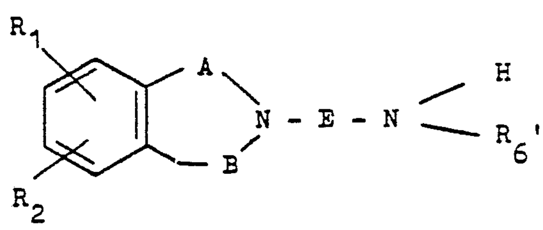

(VIII)

dans laquelle

A, B, E, $R_1$ et $R_2$ sont définis comme au début, et $R_6'$ représente un atome d'hydrogène, un groupe alcényle avec 3 à 5 atomes de carbone, un groupe alcoyle ou phénylalcoyle avec chacun 1 à 3 atomes de carbone dans la partie alcoyle, avec un composé de formule générale

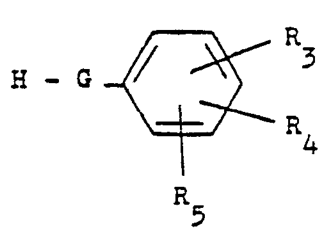

(IX)

dans laquelle G et $R_3$ à $R_5$ sont définis comme au début, dans le radical G cependant 2 atomes dhydrogène étant remplacés dans un groupe —$CH_2$— ou $CH_3$— du radical G par un atome d'oxygène, en présence d'un agent de réduction ou

e) pour la préparation de composés de formule générale I, dans laquelle A représente le groupe —NH—CO—, E représente un groupe éthylène, n-propylène ou n-butylène éventuellement substitué par un groupe alcoyle avec 1 à 3 atomes de carbone, et $R_6$ ne représente pas un atome d'hydrogène, on fait réagir un composé de formule générale

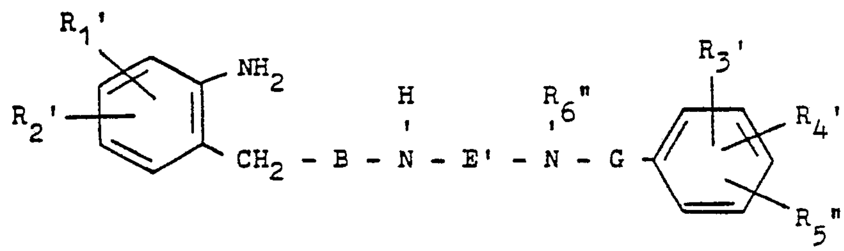

(X)

dans laquelle

B et G sont définis comme au début,

E′ représente un groupe éthylène, n-propylène ou n-butylène éventuellement substitué par un groupe alcoyle avec 1 à 3 atomes de carbone,

$R_1'$ à $R_4'$ représentent chacun un groupe hydroxy protégé par un radical protecteur ou possèdent les significations mentionnées au début pour $R_1$ à $R_4$,

$R_5''$ représente un groupe hydroxy, amino ou alcoylamino protégé par un radical protecteur, ou possède les significations mentionnées au début pour $R_5$ à l'exception du groupe amino ou alcoylamino, et,

$R_6''$ possède les significations mentionnées au début pour $R_6$ à l'exception de l'hydrogène, ou représente un groupe protecteur pour un groupe amino, avec un dérivé d'acide carboxylique de formule générale

$$W—CO—W \qquad (XI)$$

dans laquelle W, qui peuvent être identiques ou différents, représentent chacun un groupe partant nucléophile tel qu'un atome de chlore ou de brome, un groupe alcoxy avec 1 à 3 atomes de carbone éventuellement substitué par des atomes d'halogène ou un groupe imidazolyle-(2) et éventuellement clivage subséquent d'un radical protecteur utilisé, ou

f) pour la préparation de composés de formule générale I, dans laquelle A représente le groupe —$CH_2$—CH2—, $R_1$ et $R_2$ ne représentent pas un groupe benzyloxy, $R_3$ ou $R_4$ ne représente pas un groupe nitro et $R_6$ ne représente pas un groupe benzyle, ou ne représente pas un groupe alcényle avec 3 à 5 atomes de carbone, on hydrogène un composé de formule générale

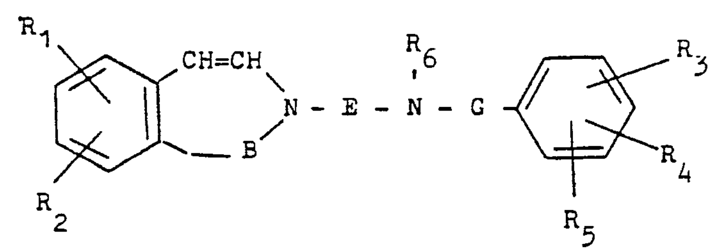

(XII)

dans laquelle $R_1$ à $R_5$, B, E et G sont définis comme au début, $R_3$ ou $R_4$ ne pouvant représenter un groupe nitro, dans le cas où $R_5$ représente un groupe amino, ou

g) on fait réagir un composé de formule générale

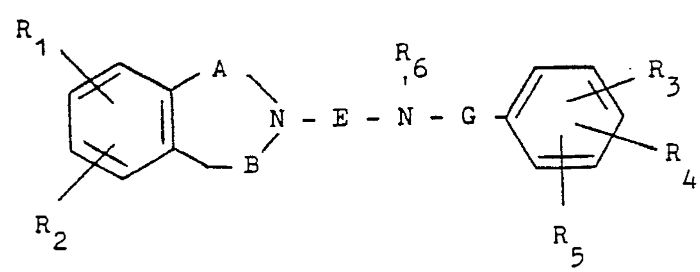

(XIII)

dans laquelle $R_1$ à $R_6$, A, B, E et G sont définis comme au début, cependant au moins l'un des radicaux $R_1$ à $R_4$ devant représenter un groupe hydroxy ou $R_5$ un groupe hydroxy, un groupe amino ou alcoylamino avec 1 à 3 atomes de carbone, ou $R_6$ un atome d'hydrogène, avec un composé de formule générale

$$R_8—X \qquad (XIV)$$

dans laquelle $R_8$ représente un groupe alcoyle avec 1 à 3 atomes de carbone ou également un groupe alcényle avec 3 à 5 atomes de carbone lorsque $R_6$ représente un atome d'hydrogène, ou également un groupe alcoyle avec 1 à 3 atomes de carbone substitué par un groupe phényle, lorsque $R_1$ ou $R_2$ représente le groupe hydroxy et/ou $R_6$ représente un atome d'hydrogène, et X représente un groupe partant nucléophile tel qu'un atome d'halogène ou un groupe sulfonyloxy ou, dans le cas où au moins l'un des radicaux $R_1$ à $R_5$ représente un groupe hydroxy, X représente ensemble avec un atome dhydrogène du radical $R_8$ en position $\alpha$ un groupe diazo ou également, lorsque $R_6$ représente un atome d'hydrogène ou $R_5$ représente un groupe amino ou alcoylamino, un atome d'oxygène ou

h) pour la préparation de composés de formule générale I, dans laquelle A ne représente pas un groupe —CH=CH—, $R_3$ représente un atome de chlore ou de brome et $R_5$ représente un groupe amino ou alcoylamino, on halogène un composé de formule générale

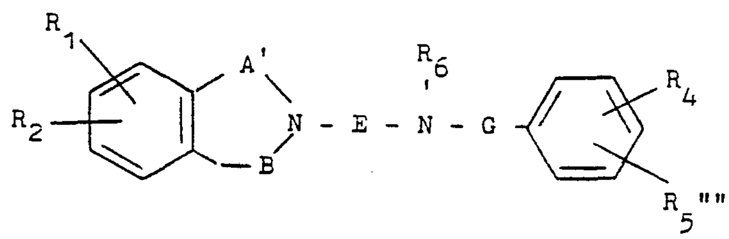

(XV)

dans laquelle

$R_1$, $R_2$, $R_4$, $R_6$, B, E et G sont définis comme au début A' possède les significations mentionnées au début pour A à l'exception du groupe —CH=CH— et

$R_5''''$ représente un groupe amino ou alcoylamino avec 1 à 3 atomes de carbone, ou

i) pour la préparation de composés de formule générale I, dans laquelle A représente le groupe —COCO—, B représente le groupe méthylène et E représente un groupe éthylène, n-propylène ou n-butylène éventuellement substitué par un groupe alcoyle avec 1 à 3 atomes de carbone, on oxyde un composé de formule générale

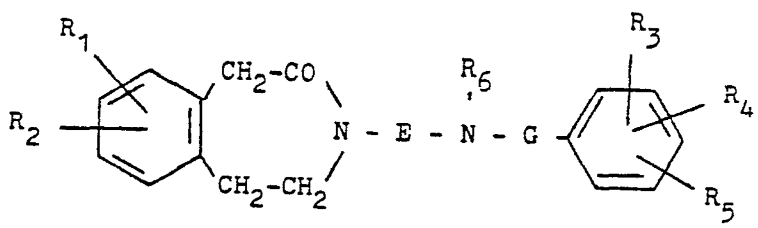

(XVI)

dans laquelle $R_1$ à $R_6$, E et G sont définis comme au début,

et si on le désire ensuite on transforme un composé obtenu de formule générale I, dans laquelle B représente le groupe carbonyle, au moyen d'une réduction en un composé correspondant de formule générale I dans laquelle B représente le groupe méthylène, et/ou

on transforme un composé obtenu de formule générale I, dans laquelle A ne représente pas un groupe —CH=CH— ou

$$-\overset{R_7}{\overset{|}{C}}=N-$$

et $R_6$ représente un groupe benzyle ou 1-phényléthyle, au moyen d'une hydrogénation catalytique en un composé correspondant de formule générale I, dans laquelle $R_6$ représente un atome d'hydrogène, et/ou

on transforme un composé obtenu de formule générale I, dans laquelle $R_3$ ou $R_4$ représente un groupe nitro, au moyen d'une réduction en un composé correspondant de formule générale I, dans laquelle $R_5$ représente un groupe amino, et/ou

on transforme un composé obtenu de formule générale I, dans laquelle $R_6$ représente un atome d'hydrogène et/ou $R_5$ représente un groupe amino, au moyen d'une acylation en un composé correspondant de formule générale I, dans laquelle $R_6$ représente un groupe alcanoyle ou alcoxycarbo-

nyle, et/ou $R_5$ représente un groupe alcanoylamino, alcoxycarbonylamino ou bis(alcoxycarbonylamino), la partie alcoyle pouvant contenir à chaque fois 1 à 3 atomes de carbone, et/ou

on transforme un composé obtenu de formule générale I, dans laquelle $R_5$ représente un groupe amino, $R_6$ ne représente pas un atome d'hydrogène, $R_3$ et $R_4$ ne représentent pas chacun un groupe cyano, par l'intermédiaire d'un sel de diazonium correspondant, en un composé correspondant de formule générale I, dans laquelle $R_5$ représente un atome d'hydrogène, un groupe hydroxy ou alcoxy ou $R_5$ représente un atome d'hydrogène et $R_3$ représente un atome d'halogène, ou le groupe cyano, et/ou

on transforme un composé obtenu de formule générale I, en ses sels d'addition d'acides, en particulier en ses sels physiologiquement supportables, avec des acides minéraux ou organiques.

2. Procédé selon la revendication 1, pour la préparation de nouveaux dérivés de benzazépine, de formule générale

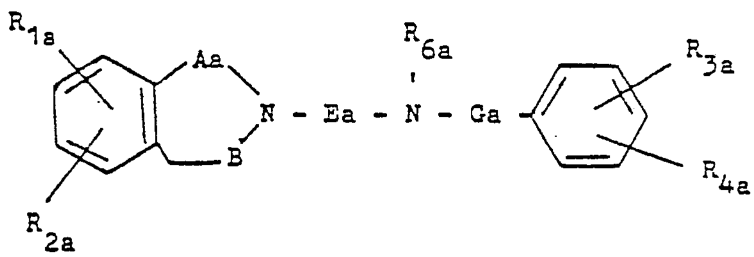

(Ia)

dans laquelle

Aa représente le groupe $-CH_2-CH_2-$, $-CH=CH-$ ou $-NH-CO-$
5

B représente le groupe méthylène ou carbonyle,

Ea représente le groupe éthylène, n-propylène ou n-butylène,

Ga représente le groupe éthylène,

$R_{1a}$ représente un groupe alcoxy avec 1 à 3 atomes de carbone,

$R_{2a}$ à $R_{4a}$, qui peuvent être identiques ou différents, représentent chacun des atomes d'hydrogène ou des groupes alcoxy avec 1 à 3 atomes de carbone ou

$R_{1a}$ ensemble avec $R_{2a}$ et/ou $R_{3a}$ ensemble avec $R_{4a}$ représentent chacun un groupe méthylène-dioxy, et

$R_{6a}$ représente un atome d'hydrogène, un groupe alcoyle avec 1 à 3 atomes de carbone éventuellement substitué par un groupe phényle ou représente le groupe allyle, et de leurs sels d'addition d'acides, en particulier de leurs sels d'adition d'acides physiologiquement supportables avec des acides minéraux ou organiques, caractérisé en ce que :

b) on fait réagir un composé de formule générale

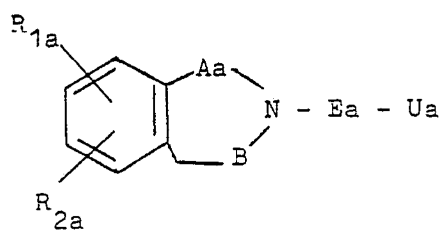

(IVa)

avec un composé de formule générale

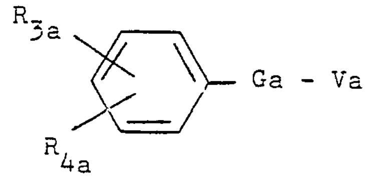

(Va)

dans lesquelles $R_{1a}$ à $R_{4a}$, Aa, B, Ea et Ga sont définis comme au début, l'un des radicaux Ua ou Va représente le groupe $R_{6a}$—NH—, $R_{6a}$ étant défini comme au début, et l'autre des radicaux Ua ou Va

représente un groupe partant nucléophile tel qu'un atome d'halogène ou un groupe sulfonyloxy, ou

e) pour la préparation d'un composé de formule générale Ia dans laquelle Aa représente le groupe —NH—CO— et $R_{6a}$ ne représente pas un atome d'hydrogène, on fait réagir un composé de formule générale

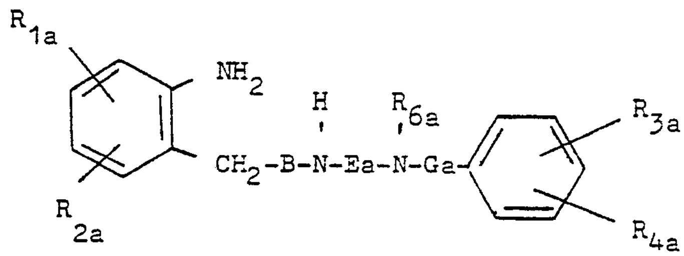

(Xa)

dans laquelle $R_{1a}$ à $R_{4a}$, B, Ea, Ga et $R_{6a}$ sont définis comme au début à l'exception de l'atome d'hydrogène, avec un dérivé d'acide carboxylique de formule générale

Wa—CO—Wa (XIa)

dans laquelle Wa, qui peuvent être identiques ou différents, représentent chacun un groupe partant nucléophile ou

f) pour la préparation d'un composé de formule générale Ia, dans laquelle Aa représente le groupe $—CH_2—CH_2—$, on hydrogène un composé de formule générale

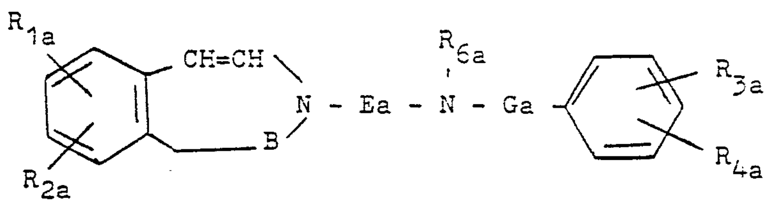

(XIIa)

dans laquelle $R_{1a}$ à $R_{4a}$, $R_{6a}$, B, Ea et Ga sont définis comme au début, en présence d'un catalyseur d'hydrogénation et dans un solvant ou

g) on fait réagir un composé de formule générale

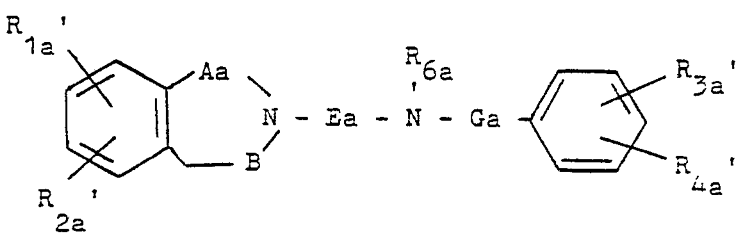

(XIIIa)

dans laquelle

$R_{6a}$, A, B, Ea et Ga sont définis comme au début, et $R_{1a}'$ à $R_{4a}'$ représentent chacun un groupe hydroxy ou possèdent les significations mentionnées au début pour $R_{1a}$ à $R_{4a}$, toutefois au moins l'un des radicaux $R_{1a}'$ à $R_{4a}'$ devant représenter un groupe hydroxy ou $R_{6a}$ un atome d'hydrogène, avec un composé de formule générale

$R_{8a}$—X (XIVa)

dans laquelle

$R_8$a représente un groupe alcoyle avec 1 à 3 atomes de carbone éventuellement substitué par un groupe phényle, et

X représente un groupe partant nucléophile tel qu'un atome d'halogène ou un groupe sulfonyloxy, ou, lorsqu'au moins l'un des radicaux $R_{1a}'$ à $R_{4a}'$ représente un groupe hydroxy, ensemble avec un atome d'hydrogène du radical $R_{8a}$ en position α représente un groupe diazo ou également un atome d'oxygène, lorsque $R_{6a}$ représente un atome d'hydrogène,

et si on le désire ensuite on transforme un composé ainsi obtenu de formule générale Ia, dans laquelle B représente le groupe carbonyle, au moyen d'une réduction en un composé correspondant de formule générale Ia dans laquelle B représente le groupe méthylène, et/ou

on transforme un composé ainsi obtenu de formule générale Ia dans laquelle $R_{6a}$ représente un groupe benzyle ou 1-phényléthyle, au moyen d'une hydrogénation catalytique en un composé correspondant de formule générale Ia, dans laquelle $R_{6a}$ représente un atome d'hydrogène et/ou

on transforme un composé obtenu de formule générale Ia en son sel d'addition d'acide, en particulier en son sel d'addition d'acide physiologiquement supportable, au moyen d'un acide minéral ou organique .

Priorité : 19 mai 1981 (P 31 19 874.0)

3. Procédé selon les revendications 1 et 2, caractérisé en ce que la réaction est effectuée dans un solvant.

4. Procédé selon les revendications 1a, 1b, 1e, 2b, 2e et 3, caractérisé en ce que la réaction est effectuée en présence d'une base et à des températures entre 0 et 150 °C, de préférence cependant à la température d'ébullition du solvant utilisé.

5. Procédé selon les revendications 1a, 1b et 3, caractérisé en ce qu'un radical protecteur utilisé est clivé hydrolytiquement en présence d'un acide ou d'une base ou en ce qu'un groupe benzyle utilisé comme radical protecteur est clivé hydrogénolytiquement.

6. Procédé selon les revendications 1c, 1d et 3, caractérisé en ce que, pour la préparation de composés de formule générale I, dans laquelle $R_6$ représente un atome d'hydrogène, la réaction est effectuée en présence d'hydrogène et d'un catalyseur d'hydrogénation.

7. Procédé selon les revendications 1c, 1d et 3, caractérisé en ce que la réaction est effectuée en présence d'un hydrure métallique complexe tel que le cyanoborohydrure de lithium ou de sodium, à une valeur de pH de 6-7 et à la température ambiante.

8. Procédé selon les revendications 1g, 2g et 3, caractérisé en ce que la réaction est effectuée en présence d'un agent fixant les acides et à des températures entre 0 et 150 °C, de préférence cependant à des températures entre 20 et 80 °C, lorsque X représente un groupe partant nucléophile.

9. Procédé selon les revendications 1g, 2g et 3, caractérisé en ce que la réaction avec un composé diazo de formule générale XIV est effectuée à des températures entre 0 et 30 °C, lorsqu'au moins l'un des radicaux $R_1$ à $R_5$ représente un groupe hydroxy.

10. Procédé selon les revendications 1g, 2g et 3, caractérisé en ce que la réaction avec un aldéhyde de formule générale XIV est effectuée en présence d'un agent de réduction à des températures entre 0 et 120 °C, lorsque $R_6$ représente un atome d'hydrogène et/ou $R_5$ représente un groupe amino ou alcoylamino.

11. Procédé pour la préparation d'un médicament, caractérisé en ce qu'on introduit un composé de formule générale I ou un sel d'addition d'acide physiologiquement supportable de celui-ci par voie non chimique dans un ou plusieurs excipients et/ou diluants inertes.